# EUROPEAN PATENT APPLICATION

(11) **EP 2 520 659 A1**
(43) Date of publication of application: **07.11.2012**
(21) Application number: 10841026.7
(22) Date of filing: 28.12.2010
(51) Int. Cl.: C12Q 1/48, C12N 15/09, G01N 33/50, C12N 9/12

(54) **SCREENING METHOD**

(30) Priority: 28.12.2009 JP 2009296811
(71) Applicant: AJINOMOTO CO., INC., Chuo-ku Tokyo 104-8315 (JP)
(72) Inventor: TAKESHITA Sen, Kawasaki-shi Kanagawa 210-0801 (JP); YAMAMOTO Takashi, Kawasaki-shi Kanagawa 210-0801 (JP); ANDOU Ayatoshi, Kawasaki-shi Kanagawa 210-0801 (JP); OKUTSU Tomohisa, Kawasaki-shi Kanagawa 210-0801 (JP); EVIRYANTI Agung, Kawasaki-shi Kanagawa 210-0801 (JP); FUKUCHI Naoyuki, Kawasaki-shi Kanagawa 210-0801 (JP); KAGEYAMA Shunsuke, Kawasaki-shi Kanagawa 210-0801 (JP)
(74) Representative: Nicholls, Kathryn Margaret
(86) International application number: PCT/JP2010/073676
(87) International publication number: WO 2011/081171

(57) **Abstract**

The present invention relates to a novel method for screening for a therapeutic or prophylactic agent for an inflammatory disease.

More particularly, the present invention relates to a method for screening for a therapeutic or prophylactic agent for an inflammatory disease by selecting a substance having an inhibitory activity specific to PIKfyve by using the presence or absence of the inhibitory activity as an indicator.

## Description

### TECHNICAL FIELD

The present invention relates to a method for screening for a therapeutic or prophylactic agent for an inflammatory disease.

### BACKGROUND ART

Interleukin-12 (IL-12) and interleukin-23 (IL-23) are cytokines that are mainly generated and secreted by phagocytes such as activated macrophage and dendritic cells, and antigen presenting cells. IL-12 is a heterodimer molecule consisting of covalently bound p35 and p40, while IL-23 is a heterodimer molecule consisting of covalently bound p19 and p40 subunit that is shared with IL-12. p35, p19 and p40 are each coded by different genes, whose productions are known to be enhanced in macrophages or the like via an increase in the gene expression due to extracellular stimulation with INF-γ or the like.

Major functions of IL-12 and IL-23 are to act on the T-cells to promote immune responses. IL-12 acts on naive T-cells and promotes differentiation into T lymphocytes of Type Th1 (type 1 helper T cells). In addition to promotion of differentiation from naive T-cells into Type Th1 T lymphocytes, IL-23 also acts on memory T-cells and IL-17-producing T-cells and results in proliferation.
Hyperfunction due to excessive production of IL-12 or IL-23 is known to be involved in worsening of pathological conditions of inflammatory diseases including various autoimmune diseases through excessive differentiation and activation of Type Th1 T cells. For example, it is known to be involved in, but not limited to, multiple sclerosis, systemic sclerosis, sepsis, myasthenia gravis, autoimmune neurological disease, Guillain-Barre syndrome, autoimmune uveitis, autoimmune hemolytic anemia, malignant anemia, autoimmune thrombocytopenia, temporal arteritis, antiphospholipid syndrome, vasculitis, Wegener's granulomatosis, Behcet's disease, psoriasis, psoriatic arthritis, herpetic dermatitis, pemphigus vulgaris, vitiligo, Crohn's disease, ulcerative colitis, interstitial pulmonary fibrosis, myelofibrosis, hepatic fibrosis, myocarditis, autoimmune thyroid disease, thyroiditis, primary biliary cirrhosis, autoimmune hepatitis, immune-mediated diabetes mellitus, Graves' disease, Hashimoto's thyroiditis, autoimmune oophoritis and orchitis, autoimmune adrenalitis, rheumatoid arthritis, juvenile rheumatoid arthritis, systemic lupus erythematosus, scleroderma, polymyositis, dermatomyositis, spondyloarthropathy, ankylosing spondylitis, Sjogren's syndrome, connective tissue disease, graft-versus-host disease and ischemic vascular disorder. Therefore, suppression of the functions of IL-12 or IL-23 is expected to ameliorate these inflammatory diseases.

So far, a number of therapeutic agents have been developed based on IL-12- and IL-23-suppressing actions of anti-IL-12 and anti-IL-23 antibodies, but they are not yet satisfactory. Although anti-IL-12/23antibody has been found to have a therapeutic effect on psoriasis in clinical trials, there are several problems. First problem involves cost. In general, treatments using antibodies have a great burden on patients and the healthcare system in terms of cost. Second problem is the method of administration. Since treatments with antibodies require intravenous administration, burdens on patients, for example, hospital visits for every administration, is considered to be great. Hence, development of a novel inexpensive agent that can orally be administered for suppressing IL-12 or IL-23 function is considered to be necessary.
One method for suppressing IL-12 and IL-23 functions without using an antibody may be a method that suppresses IL-12 and IL-23 productions. Specifically, according to this method, a therapeutic agent is developed that acts on an intracellular target molecule in IL-12- and IL-23-producing cells such as activated macrophages, thereby suppressing IL-12 and IL-23 productions. Since this method allows development of a therapeutic agent such as a low-molecular compound that can orally be administered and that is less expensive than an antibody, it is extremely beneficial for patients.

There have been a number of reports on low-molecular compounds that suppress IL-12/IL-23 productions, but most of these compounds were weak and insufficient in terms of suppressive action. For example, steroid, 1,25-dihydroxy vitamin D3, acetylsalicylic acid (ASA), retinoid, thalidomide, prostaglandin E2, s2 agonist, phosphodiesterase inhibitor and the like have been reported. Thalidomide is likely to have the strongest suppressive action among them, but it is also reported that thalidomide cannot sufficiently suppress IL-12 and IL-23 productions strongly induced by INF-γ.

Meanwhile, a novel low-molecular compound was recently reported that showed an action of IL-12 and/or IL-23 production suppression (hereinafter, also referred to as "IL-12/23 production suppression") (Patent document 1). This compound with an IL-12/23 production suppressive action has been reported that it sufficiently suppresses productions of IL-12 and IL-23 strongly induced by INF-γ at the cell level (Non-patent document 1), that it has been shown to exhibit an anti-inflammatory action in a model animal (Non-patent document 2), that it has been confirmed to be safe by clinical trials in human (Non-patent document 3) and the like. However, its target molecule has not been reported so far and remains unclear.

Although Non-patent document 1 reports that the compounds having an IL-12/23 production suppressive action suppress IL-12 and IL-23 genes at transcription level, it does not suggest the target molecule directly affected by these compounds. In addition, Patent document 1 reports that a transcription factor called c-Rel is involved in the action mechanism of the compound having an IL-12/23 production suppressive action, but there is no evidence of c-Rel being the directly affected target molecule, nor there is any suggestion about a target molecule.

### PRIOR ART DOCUMENTS

### Patent Document

[Patent document 1] US Patent Publication No. 2008-0227114

### Non-patent Documents

[Non-patent document 1] Blood. 2007 Feb 1;109(3):1156-64. Epub 2006 Oct 19
[Non-patent document 2] Arthritis Res Ther. 2008;10(5):R122. Epub 2008 Oct 13
[Non-patent document 3] Inflamm Bowel Dis. 2006 Jul;12(7):558-65

### SUMMARY OF THE INVENTION

### Problem to be Solved by the Invention

Under the above-described circumstances, development of a novel method for screening a therapeutic or prophylactic agent for an inflammatory disease has been desired.

### Means for Solving the Problem

The present inventors found that PIKfyve is involved in IL-12/23 production. Specifically, they found that inhibition of lipid kinase, PIKfyve, can suppress production of IL-12/23, thereby accomplishing the present invention.

More specifically:
1) based on examinations using multiple compounds, they found that the strength of IL-12- and IL-23-production inhibitory activity of mouse macrophage correlates with the strength of PIKfyve inhibitory activity;
2) they found that a compound with a strong PIKfyve inhibitory activity has an effect of suppressing intestinal inflammation in an enterocolitis model mouse; and
3) they found that gene knockdown in mouse macrophage using siRNA designed based on PIKfyve sequence (hereinafter, also referred to as "PIKfyve-siRNA") and siRNA designed based on Vac14 sequence, an activator of PIKfyve (hereinafter, also referred to as "Vac14-siRNA") results in suppression of IL-12 and IL-23 productions.
From these findings, they found out a novel method for screening a therapeutic or prophylactic agent for an inflammatory disease based on the understanding of PIKfyve functions that the presence or absence of a PIKfyve-specific inhibitory activity can be used as an indicator to select a substance having this activity.
Thus, the present invention relates to a screening method and the like below.
[1] A method for screening a prophylactic or therapeutic agent for an inflammatory disease, comprising a step of measuring PIKfyve inhibitory activity of a test substance to identify a substance having this inhibitory activity.
[2] The method according to [1] above, wherein the PIKfyve inhibitory activity is measured by using phosphorylation by ATP or labeled ATP as an indicator.
[3] The method according to either one of [1] and [2] above, wherein the PIKfyve inhibitory activity is measured by using a substrate selected from phosphatidylinositol, phosphatidylinositol 3-phosphate or a derivative thereof, or Rab9 effector p40 or a partial sequence thereof.
[4] The method according to [1] above, wherein the PIKfyve inhibitory activity is measured by using binding between PIKfyve and a probe selected from labeled ATP and PIKfyve inhibitors as an indicator.
[5] The method according to [1] above, wherein the PIKfyve inhibitory activity is caused by an inhibitory activity for Vac14.
[6] The method according to either one of [1] and [5] above, wherein the PIKfyve inhibitory activity is an inhibitory activity against binding between PIKfyve and Vac14.
[7] The method according to any one of [1], [5] and [6] above, wherein the PIKfyve inhibitory activity is measured using PIKfyve or a partial sequence thereof, or Vac14 or a partial sequence thereof as a probe.
[8] The method according to [1] above, wherein the PIKfyve inhibitory activity is measured using cellular vacuolation as an indicator.
[9] The method according to any one of [1] to [8] above, wherein the test substance is selected from a low-molecular compound, an antibody, an antisense oligonucleotide, siRNA and an aptamer.
   In addition, the present invention also relates to the following pharmaceutical agent, therapeutic method and the like.
[10] The method according to [1] above, wherein the inflammatory disease is an IL-12/23-overproduction-related disease.
[11] A prophylactic or therapeutic agent for an inflammatory disease, comprising a PIKfyve inhibitor.
[12] A prophylactic or therapeutic agent for an inflammatory disease, wherein an PIKfyve inhibitor is a substance obtained by the method according to [1] above.
[13] The prophylactic or therapeutic agent according to [12] above, wherein the PIKfyve inhibitor is an antibody for PIKfyve, or an antisense oligonucleotide, siRNA or aptamer for PIKfyve gene or Vac14 gene.
[14] A prophylactic or therapeutic method for an inflammatory disease, comprising a step of administering an effective amount of a PIKfyve inhibitor to a patient.
[15] The prophylactic or therapeutic method according to [12] above, wherein the PIKfyve inhibitor is a substance obtained by the method according to [1] above.
[16] The prophylactic or therapeutic method according to either one of [12] and [13] above, wherein the PIKfyve inhibitor is an antibody, or an antisense oligonucleotide, siRNA or aptamer for PIKfyve gene or Vac14 gene.
[17] Use of a PIKfyve inhibitor for producing a prophylactic or therapeutic agent for an inflammatory disease.

### EFFECT OF THE INVENTION

A screening method of the present invention is useful for creating a novel prophylactic/therapeutic agent for an inflammatory disease.

### BRIEF DESCRIPTION OF THE DRAWINGS

[Figure 1] Figure 1 shows inhibitory activities of compounds against various lipid kinases.
[Figure 2] Figure 2 shows correlation between PIKfyve inhibitory activities and mouse peritoneal macrophage IL-12-production inhibitory activity of compounds.
[Figure 3] Figure 3 shows an inflammation suppressive action of a PIKfyve-inhibiting compound in a mouse enterocolitis model.
[Figure 4] Figure 4 shows IL-12-production suppressive actions by PIKfyve and Vac14 knockdown.

### EMBODIMENTS FOR CARRYING OUT THE INVENTION

Hereinafter, terms used herein will be described.
"PIKfyve" is one type of lipid kinases and is an enzyme having an activity of phosphorylating position 5 in phosphatidylinositol or phosphatidylinositol 3-phosphate. In addition, PIKfyve also has a protein kinase activity and phosphorylates serine residues of p40 protein, an effector for Rab9 protein. PIKfyve is called Fab1 in a budding yeast and PIKfyve, PIPSK3, p235 or the like in a mammal.
Examples of the amino acid sequence of PIKfyve include, but not limited to, SEQ ID NOS:2 and 4, and Genbank Accession Nos: NP_055855.1, Q9Y2I7 and AAR19397.
Examples of the nucleotide sequence of PIKfyve gene include, but not limited to, SEQ ID NOS:1 and 3, and Genbank Accession Nos: NM_015040, AY457063 and BC032389 (cDNA).
PIKfyve comprises a homolog, a mutant, a variant and the like of PIKfyve that retains an activity of phosphorylating position 5 in phosphatidylinositol and phosphatidylinositol 3-phosphate or an ability to phosphorylate the serine residues of p40 protein.
Such a homolog, mutant or variant comprises a protein with an amino acid sequence having an identity of, for example, 80% or higher, 85% or higher, 90% or higher, 95% or higher, 97% or 99% with SEQ ID NO:2 or 4, and a protein with an amino acid sequence having, for example, 1 to 20, 1 to 15, 1 to 10, 1 to several (for example, 9), 1 to 5, 1 to 3 or 1 to 2 amino acids deleted from, substituted in, inserted into and/or added to the amino acid sequence represented by SEQ ID NO:2 or 4.

PIKfyve gene can be hybridized with sequences represented by SEQ ID NO:1 or 3, GenBank Accession No. AY457063 or BC032389 or the like under stringent conditions, and comprises DNA that retains PIKfyve activity (the activity of phosphorylating position 5 in phosphatidylinositol or phosphatidylinositol 3-phosphate, the ability to phosphorylate the serine residues of p40 protein or the like).
A partial sequence of PIKfyve may be any sequence containing all or part of CCT domain of PIKfyve that has been reported to have at least a binding ability with Vac14, for example, a sequence containing amino acid residues 346-1134 of human PIKfyve or a part thereof. The length is preferably that of a peptide having 5 to 1000, and preferably 501 to 1000 amino acid residues.

"Vac14" is an activator protein of PIKfyve, which forms a PIKfyve-Vac14 complex by binding to PIKfyve via the HEAT-repeat motifs on the N-terminal side. This complex formation is considered to be necessary for intracellular activation of PIKfyve. Vac14 is called Vac14 in a budding yeast, and Vac14, ArPIKfyve, TAX1BP2 or the like in a mammal.
Examples of the amino acid sequence of Vac14 include, but not limited to, the amino acid sequences represented by SEQ ID NOS:6 and 8, and Genbank Accession Nos: NP_060522.3,AAI25110.1 and AAI25109.1.
Examples of the nucleotide sequence of Vac14 gene include DNAs represented by SEQ ID NOS:5 and 7.
Vac14 comprises a homolog, a mutant, a variant and the like of Vac14 that retains the ability to form a PIKfyve-Vac14 complex.
Such a homolog, mutant or variant comprises a protein with an amino acid sequence having an identity of, for example, 80% or higher, 85% or higher, 90% or higher, 95% or higher, 97% or 99% with SEQ ID NO:6 or 8, and a protein with an amino acid sequence having, for example, 1 to 20, 1 to 15, 1 to 10, 1 to several (for example, 9), 1 to 5, 1 to 3 or 1 to 2 amino acids deleted from, substituted in, inserted into and/or added to the amino acid sequence represented by SEQ ID NO:6 or 8.
Vac14 gene can be hybridized with sequences represented by SEQ ID NO:5 or 7, GenBank Accession No. BC125109.1 or NM_018052 or the like under stringent conditions, and comprises DNA that retains the ability to form a PIKfyve-Vac14 complex.
A partial sequence of Vac14 is a sequence containing 1st to 11th HEAT-repeat motifs on the N-terminal side of Vac14 or a part thereof that has been reported of its binding ability with PIKfyve, which is preferably a peptide of 5 to 500, or 100 to 500 amino acid residues.
p40 interacts with p40-PIKfyve. PIKfyve interaction and subsequent PIKfyve-catalyzed p40 phosphorylation anchor p40 to discrete membranes to facilitate late endosome-to-trans-Golgi transport. Moreover, p40 can bind to Rab9-GTP and stimulate endosome-to-trans-Golgi transport (see Diaz et al., J. Cell Biol. 138: 283-90, 1997, etc.). p40 is an effector for Rab9 and also called Rab9 effector p40, or Rab9p40.

Examples of the amino acid sequence of p40 include, but not limited to, SEQ ID NO:10, and Genbank Accession Nos: NP_005824 and CAG33118. p40 comprises a homolog, a mutant, a variant and the like of p40.
Examples of the nucleotide sequence of p40 gene include, but not limited to, SEQ ID NO:9, and Genbank Accession Nos: NM_015040, NM_005833.2 and CR456837.1 (cDNA).
p40 comprises a homolog, a mutant, a variant and the like of PIKfyve-phosphorylated p40.
Such a homolog, mutant or variant comprises a protein with an amino acid sequence having an identity of, for example, 80% or higher, 85% or higher, 90% or higher, 95% or higher, 97% or 99% with SEQ ID NO:10, and a protein with an amino acid sequence having, for example, 1 to 20, 1 to 15, 1 to 10, 1 to several (for example, 9), 1 to 5, 1 to 3 or 1 to 2 amino acids deleted from, substituted in, inserted into and/or added to the amino acid sequence represented by SEQ ID NO:10.

p40 gene can hybridize with the sequence represented by SEQ ID NO:9, or GenBank Accession No. NM_005833.2 or CR456837.1 under stringent conditions, and comprises those that can be phosphorylated by PIKfyve.
A partial sequence of p40 is a sequence including the Ser residues contained in p40 protein, which is a peptide containing preferably 5 to 300, and more preferably 100 to 300 amino acid residues.

Here, the phrase "DNA that hybridizes under stringent conditions" refers to DNA that can be obtained by employing a colony hybridization technique, a plaque hybridization technique or a Southern hybridization technique using whole or a part of DNA containing a nucleotide sequence complementary to the nucleotide sequence represented by SEQ ID NO:1, 3, 5, 7 or 9 as a probe. A hybridization technique that can be employed may be, for example, a technique described in Molecular Cloning 3rd Ed., Current Protocols in Molecular Biology, John Wiley & Sons 1987-1997 or the like.
As used herein, the term "stringent conditions" may refer to any of lowly stringent conditions, mildly stringent conditions and highly stringent conditions. For example, "lowly stringent conditions" may be 5 X SSC, 5 X Denhardt solution, 0.5% SDS and 50% formamide at 32°C. Furthermore, "mildly stringent conditions" may be, for example, 5 X SSC, 5 X Denhardt solution, 0.5% SDS and 50% formamide at 42°C. "Highly stringent conditions" may be, for example, 5 X SSC, 5 X Denhardt solution, 0.5% SDS and 50% formamide at 50°C. Under these conditions, higher temperature is expected to result in efficient acquirement of a polynucleotide (for example, DNA) with higher homology. However, multiple factors are considered to influence the stringency of hybridization, including temperature, probe concentration, probe length, time, ionic strength, salt concentration and the like, which can appropriately be selected by those skilled in the art to realize similar stringency.
In a case where a commercially available kit is used for hybridization, for example, AlkphosDirect Labelling Reagents (produced by GE Healthcare Japan) can be used. In this case, in accordance with the protocol attached to the kit, incubation with a labeled probe is carried out overnight, then the membrane is washed with a primary wash buffer containing 0.1% (w/v) SDS under the conditions of 55°C, and subsequently the hybridized polynucleotide (for example, DNA) can be detected.
Other than these, examples of hybridizable DNAs include DNAs having an identity of 80% or higher, 81% or higher, 82% or higher, 83% or higher, 85% or higher, 90% or higher, 95% or higher, 97% or higher, or 99% or higher with a polynucleotide coding for the amino acid sequence represented by SEQ ID NO:2, 4, 6, 8 or 10 when calculated with a homology search software such as FASTA and BLAST using default parameters.
Identity of an amino acid sequence or a nucleotide sequence can be determined using algorithm BLAST by Karlin and Altschul (Proc. Natl. Acad. Sci. USA, 87, 2264-2268, 1990; Proc. Natl. Acad. Sci. USA, 90, 5873, 1993). Programs called BLASTN and BLASTX based on the algorithm of BLAST have been developed (Altschul SF, et al: J. Mol. Biol. 215: 403, 1990). When BLASTN is used to analyze a nucleotide sequence, parameters are, for example, score = 100 and wordlength = 12. When BLASTX is used to analyze an amino acid sequence, parameters are, for example, score = 50 and wordlength = 3. When BLAST and Gapped BLAST programs are used, default parameters for each program are used.

A "PIKfyve inhibitory activity" refers to a degree of the effect to reduce or annihilate the enzymatic activity ofPIKfyve. Specifically, a PIKfyve inhibitory activity can be determined by measuring a degree of reduction in phosphorylation or binding by a test substance, namely, phosphorylation of position 5 in phosphatidylinositol or phosphatidylinositol 3-phosphate, phosphorylation of Rab9 effector p40, binding between PIKfyve and ATP or the like, caused by the enzymatic activity of PIKfyve. Measurement of a PIKfyve inhibitory activity is described, for example, in EMBO Rep. 2008 Feb;9(2):164-70. Epub 2008 Jan 11.
PIKfyve inhibitory activity resulting from Vac14 inhibitory activity means that the enzymatic activity ofPIKfyve is reduced or annihilated by inhibiting the function of Vac14. Inhibition of Vac14 function include eliminating or reducing the function by altering the structure of Vac14, or disturbing the formation of a PIKfyve-Vac14 complex by inhibiting binding between PIKfyve and Vac14, thereby reducing or annihilating the enzymatic activity ofPIKfyve. Preferably, it is inhibition of binding between PIKfyve and Vac14.
The term "cellular vacuolation" refers to a change in cell morphology resulting from inhibition of intracellular vesicular transport due to PIKfyve inhibition or Vac14 inhibition. By measuring the degree of cellular vacuolation, inhibitory activity of PIKfyve or inhibitory activity of Vac14 can be determined.

Examples of the "PIKfyve inhibitor" include the above-described low-molecular compound having a PIKfyve inhibitory activity with IC₅₀ of 1mM or less, an antibody for PIKfyve, or an antisense oligonucleotide, siRNA or aptamer for PIKfyve gene or Vac14 gene.
The "PIKfyve inhibitor" also includes a derivative of a PIKfyve inhibitor. A derivative of a PIKfyve inhibitor refers to a substance that can be obtained by derivatizing a PIKfyve inhibitor.
Derivatization refers to virtual or actual synthesis of a compound obtained by replacing a certain atom or group in a lead compound with other atom or group, or a compound obtained through addition reaction on a lead compound. For example, the lead compound may be a substance obtained by a screening method of the present invention.

Derivatization of a PIKfyve inhibitor can be carried out while considering water solubility/lipid solubility, stability, disposition, biological availability, toxicity and other properties of the resulting derivative as appropriate such that it retains inhibitory capacity for PIKfyve.

For example, derivatization of a PIKfyve inhibitor may be carried out based on SBDD (Structure-Based Drug Design) or CADD (Computer-Aided Drug Design). Examples of such design include virtual screening, de novo design, pharmacophore analysis and QSAR (Quantitative Structure Activity Relationship). If conformational information of the protein itself or the target site of the protein is necessary upon such design, conformational information known from a structural analysis technique such as NMR, X-ray crystallographic analysis or radiation analysis, if any, can be used. If conformation is unknown, information obtained by a structural prediction method such as a homology method or Threading method is used. In the case of virtual screening, a program known per se can be used, examples of such program being DOCK (Kuntz, I. D. et al., Science, 1992, 257, 1078), Gold (Jones, G. et al., J. Mol. Biol., 1995, 245, 43), FlexX (Rarey, M. et al., J. Mol. Biol., 1996, 261, 470), AutoDock (Morris, G. M. et al., J. Comput. Chem., 1998, 19, 1639), ICM (Abagyan, R. A. et al., J. Comput. Chem., 1994, 15, 488).

Examples of a test substance used in a method of the present invention include a low-molecular compound, a peptide, a protein, an antibody, an antisense oligonucleotide, siRNA, an aptamer and the like.
A "low-molecular compound" refers to a low-molecular organic compound, which may be produced by using a method known in the art of synthetic organic chemistry. Preferably, it is an organic compound with a molecular weight of 1000 or less. The low-molecular compound also comprises a salt, a hydrate or a solvate thereof. In addition, an available compound library or the like can also be used.
According to the present invention, a "protein" or a "peptide" may be a commercially available product. A peptide can be acquired by appropriately employing a known method such as (1) a chemical synthesis method or (2) a synthesis method using enzymatic reaction. When the number of amino acid residues contained is relatively small, i.e., 2 to 3 residues, a chemical synthesis method is convenient. In the case of chemical synthesis, it may be carried out by synthesizing or semi-synthesizing the oligopeptide using a peptide synthesizer.
An "antibody" may be either a monoclonal antibody or a polyclonal antibody for PIKfyve, Vac14 or p40. A monoclonal antibody can be prepared, for example, according to a method described in J Virology (1992); 66: 5999-6007.
An "antisense oligonucleotide" refers to nucleotides that are complementary to or that can hybridize with a sequence of 5 to 100 consecutive nucleotides in DNA of PIKfyve gene or Vac14 gene, which may be either DNA or RNA, and which may be modified as long as it does not interfere with the function. As used herein, the term "antisense oligonucleotide" not only comprises those whose nucleotides are completely complementary to the corresponding nucleotides constituting a predetermined region of DNA or mRNA, but it may also contain some mismatches as long as the DNA or mRNA can stably hybridize with the oligonucleotide.

An antisense oligonucleotide may be modified. Appropriate modification can render the antisense oligonucleotide to be less biodegradable, and more stable for inhibiting PIKfyve. Examples of such modified oligonucleotide include antisense oligonucleotides having modification types such as S-oligo type (phosphorothioate type), C-5 thiazole type, D-oligo type (phosphodiester type), M-oligo type (methylphosphonate type), peptide nucleic acid type, phosphodiester bond type, C-5 propynyl pyrimidine type, 2-O-propyl ribose, 2'-methoxyethoxyribose type and the like. Furthermore, an antisense oligonucleotide may be those having at least some of the oxygen atoms constituting the phosphate groups substituted or modified into sulfur atoms. Such an antisense oligonucleotide is particularly superior in nuclease resistance, water solubility, and affinity to RNA. An example of an antisense oligonucleotide having at least some of the oxygen atoms constituting the phosphate groups substituted or modified into sulfur atoms includes an oligonucleotide of S-oligo type.

The number of nucleotides in an antisense oligonucleotide is preferably 50 or less, and more preferably 25 or less. An excessive number of nucleotides increases the labor and the cost for oligonucleotide synthesis and also reduces the yield. At the same time, the number of nucleotides of the antisense oligonucleotide is 5 or more, and preferably 9 or more. If the number of nucleotides is 4 or less, specificity for the target gene will be reduced, which is unfavorable.

An antisense oligonucleotide (or a derivative thereof) can readily be synthesized by an ordinary method, for example, with a commercially available DNA synthesizer (e.g., AppliedBiosystems, etc.). The antisense oligonucleotide can be obtained by a synthetic method such as a solid-phase synthetic method using phosphoramidite, a solid-phase synthetic method using hydrogen phosphonate and the like.

An antisense oligonucleotide is preferably one that corresponds to at least 15 consecutive nucleotides in the nucleotide sequence represented by SEQ ID NO:1, 3, 5 or 7. More preferably, it is an antisense oligonucleotide that contains an initiation codon in the at least 15 consecutive nucleotides described above.
"A derivative of phosphatidylinositol, phosphatidylinositol 3-phosphate" comprises phosphatidylinositol(3) monophosphate (PtdIns(3)P), phosphatidylinositol(3,4) diphosphate (PtdIns(3,4)P2) and the like.

siRNA refers to double-stranded RNA comprising a part of RNA coding for PIKfyve or Vac14 and RNA complementary thereto. Specifically, siRNA (Example) is used that includes a sense strand having the nucleotide sequence represented by SEQ ID NO:11 and an antisense strand having the nucleotide sequence represented by SEQ ID NO:12.
siRNA can be designed and produced based on a sequence of a polynucleotide of the present invention according to a known method (e.g., Nature, vol. 411, page 494, 2001).
siRNA can also be obtained by employing a software such as siRNA Target Finder [http://www.ambion.com/jp/techlib/misc/siRNA_finder.html] (Ambion). For example, the following sequences may be obtained as siRNA for PIKfyve gene or Vac14 gene designed by the above-mentioned software.

PIKfyve 5'→3' each
Sense strand siRNA: GACGUCCCCAACACUGGACtt (SEQ ID NO:13)
Antisense strand siRNA: GUCCAGUGUUGGGGACGUCtt (SEQ ID NO:14)

Sense strand siRNA: CACUGGACUCUGCUAAUGAtt (SEQ ID NO:15)
Antisense strand siRNA: UCAUUAGCAGAGUCCAGUGtt (SEQ ID NO:16)

Sense strand siRNA: UGAUUUGCCUCGAUCUCCUtt (SEQ ID NO:17)
Antisense strand siRNA: AGGAGAUCGAGGCAAAUCAtt (SEQ ID NO:18)

Sense strand siRNA: CAGCAGCCUUUGAGUGGAAtt (SEQ ID NO:19)
Antisense strand siRNA: UUCCACUCAAAGGCUGCUGtt (SEQ ID NO:20)

Sense strand siRNA: AAAGCAGCUUAAUGAGGAAtt (SEQ ID NO:21)
Antisense strand siRNA: UUCCUCAUUAAGCUGCUUUtt (SEQ ID NO:22)

Sense strand siRNA: GGAAAGCAGAACCUACCUUtt (SEQ ID NO:23)
Antisense strand siRNA: AAGGUAGGUUCUGCUUUCCtt (SEQ ID NO:24)

Sense strand siRNA: CCUACCUUUGGAGGUCAUGtt (SEQ ID NO:25)
Antisense strand siRNA: CAUGACCUCCAAAGGUAGGtt (SEQ ID NO:26)

Sense strand siRNA: CGCCUCAAGGAAAUCAUGGtt (SEQ ID NO:27)
Antisense strand siRNA: CCAUGAUUUCCUUGAGGCGtt (SEQ ID NO:28)

Sense strand siRNA: GUUAUGCUCAUUCCACAGAtt (SEQ ID NO:29)
Antisense strand siRNA: UCUGUGGAAUGAGCAUAACtt (SEQ ID NO:30)

Sense strand siRNA: CAUAUGUUAGGACAGAGACtt (SEQ ID NO:31)
Antisense strand siRNA: GUCUCUGUCCUAACAUAUGtt (SEQ ID NO:32)

Vac14 5' →3' each
Sense strand siRNA: CCCCGAGAAGGAUUUCGCGtt (SEQ ID NO:33)
Antisense strand siRNA: CGCGAAAUCCUUCUCGGGGtt (SEQ ID NO:34)

Sense strand siRNA: AAGCGGAAGGUGGCAGCGCtt (SEQ ID NO:35)
Antisense strand siRNA: GCGCUGCCACCUUCCGCUUtt (SEQ ID NO:36)

Sense strand siRNA: AUCAAGCAUGUGAUCCAGAtt (SEQ ID NO:37)
Antisense strand siRNA: UCUGGAUCACAUGCUUGAUtt (SEQ ID NO:38)

Sense strand siRNA: GGAGCUGAUCGAGCCAGUGtt (SEQ ID NO:39)
Antisense strand siRNA: CACUGGCUCGAUCAGCUCCtt (SEQ ID NO:40)

Sense strand siRNA: GCGGAUCUGAGCUCCUAGAtt (SEQ ID NO:41)
Antisense strand siRNA: UCUAGGAGCUCAGAUCCGCtt (SEQ ID NO:42)

Sense strand siRNA: GUUUGACCUGGUGAGCUUCtt (SEQ ID NO:43)
Antisense strand siRNA: GAAGCUCACCAGGUCAAACtt (SEQ ID NO:44)

Sense strand siRNA: AUUAAGAAGAACCCCUCCAtt (SEQ ID NO:45)
Antisense strand siRNA: UGGAGGGGUUCUUCUUAAUtt (SEQ ID NO:46)

Sense strand siRNA: GUUUGCUGAGAUGGCCAACtt (SEQ ID NO:47)
Antisense strand siRNA: GUUGGCCAUCUCAGCAAACtt (SEQ ID NO:48)

Sense strand siRNA: AAGCAUCAAAGAAGUGGCCtt (SEQ ID NO:49)
Antisense strand siRNA: GGCCACUUCUUUGAUGCUUtt (SEQ ID NO:50)

Sense strand siRNA: GCUCCUGGAGGUCAGAGGCtt (SEQ ID NO:51)
Antisense strand siRNA: GCCUCUGACCUCCAGGAGCtt (SEQ ID NO:52)

An aptamer is a 20 to 60-mer oligonucleotide (RNA/DNA) that specifically binds to a target protein, which has an ability to penetrate into a pocket (indent) of a protein to form a stable three-dimensional conformation, thereby inhibiting the function. A nucleotide sequence of an aptamer can be determined by a procedure called SELEX (Systematic Evolution of Ligands by Exponential enrichment) method.
First, sequences that bind to a target protein are selected from a group of nucleic acids having random nucleotide sequences and amplified. Subsequently, these selection and amplification are repeated to search for a sequence having high specificity.
An "inflammatory disease" refers to a condition or a disease associated with inflammation, and in particular an IL-12- or IL-23-production-meditated disease comprising an autoimmune disease. Specifically, examples include multiple sclerosis, systemic sclerosis, sepsis, myasthenia gravis, autoimmune neurological disease, Guillain-Barre syndrome, autoimmune uveitis, autoimmune hemolytic anemia, malignant anemia, autoimmune thrombocytopenia, temporal arteritis, antiphospholipid syndrome, vasculitis, Wegener's granulomatosis, Behcet's disease, psoriasis, psoriatic arthritis, herpetic dermatitis, pemphigus vulgaris, vitiligo, Crohn's disease, ulcerative colitis, interstitial pulmonary fibrosis, myelofibrosis, hepatic fibrosis, myocarditis, autoimmune thyroid disease, thyroiditis, primary biliary cirrhosis, autoimmune hepatitis, immune-mediated diabetes mellitus, Graves' disease, Hashimoto's thyroiditis, autoimmune oophoritis and orchitis, autoimmune adrenalitis, rheumatoid arthritis, juvenile rheumatoid arthritis, systemic lupus erythematosus, scleroderma, polymyositis, dermatomyositis, spondyloarthropathy, ankylosing spondylitis, Sjogren's syndrome, connective tissue disease, graft-versus-host disease, ischemic vascular disorder and Castleman's disease.
Among them, preferable examples include Crohn's disease, ulcerative colitis, multiple sclerosis, psoriasis, psoriatic arthritis, primary biliary cirrhosis, autoimmune uveitis and rheumatoid arthritis.
Here, diseases mediated by IL-12 or IL-23 production include, for example, IL-12/23-overproduction-related diseases where overproduction of IL-12 or IL-23 is involved in the pathological condition, and diseases associated with mutation of the receptor mechanism of IL-12 or IL-23. Examples of such IL-12/23-overproduction-related disease include multiple sclerosis, systemic sclerosis, sepsis, myasthenia gravis, autoimmune neurological disease, Guillain-Barre syndrome, autoimmune uveitis, autoimmune hemolytic anemia, malignant anemia, autoimmune thrombocytopenia, temporal arteritis, antiphospholipid syndrome, vasculitis, Wegener's granulomatosis, Behcet's disease, psoriasis, psoriatic arthritis, herpetic dermatitis, pemphigus vulgaris, vitiligo, Crohn's disease, ulcerative colitis, interstitial pulmonary fibrosis, myelofibrosis, hepatic fibrosis, myocarditis, autoimmune thyroid disease, primary biliary cirrhosis, autoimmune hepatitis, immune-mediated diabetes mellitus, autoimmune oophoritis and orchitis, autoimmune adrenalitis, rheumatoid arthritis, juvenile rheumatoid arthritis, systemic lupus erythematosus, scleroderma, polymyositis, dermatomyositis, spondyloarthropathy, ankylosing spondylitis, Sjogren's syndrome and graft-versus-host disease. In particular, psoriasis (Lancet. 2008 May 17;371(9625):1665-74), Crohn's disease and ulcerative colitis (Gastroenterology. 2008 Oct;135(4):1130-41.Epub 2008 Jun 17, Am J Gastroenterol. 2008 Mar;103(3):621-7. Epub 2007 Nov 28) are favorable.
An example of a disease associated with mutation of the receptor mechanism of IL-23 includes Crohn's disease (Nat Genet. 2008 Aug;40(8):955-62. Epub 2008 Jun 29).

Clinical effects of an IL-12/23 inhibitor on psoriasis or psoriatic arthritis are reported in Lancet. 2008 May 17;371(9625): 1665-74 and Lancet. 2009 Feb 21; 373(9664): 633-40. Epub 2009 Feb 11. In addition, J Invest Dermatol. 2009 Feb; 129(2): 355-8. Epub 2008 Sep 18, Arthritis Rheum. 2008 Dec;58(12):3705-9 and else also describe relevance between these diseases and IL-12 and/or IL-23. Furthermore, relevance with rheumatoid arthritis is reported in Ann Rheum Dis. 2008 Feb;67(2):248-50. Epub 2007 Jul 2. Relevance with Crohn's disease and ulcerative colitis is reported in Gastroenterology. 2008 Oct;135(4):1130-41. Epub 2008 Jul 17 and Am J Gastroenterol. 2008 Mar;103(3):621-7. Epub 2007 Nov 28. Relevance with multiple sclerosis is reported in Expert Rev Neurother. 2009 Mar;9(3):319-21. Relevance with primary biliary cirrhosis is reported in N Engl J Med. 2009 Jun 11;360(24):2544-55. Epub 2009 May 20.
Moreover, relevance with vasculitis (Hum Immunol. 2006 Sep;67(9):735-40. Epub 2006 Jul 20.), ankylosing spondylitis (Curr Opin Rheumatol. 2009 Jul;21(4):318-23.), autoimmune thyroiditis (J Clin Endocrinol Metab. 2008 Mar;93(3):1077-81. Epub 2007 Dec 11.), systemic sclerosis (J Rheumatol. 2009 Nov 16.), Behcet's disease (J Invest Dermatol. 2006 Jul;126(7): 1534-40. Epub 2006 Mar 2.), immune-mediated diabetes mellitus (Nat Genet. 2001 Feb;27(2):218-21.), graft-versus-host disease (Biol Blood Marrow Transplant. 2009 Dec; 15(12):1571-7. Epub 2009 Oct 1.) and Castleman's disease (Int J Hematol. 2007 Apr;85(3):207-11.) is also reported in the respective documents.

Hereinafter, a screening method of the present invention will be described.
According to a screening of the present invention, PIKfyve inhibitory activity can be calculated using phosphorylation of position 5 in phosphatidylinositol or phosphatidylinositol 3-phosphate due to enzymatic activity of PIKfyve or phosphorylation of Rab9 effector p40, as an indicator. Alternatively, it can also be calculated by measuring a change in the binding amount between PIKfyve and Vac14, the binding amount between PIKfyve and ATP, cellular vacuolation or the like upon addition of a substance to be evaluated.
When phosphorylation is used as an indicator, a phosphorylation assay is employed whereas when an inhibitory activity against binding is to be assessed, measurement can be carried out by a binding assay using various probes.
When cellular vacuolation is used as an indicator, it may be measured by an assay for morphological change using High Content Screening.

### (1) Phosphorylation assay

Using PIKfyve or a partial sequence of PIKfyve as an enzyme, and phosphatidylinositol or phosphatidylinositol 3-phosphate or a derivative thereof or Rab9 effector p40 or a partial sequence thereof as a substrate, the substrate is phosphorylated with PIKfyve in the presence of ATP or labeled ATP and a substance to be evaluated is added to the reaction system for reaction. Subsequently, a substance having an inhibitory activity against PIKfyve is selected by using the labeled ATP bound to the substrate or the phosphate group as an indicator, thereby accomplishing screening.
As labeled ATP, ³²P-labeled form can favorably be used. An example of ³²P-labeled ATP includes [γ-³²P] ATP (GE Healthcare Japan, Daiichi Kagaku).
An inhibitory activity can be measured according to a method in which an intracellular enzymatic activity is measured using a highly expressing cell or a culture cell of PIKfyve, a method using an *in vitro* activity measurement system, or the like.
For example, the method using a highly expressing cell of PIKfyve may be a method in which a human lymphocyte-derived Jurkat cell is suspended in a lysis buffer or the like by sonication and the supernatant resulting from centrifugation is used as a cell extract containing PIKfyve.
An *in vitro* measurement system may be a system in which human or mouse PIKfyve is expressed in an insect cell and purified, for example, by a known method using a baculovirus vector or the like, and the purified PIKfyve is used to measure the enzymatic activity.
As p40 or a partial sequence thereof, at least a part or whole sequence of the above-described sequence can be used, which is preferably a peptide containing Ser residues of p40 and a peptide containing preferably 5 to 300, and more preferably 100 to 300 amino acid residues.
Specifically, a method that uses p40 as a substrate can be realized by preparing p40 by expressing GST-p40 by a known method using an *E. coli* expression system, purifying the resultant by a method using Glutathione-agarose beads, and using the resultant as a substrate to perform *in vitro* activity measurement with purified PIKfyve. The *E.coli* expression system may be pET Expression System (Takara Bio) or the like while Glutathione-agarose beads may be Immobilized Glutathione (Thermo Scientific) or the like.

### (2) Binding assay

Any conventionally known method can be applied that uses PIKfyve as an enzyme for examining the presence or absence of binding or the presence or absence of disassociation between the substances. For example, a substance to be evaluated is added to the reaction system for reaction in the presence of PIKfyve, labeled ATP and a probe selected from PIKfyve inhibitors. Subsequently, a substance having an inhibitory activity against PIKfyve binding is selected through a step of judging the inhibitory activity of the substance to be evaluated against PIKfyve binding using the labeled ATP bound to PIKfyve as an indicator, thereby accomplishing screening.
A probe selected from PIKfyve inhibitors may be, for example, a low-molecular compound having a PIKfyve inhibitory activity, an antibody for PIKfyve, an antisense oligonucleotide, siRNA or aptamer for PIKfyve gene or Vac14 gene. For example, compounds indicated herein in Synthesis Examples 1-3 can be used.
Specifically, in order to screen for a compound that inhibits binding between a probe and PIKfyve, first, a cell or a cell membrane fraction containing PIKfyve is suspended in a buffer suitable for screening to prepare a PIKfyve sample. The buffer may be any buffer such as a phosphate buffer or Tris-hydrochloric buffer with pH 4-10 (preferably, pH 6-8) as long as it does not inhibit the binding between the probe and PIKfyve. In addition, for the purpose of reducing non-specific binding, a surfactant such as CHAPS, Tween-80, deoxycholate or digitonin may be added to the buffer. Moreover, for the purpose of preventing degradation of the receptor or the ligand by protease, a protease inhibitor such as leupeptin, PMSF, E-64 (Peptide Institute) or pepstatin can also be added. To 0.01 to 10 ml of the receptor solution, a constant amount (5000 to 500000 cpm) of the labeled probe is added, and at the same time 10⁻⁴ M to 10⁻¹⁰ M test compound is allowed to coexist. In order to find out the non-specific binding amount (NSB), a reaction tube added with an overly excessive amount of unlabeled probe is also prepared. The reaction is carried out at about 0 to 50°C, preferably about 4 to 37°C for about 20 minutes to 24 hours, preferably about 30 minutes to 3 hours. After the reaction, the resultant is filtrated by glass fiber filter paper or the like, and washed with an appropriate amount of the same buffer. Subsequently, radioactivity remaining on the glass fiber filter paper is counted with a liquid scintillation counter or a γ-counter. Where the count (B0-NSB) resulting from subtracting the non-specific binding amount (NSB) from the count without any antagonistic substance (B0) is assumed to be 100%, a test compound whose specific binding amount (B-NSB) is, for example, 50% or less is selected as a candidate substance having an antagonistic inhibitory capacity.

### (3) PIKfyve-Vac14 binding assay

Similarly, any known method can be used that uses PIKfyve or a partial sequence thereof to measure an activity to inhibit binding with Vac14 or a partial sequence thereof. For example, where PIKfyve or a partial sequence thereof is used as a probe and Vac14 or a partial sequence is added as a probe, a substance to be evaluated is added to the reaction system for reaction. Subsequently, a substance having a PIKfyve inhibitory activity is selected through a step of judging an inhibitory activity against binding between PIKfyve and Vac14 by employing any conventionally known method using binding between PIKfyve or a partial sequence thereof and Vac14 or a partial sequence thereof as an indicator.
As a probe of PIKfyve or a partial sequence thereof, DNA with a chain length of at least 15 bp having at least a part or whole PIKfyve sequence (or a complementary sequence thereof) can be used. A preferable example may be a sequence of 1038 bp to 3402 bp, or a part thereof containing CCT domain of human PIKfyve gene that has been reported to have a binding ability to Vac14. Preferably, the length is that of a partial sequence having 15 to 3000, and preferably 1503 to 3000 nucleotides.
As a probe of Vac14 or a partial sequence thereof, DNA with a chain length of at least 15 bp having at least a part or whole Vac14 sequence (or a complementary sequence thereof) can be used. A preferable example may be a gene sequence containing 1st to 11th HEAT-repeat motifs on the N-terminal side of Vac14 gene that has been reported to have a binding ability to PIKfyve. The length of the partial sequence is preferably 15 to 1500, and preferably 300 to 1500 nucleotides.
Specifically, yeast Two-Hybrid method described in Fields, S & Song, O. (1989) Nature 340(6230):245-246 can be exemplified. For example, in order to carry out PIKfyve-Vac14 binding assay, first, a recombinant gene in which PIKfyve gene is fused to the DNA-binding domain of transcription factor Gal4 and a recombinant gene in which Vac14 gene is fused to the transcription activated domain of Ga14 are prepared and transferred into a yeast cell for measurement carried out by a reporter assay according to yeast Two-Hybrid method. Matchmaker Gold Two-Hybrid System (Takara Bio) can be used for the yeast Two-Hybrid method.
Another exemplary method is Alpha Screen method described in Eglen RM, Reisine T, Roby P, et al, Curr Chem Genom. 2008; 1:2-10. Specifically, PIKfyve-Vac14-binding assay may be Alpha screen method in which PIKfyve protein tagged with biotin and Vac14 protein tagged with GST tag sequence are prepared and mixed with streptavidin-bound donor beads and Anti-GST IgG-bound acceptor beads to perform a chemically amplified luminescence proximity homogeneous assay. The donor beads and the acceptor beads may be Streptavidin-coated Donor Beads (Perkin Elmer) and Anti-GST IgG conjugated Acceptor Beads (Perkin Elmer), respectively.

### (4) Assay for change in cell morphology

A cell expressing PIKfyve is used to apply any conventionally known method for examining the presence or absence of change in cell morphology. A specific example includes a method reported in Nat Cell Biol. 2010 Aug;12(8):747-57.
For example, a substance to be evaluated is added to a reaction system containing a PIKfyve-expressing cell. Subsequently, a substance having a PIKfyve inhibitory activity is selected through a step of measuring the change in cell morphology using High Content Screening, thereby accomplishing screening.

A "prophylactic or therapeutic agent" according to the present invention refers to a substance obtained by the above-defined screening method of the present invention, namely, a pharmaceutical composition comprising any of a "low-molecular compound", an "antibody", an "antisense oligonucleotide", "siRNA" or an "aptamer" as an effective component together with a pharmaceutically acceptable carrier.
These pharmaceutical compositions of the present invention are useful as therapeutic agents for the above-described inflammatory diseases.
Here, examples of the "pharmaceutically acceptable carrier" include an excipient, a diluent, a filler, a disintegrant, a stabilizer, a preserving agent, a buffer, an emulsifier, an aromatic agent, a coloring agent, a sweetening agent, a thickening agent, a flavoring agent, a solubilizing aid or other additives. By using one or more of such carriers, a pharmaceutical composition in a dosage form of a tablet, pill, powdered agent, granules, an injectable agent, a liquid agent, a capsule, elixir, lozenge, suspension, emulsion, syrup or the like can be prepared. These pharmaceutical compositions can be administered orally or parenterally. Other dosage forms for parenteral administration include a liquid agent for external application, suppository for enteric administration and pessary formulated by ordinary methods, which contain one or more active substances.

Although a dosage amount differs according to the age, sex, weight and condition of the patient, therapeutic effect, administration method, treating time and the type of an active component (the above-described polypeptide, antibody or the like) contained in the pharmaceutical composition, a single dosage can generally be in a range of 10 µg to 1000 mg (alternatively, 10 µg to 500 mg) per adult. However, since a dosage amount may vary according to various conditions, a dosage amount may be sufficient in an amount less than the above-mentioned dosage amount, or a dosage amount required may exceed the above-mentioned range.

Particularly in the case of an injectable agent, it may be produced by dissolving or suspending the effective component in a non-toxic pharmaceutically acceptable carrier such as physiological saline or a commercially available injectable distilled water to a concentration of 0.1 µg to 10 mg/ml carrier. The thus-produced injectable agent can be administered, once or several times a day, for a single dosage of 1 µg to 100 mg and preferably 50 µg to 50 mg per kg weight of a human patient who needs treatment. Exemplary administration forms include medically appropriate administration forms such as intravenous injection, subcutaneous injection, intradermal injection, intramuscular injection or intraperitoneal injection. Preferably, it is intravenous injection.

In some cases, an injectable agent can also be prepared as a non-aqueous diluent (for example, propylene glycol, polyethylene glycol, a vegetable oil such as olive oil, and alcohol such as ethanol), suspension or emulsion.

Sterilization of such an injectable agent can be carried out by filtration sterilization by passing through a bacteria-retaining filter, by blending a disinfectant or by irradiation. An injectable agent can be produced in a form that can be prepared before use. Specifically, it can be made into a sterile solid composition by lyophilization or the like, which can be dissolved in sterile injectable distilled water or other solvent for use.

### EXAMPLES

Hereinafter, the present invention will specifically be described by means of examples. The scope of the present invention is not limited to these examples.

### [Synthesis Example 1]

### Synthesis of Compound 1: (N-(3-methyl-benzylidene)-N'-(7-morpholine-4-yl-2-pyridine-4-yl-pyrazolo[1,5-a]pyrimidine-5-yl)-hydrazine)

### (Step1) 5-pyridine-4-yl-2H-pyrazole-3-yl-amine

To a THF (10 mL) solution containing 18-crown-6 (875 mg, 3.31 mmol) and tert-butoxy potassium (1M THF solution, 36.4 mL, 36.4 mmol), acetonitrile (2.09 mL, 39.7 mmol) was added at 60°C and agitated for 5 minutes. To this solution, ethyl isonicotinate (5.00 g, 33.1 mmol) was added and agitated for 30 minutes. This suspension was filtrated and the solid was washed with diethyl ether.
The resulting solid was suspended in ethanol (60 mL), added with concentrated hydrochloric acid in ice (3.0 mL) and methyl carbazate (3.61 g, 39.7 mmol), and agitated at room temperature for 20 hours. After agitation, potassium carbonate (2.74 g, 19.8 mmol) was added to this suspension and agitated at 90°C for an hour. The reaction solution was vacuum-concentrated, diluted with water, and subjected to extraction with ethyl acetate. The extracts were combined and dried with anhydrous sodium sulfate, and then the solvent was distilled away. The resulting solid was washed thoroughly with diethyl ether, thereby obtaining the title compound (1.33 g, 25%).
¹H-NMR (300 MHz, DMSO): δ 4.65-5.16 (br, 2H), 5.81 (br, 1H), 7.59-7.61 (m, 2H), 8.50-8.52 (m, 2H), 11.80 (br, 1H); MS (ESI) m/z 161 (M+H)+.

### (Step2) 5,7-dichloro-4-yl-2-pyridine-4-yl-pyrazolo[1,5-a]pyrimidine

To an ethanol solution (20 mL) containing 5-pyridine-4-yl-2H-pyrazole-3-yl-amine (1.29 g, 8.05 mmol), sodium ethoxide (1.21 g, 17.7 mmol) and diethyl malonate (1.34 mL, 8.86 mmol) were added and agitated for 9 hours while heating under reflux. Subsequent to agitation, the reaction solution was filtrated and the solid was washed with diethyl ether.
Phosphoryl chloride in ice (15 mL) was added to the resulting solid, and the suspension was agitated for 4 hours while heating under reflux. Phosphoryl chloride was distilled away from this reaction solution, ethanol in ice was added to the residue and agitated for 15 minutes. After concentrating the reaction solution, the residue was purified by silica-gel column chromatography (methanol/methylene chloride = 1:50 to 1:20), thereby obtaining the title compound (380 mg, 18%).
¹H-NMR (300 MHz, DMSO): δ 7.79 (s, 1H), 7.84 (s, 1H), 8.41 (d, 2H, J=6.6 Hz), 8.97 (d, 2H, J=6.6 Hz); MS (ESI) m/z 265 (M+H)+.

### (Step3) 5-chloro-7-morpholine-4-yl-2-pyridine-4-yl-pyrazolo[1,5-a]pyrimidine

5,7-dichloro-4-yl-2-pyridine-4-ylpyrazolo[1,5-a]pyrimidine (380 mg, 1.43 mmol) was dissolved in 1,4-dioxane (3 mL), added with morpholine (249 µL, 2.86 mmol) and agitated at room temperature for an hour. After reaction, the suspension was concentrated, and the residue was diluted with water and subjected to extraction with ethyl acetate. The extracts were combined and dried with anhydrous sodium sulfate, and then the solvent was distilled away. The resulting solid was washed with diethyl ether, thereby obtaining the title compound (306 mg, 68%).
¹H-NMR (300 MHz, DMSO): δ 3.83-3.85 (m, 4H), 3.89-3.91 (m, 4H), 6.50 (s, 1H), 7.35 (s, 1H), 8.21 (d, 2H, J=6.2 Hz), 8.81 (d, 2H, J=6.2 Hz); MS (ESI) m/z 316 (M+H)+.

### (Step 4) N-(3-methyl-benzylidene)-N'-(7-morpholine-4-yl-2-pyridine-4-yl-pyrazolo[1,5-a]pyrimidine-5-yl)-hydrazine (APY0201)

5-chloro-7-morpholine-4-yl-2-pyridine-4-yl-pyrazolo[1,5-a]pyrimidine (84.0 mg, 0.266 mmol) was suspended in 1,4-dioxane (2 mL), added with hydrazine monohydrate (129 µL, 2.66 mmol) and agitated for 10 hours while heating under reflux. After agitation, the reaction solution was diluted with water and subjected to extraction with ethyl acetate. The extracts were combined and dried with anhydrous sodium sulfate, and then the solvent was distilled away.
The resulting residue was suspended in ethanol (2 mL), added with acetic acid (5.0 µL, 0.087 mmol) and 3-methylbenzaldehyde (31.3 µL, 0.266 mmol), and agitated at room temperature for an hour. After agitation, the suspension was filtrated and the resulting solid was purified by reversed-phase HPLC followed by desalination, thereby obtaining the title compound (36.2 mg, 2-step yield 33%).
¹H-NMR (300 MHz, DMSO): δ2.35 (s, 3H), 3.71-7.75 (m, 4H), 3.87-3.90 (m, 4H), 6.36 (s, 1H), 6.74 (s, 1H), 7.18 (d, 1H, J=7.6 Hz), 7.32 (t, 1H, J=7.6 Hz), 7.48 (s, 1H), 7.53 (d, 1H, J=7.6 Hz), 7.90 (d, 2H, J=4.8 Hz), 8.04 (s, 1H), 8.64 (d, 2H, J=4.8 Hz), 11.27 (s, 1H); MS (ESI) m/z 414 (M+H)+.

### [Synthesis Example 2]

### Synthesis of Compound 2: N-(1H-indole-6-ylmethylene)-N'-(7-morpholine-4-yl-2-pyridine-4-yl-pyrazolo[1,5-a]pyrimidine-5-yl)-hydrazine

5-chloro-7-morpholine-4-yl-2-pyridine-4-ylpyrazolo[1,5-a]pyrimidine (70 mg, 0.22 mmol) was suspended in ethanol (3.0 mL), added with acetic acid (6.3 µL, 0.11 mmol) and 1H-indole-6-carbaldehyde (35 mg, 0.24 mmol), and agitated at room temperature for 16 hours. This reaction solution was filtrated and washed with ethanol and then with diethyl ether, thereby obtaining the title compound (45 mg, yield 10%). ¹H-NMR (300 MHz, DMSO-d6) ; δ3.48 (bs, 4H), 3.91 (bs, 4H), 6.40 (s, 1H), 6.47 (s, 1H), 6.73 (s, 1H), 7.42-7.50 (m, 2H), 7.58-7.64 (m, 2H), 7.92 (d, 2H, J=6 Hz), 8.17 (s, 1H), 8.66 (d, 2H, J=6.3 Hz), 11.1 (s, 1H), 11.3 (s, 1H); MS (ESI) m/z 439 (M+H)+.

### [Synthesis Example 3]

### Synthesis of Compound 3: N-(3-methyl-benzylidene)-N'-(7-morpholine-4-yl-pyrazolo[1,5-a]pyrimidine-5-yl)-hydrazine

### (Step 1) Synthesis of 5-chloro-7-morpholine-4-yl-pyrazolo[1,5-a]pyrimidine

5,7-dichloro-pyrazolo[1,5-a]pyrimidine (400 mg, 2.13 mmol) was dissolved in 1,4-dioxane (8 mL), added with morpholine (372 µL, 4.26 mmol) and agitated at room temperature for 30 minutes. The solvent was distilled away from this reaction mixture. The residue was diluted with water and subjected to extraction with methylene chloride. The extracts were combined and dried with anhydrous sodium sulfate, and then the solvent was distilled away, thereby obtaining the title compound (461 mg, 91%).
¹H-NMR (300 MHz, CDCl3): δ 3.77-3.80 (m, 4H), 3.93-3.96 (m, 4H), 6.07 (s, 1H), 6.50 (d, 1H, J=2.2 Hz), 8.02 (d, 1H, J=2.2 Hz); MS (ESI) m/z 239 (M+H)+.

### (Step 2) Synthesis of N-(3-methyl-benzylidene)-N'-(7-morpholine-4-yl-pyrazolo[1,5-a]pyrimidine-5-yl)-hydrazine

5-chloro-7-morpholine-4-yl-pyrazolo[1,5-a]pyrimidine (52.0 mg, 0.218 mmol) was suspended in ethanol (2 mL) and added with potassium carbonate (33.1 mg, 0.240 mmol) and hydrazine monohydrate (53.0 µL, 1.09 mmol). This suspension was agitated at 150°C under microwave radiation for 15 minutes. This reaction solution was diluted with saturated saline and subjected to extraction with ethyl acetate. The extracts were combined and dried with anhydrous sodium sulfate, and then the solvent was distilled away. The resulting residue was suspended in ethanol (2 mL), added with acetic acid (5.0 µL, 0.087 mmol) and 3-methyl-benzaldehyde (23.2 µL, 0.197 mmol), and agitated at room temperature for an hour. This reaction mixture was filtrated and the resulting solid was washed with methanol, thereby obtaining the title compound (35.1 mg, 2-Step yield 48%).
¹H-NMR (300 MHz, DMSO): δ 2.34 (s, 3H), 3.64-3.67 (m, 4H), 3.80-3.83 (m, 4H), 6.05 (d, 1H, J=2.1 Hz), 6.29 (s, 1H), 7.16 (m, 1H), 7.30 (m, 1H), 7.46 (m, 1H), 7.51 (m, 1H), 7.88 (d, 1H, J=2.1 Hz), 8.01 (s, 1H), 11.14 (s, 1H); MS (ESI) m/z 337 (M+H)+.

### [Laboratory Test Example 1] Assessment of lipid kinase inhibitory activity

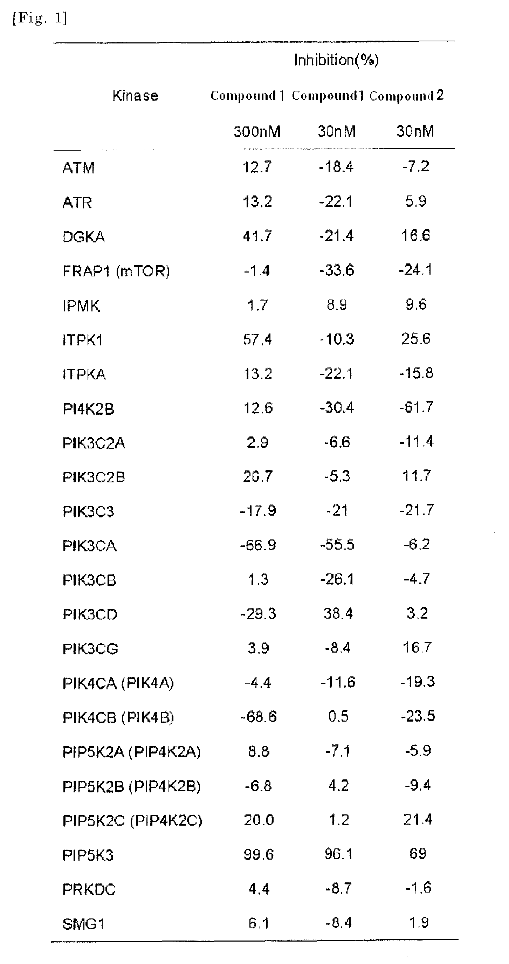

Lipid kinase inhibitory activity was assessed according to the method described in Biochemistry 2007, 46, 350-358. Jurkat cell derived from human lymphocyte was suspended in a lysis buffer (20 mM Hepes, 150 mM NaCl, 0.1% TritonX-100, 20 mM MnCl₂) by sonication, and the supernatant resulting from centrifugation at 100,000 x g for 60 minutes was used as a cell extract. To the cell extract (5 mg/mL), a test compound (30 nM or 300 nM) or a solvent alone was added and incubated for 5 minutes. Then, an ATP derivative labeled with biotinyl acyl phosphate (20 µM) was added and incubated for 5 minutes, and the resultant was further subjected to digestion with trypsin. After adding streptavidin agarose beads, streptavidin agarose bound to biotin-labeled peptide was separated by centrifugation procedure, washed, then dissolved in a solution containing 50% CH3CN and 0.1% TFA, and incubated at 65°C to elute the biotin-labeled peptide. The biotin-labeled peptide was analyzed by LC-MS/MS for sequence identification and measurement of the elution amount of the lipid-kinase-derived peptide so as to assess competitive inhibitory activity of the test compound for the binding of the ATP derivative to various lipid kinases. Assuming that the elution amount of the labeled peptide without addition of the test compound is 100%, the amount of reduction in the elution amount of the labeled peptide added with the test compound is indicated as inhibitory activity (%).
Using this assessment system, the inhibitory activity of the test compound for other lipid kinases was assessed (other lipid kinases may be commercially available or may be produced according to a method described in a known document) and the results thereof are shown in Figure 1. Compounds 1 and 2 showed inhibitory activity selective to PIKfyve.

### [Laboratory Test Example 2] Assessment of cytokine suppressive action using mouse peritoneal macrophage

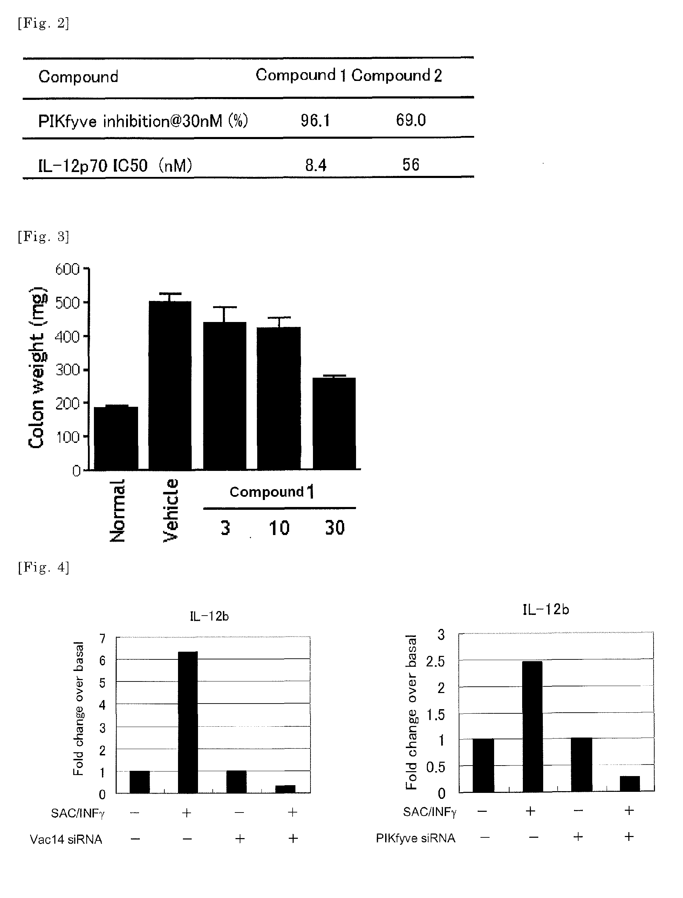

Cytokine suppressive action was assessed using mouse peritoneal macrophage according to the following method. To a male Balb/c mouse (Charles River Laboratories Japan), 1 mL of 3% (w/v) thioglycollate solution was intraperitoneally administered. After 5 or 6 days, the peritoneal cell was collected and the adherent cell was used for assessment. 100 ng/mL mouse interferon-γ, 0.05% (v/v) *Staphylococcus aureus* Cowan I strain-derived killed culture (SAC) and the compound were added and cultured overnight. After cultivation, the survival rate was determined, and IL-12p70 (complex of IL-12p35 and p40) and TNF-α were quantitated by ELISA (BD OptEIA™ Mouse ELISA Set, BD Biosciences) using the collected culture supernatant to calculate 50% production inhibition concentration (IC₅₀).
Results from the measurement of cytokine suppressive action of the test compound using this assessment system and results from the measurement of inhibitory activity for PIKfyve are shown in Figure 2. The strength of the PIKfyve inhibitory activity of the compound showed correlation with the strength of the mouse peritoneal macrophage IL-12-production inhibitory activity.

### [Laboratory Test Example 3] Assessment of inflammation suppressive action of Compound 1 in mouse enterocolitis model

A method for producing a mouse IL-10^{-/-} cell transfer enterocolitis model is described, for example, in Gastroenterology. 2009 Feb;136(2):564-74.e2. Epub 2008 Oct 7.

**[Table 1]**

| Group No. | Animal | Drug | Dose | Number of animals |
|---|---|---|---|---|
| 1 | Normal | - | - | 8 |
| 2 | Enterocolitis model | Vehicle | - | 8 |
| 3 | Enterocolitis model | Compound 1 | 3 mg/kg | 8 |
| 4 | Enterocolitis model | Compound 1 | 10 mg/kg | 8 |
| 5 | Enterocolitis model | Compound 1 | 30 mg/kg | 8 |

Measurement of an intestine weight as a drug efficacy indicator was carried out according to the following method. On the last day for autopsy, large intestine from the anus to immediately before the cecum was removed. The content of the intestine was washed with physiological saline, water was roughly removed and then the weight was measured.
Results from the assessment of the inflammation suppressive action of Compound 1 in mouse enterocolitis model using this assessment system are shown in Figure 3. The weight of the large intestine increased by IL-10^{-/-} cell transfer but was suppressed dependent on the dosage of Compound 1. Significant suppressive action was shown in the administration group of 30 mg/kg, once a day.

### [Laboratory Test Example 4] Assessment of IL-12-production suppressive action by PIKfyve and Vac14 knockdown

Assessment of the effects of knockdown of PIKfyve and Vac14 expressed in mouse peritoneal macrophage using siRNA method was carried out as described below. Mouse peritoneal macrophage was isolated according to the method described in Laboratory Test Example 2, and subsequently suspended in Nucleofection solution attached to Mouse macrophage nucleofection kit (Lonza) to give 3.3 x 10⁶ cells/mL. siRNA was added to the cell suspension to a final concentration of 1 µM and subjected to Nucleofection according to the attached protocol. The following siRNAs were used. For PIKfyve knockdown, ON-TARGETplus Non-targeting siRNA #1 (Dharmacon, D-001810-01-05) as negative control siRNA and ON-TARGETplus SMARTpool (Dharmacon, L-040127-00-0005) as PIKfyve siRNA were used. For Vac14 knockdown, Silencer Select Negative Control#1 siRNA (Ambion, 4390843) as negative control siRNA and Silencer Select Pre-designed siRNA (Sense strand: GAUUUACUCCAACAACCAAtt (SEQ ID NO:11), Antisense strand: UUGGUUGUUGGAGUAAAUCct (SEQ ID NO:12), Ambion, s107929) as Vac14 knockdown were used.
A FBS-free RPMI1640 medium was used for culture at 37°C in the presence of 5% CO₂ for 2 hours and then the medium was replaced by 0.5 ml of Dulbecco's MEM medium containing RPMI 1640 medium supplemented with 3% FBS for culture at 37°C in the presence of 5% CO₂ for 4 hours. Subsequently, 3.3 ng/mL mouse interferon-γ and 0.0017% (v/v) SAC were added and cultured for 3.5 hours and then the expression level of cytokine mRNA in the cell was measured.
Extraction and purification of total RNA from the mouse peritoneal macrophage was carried out using RNeasy midi kit (Qiagen 75144) according to the attached protocol. The concentration of the purified total RNA was measured using Biophotometer (Biorad) and then capillary electrophoresis using RNA nano 6000 (Agilent Technologies) was carried out with Bioanalyzer (Agilent Technologies) to confirm the quality of the total RNA. Using the total RNA as a template and 0.5 µg of oligo dt 20-mer primer (Invitrogen), cDNA was synthesized with Superscript III (Invitrogen). cDNA synthesis was performed according to the attached protocol. At the end of the reaction, incubation was carried out with RNaseH (Invitrogen) at 37°C for 20 minutes. The resultant removed of the remaining RNA was used as cDNA for quantitative PCR.
Quantitative PCR was carried out using SYBR Green Realtime PCR Master Mix (TOYOBO) according to the method described in the attached protocol in a 15 µl/well reaction system. cDNA was added as a template in an amount of 1/25 of the total amount of the reaction solution. Measurement was carried out with ABI7700 (Applied Biosystems) in the presence of 250 nM primer shown in Table 2. The relative expression level was calculated from the resulting Ct value. A value was calculated by dividing the relative expression level with the relative expression level of GAPDH used as the housekeeping gene. For each knockdown condition, the relative expression level normalized to GAPDH was divided by the value of the cell that had not been added with mouse interferon-a and SAC stimulation to calculate the rates of rising in IL-12b expression (fold-change over basal) caused by mouse interferon-a and SAC stimulation, which were used for verifying the effects by knockdown.

**[Table 2]**

| Primer | Forward | Reverse |
|---|---|---|
| GAPDH | ATCACTGCCACCCAGAAGAC (SEQ ID NO:53) | GGATGCAGGGATGATGTTCT (SEQ ID NO:54) |
| IL12b | CAGCACCAGCTTCTTCATCA (SEQ ID NO:55) | TACTCCCAGCTGACCTCCAC (SEQ ID NO:56) |

The results obtained by assessing the effect ofPIKfyve or Vac14 knockdown on IL-12 production using the present assessment system are shown in Figure 4. Since IL-12b expression was significantly suppressed by PIKfyve or Vac14 knockdown, IL-12-production suppression was shown to result from suppression of the expressions of these genes.

### INDUSTRIAL APPLICABILITY

A screening method of the present invention is useful for creating a novel prophylactic/therapeutic agent for an inflammatory disease.

## Claims

1. A method for screening a prophylactic or therapeutic agent for an inflammatory disease, comprising a step of measuring PIKfyve inhibitory activity of a test substance to identify a substance having this inhibitory activity.

2. The method according to Claim 1, wherein the PIKfyve inhibitory activity is measured by using phosphorylation by ATP or labeled ATP as an indicator.

3. The method according to either one of Claims 1 and 2, wherein the PIKfyve inhibitory activity is measured by using a substrate selected from phosphatidylinositol, phosphatidylinositol 3-phosphate or a derivative thereof, or Rab9 effector p40 or a partial sequence thereof.

4. The method according to Claim 1, wherein the PIKfyve inhibitory activity is measured by using binding between PIKfyve and a probe selected from labeled ATP and PIKfyve inhibitors as an indicator.

5. The method according to Claim 1, wherein the PIKfyve inhibitory activity is caused by an inhibitory activity for Vac14.

6. The method according to either one of Claims 1 and 5, wherein the PIKfyve inhibitory activity is an inhibitory activity against binding between PIKfyve and Vac14.

7. The method according to any one of Claims 1, 5 and 6, wherein the PIKfyve inhibitory activity is measured using PIKfyve or a partial sequence thereof, or Vac14 or a partial sequence thereof as a probe.

8. The method according to Claim 1, wherein the PIKfyve inhibitory activity is measured using cellular vacuolation as an indicator.

9. The method according to any one of Claims 1 to 8, wherein the test substance is selected from a low-molecular compound, an antibody, an antisense oligonucleotide, siRNA and an aptamer.

10. The method according to Claim 1, wherein the inflammatory disease is an IL-12/23-overproduction-related disease.


SEQUENCE LISTING

<110>  Ajinomoto Co., INC.

<120>  Screening Method

<130>  PCT10-0062

<150>  JP2009-296811
<151>  2009-12-28

<160>  56

<170>  PatentIn version 3.5

<210>  1
<211>  6297
<212>  DNA
<213>  human PIKfyve


<220>
<221>  CDS
<222>  (1)..(6297)

<400>  1
atg gcc aca gat gat aag acg tcc cca aca ctg gac tct gct aat gat      48
Met Ala Thr Asp Asp Lys Thr Ser Pro Thr Leu Asp Ser Ala Asn Asp
1               5                   10                  15

ttg cct cga tct cct act agt cct tct cat ctc aca cac ttt aaa cct      96
Leu Pro Arg Ser Pro Thr Ser Pro Ser His Leu Thr His Phe Lys Pro
            20                  25                  30

ttg act cct gat caa gat gag ccc cct ttt aaa tca gct tat agt tct      144
Leu Thr Pro Asp Gln Asp Glu Pro Pro Phe Lys Ser Ala Tyr Ser Ser
        35                  40                  45

ttt gta aat ctc ttt cgt ttt aac aaa gag aga gca gaa gga ggc cag      192
Phe Val Asn Leu Phe Arg Phe Asn Lys Glu Arg Ala Glu Gly Gly Gln
    50                  55                  60

gga gaa cag cag cct ttg agt gga agt tgg acc agc cct cag ctc cct      240
Gly Glu Gln Gln Pro Leu Ser Gly Ser Trp Thr Ser Pro Gln Leu Pro
65                  70                  75                  80

tcg agg aca cag tct gtt agg tca ccc aca cct tat aaa aag cag ctt      288
Ser Arg Thr Gln Ser Val Arg Ser Pro Thr Pro Tyr Lys Lys Gln Leu
                85                  90                  95

aat gag gaa ctc cag cgg cgc tct tca gca tta gac aca aga agg aaa      336
Asn Glu Glu Leu Gln Arg Arg Ser Ser Ala Leu Asp Thr Arg Arg Lys
            100                 105                 110

gca gaa cct acc ttt gga ggt cat gac cct cgt aca gct gtt cag ctt      384
Ala Glu Pro Thr Phe Gly Gly His Asp Pro Arg Thr Ala Val Gln Leu
        115                 120                 125

cga agc ctc agc aca gta tta aaa cgc ctc aag gaa atc atg gag ggg      432
Arg Ser Leu Ser Thr Val Leu Lys Arg Leu Lys Glu Ile Met Glu Gly
    130                 135                 140

aaa agc cag gat agt gac ctg aaa caa tac tgg atg cca gat agc caa      480
Lys Ser Gln Asp Ser Asp Leu Lys Gln Tyr Trp Met Pro Asp Ser Gln
145                 150                 155                 160

tgt aaa gag tgc tat gac tgt agt gag aaa ttt aca acc ttt agg cgc 528
Cys Lys Glu Cys Tyr Asp Cys Ser Glu Lys Phe Thr Thr Phe Arg Arg
                165                 170                 175

aga cac cat tgc cga cta tgt ggg cag att ttc tgc agt cgt tgc tgt 576
Arg His His Cys Arg Leu Cys Gly Gln Ile Phe Cys Ser Arg Cys Cys
            180                 185                 190

aat caa gaa atc cct gga aaa ttt atg ggc tat aca gga gac ctc cga 624
Asn Gln Glu Ile Pro Gly Lys Phe Met Gly Tyr Thr Gly Asp Leu Arg
        195                 200                 205

gct tgc aca tat tgt aga aaa ata gcc tta agt tat gct cat tcc aca 672
Ala Cys Thr Tyr Cys Arg Lys Ile Ala Leu Ser Tyr Ala His Ser Thr
    210                 215                 220

gac agt aat tct att ggg gaa gac ttg aat gct ctt tca gat tct gct 720
Asp Ser Asn Ser Ile Gly Glu Asp Leu Asn Ala Leu Ser Asp Ser Ala
225                 230                 235                 240

tgc tct gtg tct gtg ctt gat cca agt gaa ccc cga aca cct gtt ggg 768
Cys Ser Val Ser Val Leu Asp Pro Ser Glu Pro Arg Thr Pro Val Gly
                245                 250                 255

agt agg aaa gcc agc cgt aac ata ttt tta gag gat gat ttg gcc tgg 816
Ser Arg Lys Ala Ser Arg Asn Ile Phe Leu Glu Asp Asp Leu Ala Trp
            260                 265                 270

caa agt ttg att cat cca gat tcc tca aat act cct ctt tca aca aga 864
Gln Ser Leu Ile His Pro Asp Ser Ser Asn Thr Pro Leu Ser Thr Arg
        275                 280                 285

ctt gta tct gtg caa gag gat gct ggg aaa tct cct gct cga aat aga 912
Leu Val Ser Val Gln Glu Asp Ala Gly Lys Ser Pro Ala Arg Asn Arg
    290                 295                 300

tca gcc agc att act aac ctg tca ctg gat aga tct ggt tct cct atg 960
Ser Ala Ser Ile Thr Asn Leu Ser Leu Asp Arg Ser Gly Ser Pro Met
305                 310                 315                 320

gta cct tca tat gag aca tct gtc agt ccc cag gct aac cga aca tat 1008
Val Pro Ser Tyr Glu Thr Ser Val Ser Pro Gln Ala Asn Arg Thr Tyr
                325                 330                 335

gtt agg aca gag acc act gag gat gaa cgc aaa att ctt ctg gac agt 1056
Val Arg Thr Glu Thr Thr Glu Asp Glu Arg Lys Ile Leu Leu Asp Ser
            340                 345                 350

gtg cag tta aaa gac ctg tgg aaa aaa atc tgc cat cac agc agt gga 1104
Val Gln Leu Lys Asp Leu Trp Lys Lys Ile Cys His His Ser Ser Gly
        355                 360                 365

atg gag ttt cag gat cac cgc tac tgg ttg aga acg cat ccc aac tgc 1152
Met Glu Phe Gln Asp His Arg Tyr Trp Leu Arg Thr His Pro Asn Cys
    370                 375                 380

att gta gga aag gaa tta gtc aac tgg cta atc cga aat ggg cat att 1200
Ile Val Gly Lys Glu Leu Val Asn Trp Leu Ile Arg Asn Gly His Ile
385                 390                 395                 400

gcc aca agg gca caa gct ata gca att gga caa gca atg gtt gat gga 1248
Ala Thr Arg Ala Gln Ala Ile Ala Ile Gly Gln Ala Met Val Asp Gly
                405                 410                 415

cgt tgg ctg gat tgt gtt agt cat cac gac cag ctt ttc aga gat gag     1296
Arg Trp Leu Asp Cys Val Ser His His Asp Gln Leu Phe Arg Asp Glu
            420                 425                 430

tat gcg ctg tat aga cca ctg cag agt aca gaa ttt tct gag acg cct     1344
Tyr Ala Leu Tyr Arg Pro Leu Gln Ser Thr Glu Phe Ser Glu Thr Pro
        435                 440                 445

tct ccc gac agt gac tca gtg aac tcc gtg gaa gga cac tct gag cca     1392
Ser Pro Asp Ser Asp Ser Val Asn Ser Val Glu Gly His Ser Glu Pro
    450                 455                 460

tcc tgg ttt aaa gac ata aag ttt gat gac agt gac aca gaa cag ata     1440
Ser Trp Phe Lys Asp Ile Lys Phe Asp Asp Ser Asp Thr Glu Gln Ile
465                 470                 475                 480

gct gaa gaa ggt gac gat aat ttg gct aat tct gcc agt cct agc aag     1488
Ala Glu Glu Gly Asp Asp Asn Leu Ala Asn Ser Ala Ser Pro Ser Lys
                485                 490                 495

cgc aca tca gtc agc agt ttc cag tcc aca gtg gac agt gac tca gcc     1536
Arg Thr Ser Val Ser Ser Phe Gln Ser Thr Val Asp Ser Asp Ser Ala
            500                 505                 510

gct tct atc agc ctg aac gtg gag ctg gac aac gtg aac ttc cat atc     1584
Ala Ser Ile Ser Leu Asn Val Glu Leu Asp Asn Val Asn Phe His Ile
        515                 520                 525

aag aag ccc tcc aag tac cca cat gtg ccc cct cac cct gct gac caa     1632
Lys Lys Pro Ser Lys Tyr Pro His Val Pro Pro His Pro Ala Asp Gln
    530                 535                 540

aaa gag tat ttg att tct gac act gga gga caa cag ctc tca ata agt     1680
Lys Glu Tyr Leu Ile Ser Asp Thr Gly Gly Gln Gln Leu Ser Ile Ser
545                 550                 555                 560

gac gct ttc atc aaa gaa tcc tta ttt aat cgc cga gta gag gaa aaa     1728
Asp Ala Phe Ile Lys Glu Ser Leu Phe Asn Arg Arg Val Glu Glu Lys
                565                 570                 575

tcc aaa gag ctg cct ttc aca cct ttg ggc tgg cat cat aac aac ctg     1776
Ser Lys Glu Leu Pro Phe Thr Pro Leu Gly Trp His His Asn Asn Leu
            580                 585                 590

gag ctc ctg agg gag gag aat ggg gag aaa caa gcc atg gag agg ttg     1824
Glu Leu Leu Arg Glu Glu Asn Gly Glu Lys Gln Ala Met Glu Arg Leu
        595                 600                 605

ctt tca gct aat cat aac cac atg atg gca cta ctc cag cag ttg ctc     1872
Leu Ser Ala Asn His Asn His Met Met Ala Leu Leu Gln Gln Leu Leu
    610                 615                 620

cat agt gac tca ctg tca tca tct tgg agg gac atc atc gtg tca ttg     1920
His Ser Asp Ser Leu Ser Ser Ser Trp Arg Asp Ile Ile Val Ser Leu
625                 630                 635                 640

gtc tgc cag gtt gtt cag aca gtc cga cct gat gtc aag aac cag gat     1968
Val Cys Gln Val Val Gln Thr Val Arg Pro Asp Val Lys Asn Gln Asp
                645                 650                 655

gat gac atg gat atc cgt cag ttt gtc cac atc aaa aaa atc cca ggt     2016
Asp Asp Met Asp Ile Arg Gln Phe Val His Ile Lys Lys Ile Pro Gly
            660                 665                 670

gga aag aag ttt gat tct gtg gtt gtc aat ggc ttt gtt tgt acc aag     2064

Gly Lys Lys Phe Asp Ser Val Val Val Asn Gly Phe Val Cys Thr Lys
        675                 680                 685

aac att gca cat aaa aag atg aat tct tgt att aaa aac cct aaa att        2112
Asn Ile Ala His Lys Lys Met Asn Ser Cys Ile Lys Asn Pro Lys Ile
    690                 695                 700

ctt ctg ttg aag tgt tcc att gag tat ctc tac aga gaa gaa act aag        2160
Leu Leu Leu Lys Cys Ser Ile Glu Tyr Leu Tyr Arg Glu Glu Thr Lys
705                 710                 715                 720

ttt act tgc att gat cct att gtg ctt cag gaa agg gaa ttc ttg aag        2208
Phe Thr Cys Ile Asp Pro Ile Val Leu Gln Glu Arg Glu Phe Leu Lys
                725                 730                 735

aat tat gtc cag cga ata gtt gat gtt cga ccc acc ttg gtt ctt gtt        2256
Asn Tyr Val Gln Arg Ile Val Asp Val Arg Pro Thr Leu Val Leu Val
            740                 745                 750

gag aaa aca gtg tct cgg att gcc cag gac atg tta ttg gaa cat ggc        2304
Glu Lys Thr Val Ser Arg Ile Ala Gln Asp Met Leu Leu Glu His Gly
        755                 760                 765

att act ttg gtc att aat gta aag tca caa gtt ttg gaa cga atc agt        2352
Ile Thr Leu Val Ile Asn Val Lys Ser Gln Val Leu Glu Arg Ile Ser
    770                 775                 780

cga atg acc caa ggt gat tta gtg atg tca atg gac cag ctg ctt acg        2400
Arg Met Thr Gln Gly Asp Leu Val Met Ser Met Asp Gln Leu Leu Thr
785                 790                 795                 800

aaa cca cac ctg ggc act tgt cac aaa ttt tat atg cag ata ttt cag        2448
Lys Pro His Leu Gly Thr Cys His Lys Phe Tyr Met Gln Ile Phe Gln
                805                 810                 815

ttg cct aat gaa caa acc aag aca ctg atg ttt ttt gaa ggt tgt cca        2496
Leu Pro Asn Glu Gln Thr Lys Thr Leu Met Phe Phe Glu Gly Cys Pro
            820                 825                 830

cag cac cta ggc tgt aca atc aag cta aga gga ggc tct gat tat gag        2544
Gln His Leu Gly Cys Thr Ile Lys Leu Arg Gly Gly Ser Asp Tyr Glu
        835                 840                 845

ctg gct cga gtt aag gag atc cta ata ttt atg atc tgt gtt gct tat        2592
Leu Ala Arg Val Lys Glu Ile Leu Ile Phe Met Ile Cys Val Ala Tyr
    850                 855                 860

cat tct caa cta gaa ata tcc ttt ctc atg gat gaa ttt gct atg cct        2640
His Ser Gln Leu Glu Ile Ser Phe Leu Met Asp Glu Phe Ala Met Pro
865                 870                 875                 880

ccc aca tta atg caa aac cct tca ttc cat tcc ctg att gag gga cga        2688
Pro Thr Leu Met Gln Asn Pro Ser Phe His Ser Leu Ile Glu Gly Arg
                885                 890                 895

ggg cat gag ggg gct gtc caa gag cag tac ggt gga ggt tcc atc ccc        2736
Gly His Glu Gly Ala Val Gln Glu Gln Tyr Gly Gly Gly Ser Ile Pro
            900                 905                 910

tgg gat cct gac atc cct cct gag tct ctg ccc tgt gat gat agc agt        2784
Trp Asp Pro Asp Ile Pro Pro Glu Ser Leu Pro Cys Asp Asp Ser Ser
        915                 920                 925

ttg ctg gaa tcg agg att gtg ttt gag aag ggt gag cag gaa aat aaa        2832
Leu Leu Glu Ser Arg Ile Val Phe Glu Lys Gly Glu Gln Glu Asn Lys

930                 935                 940

aat ctt ccg cag gct gtt gcc tct gtg aag cat caa gaa cat agc aca        2880
Asn Leu Pro Gln Ala Val Ala Ser Val Lys His Gln Glu His Ser Thr
945                 950                 955                 960

aca gct tgc ccg gcg ggt ctc cct tgt gct ttc ttt gca cct gta ccg        2928
Thr Ala Cys Pro Ala Gly Leu Pro Cys Ala Phe Phe Ala Pro Val Pro
                965                 970                 975

gaa tca ttg ttg cca ctc cct gtg gat gac caa caa gat gct tta ggc        2976
Glu Ser Leu Leu Pro Leu Pro Val Asp Asp Gln Gln Asp Ala Leu Gly
            980                 985                 990

agc gag ctg cca gag agt ttg cag  caa aca gtt gtg ctg  cag gat ccc      3024
Ser Glu Leu Pro Glu Ser Leu Gln  Gln Thr Val Val Leu  Gln Asp Pro
        995                 1000                 1005

aaa agc  cag ata aga gcc ttt  aga gac cct cta cag  gat gac act        3069
Lys Ser  Gln Ile Arg Ala Phe  Arg Asp Pro Leu Gln  Asp Asp Thr
    1010                 1015                 1020

gga tta  tat gtt act gag gaa  gtc acc tcc tct gaa  gat aaa cga        3114
Gly Leu  Tyr Val Thr Glu Glu  Val Thr Ser Ser Glu  Asp Lys Arg
    1025                 1030                 1035

aag act  tat tct ttg gcc ttt  aag cag gaa tta aaa  gat gtg atc        3159
Lys Thr  Tyr Ser Leu Ala Phe  Lys Gln Glu Leu Lys  Asp Val Ile
    1040                 1045                 1050

ctc tgt  atc tcc cca gta atc  aca ttc cga gaa ccc  ttt ctt tta        3204
Leu Cys  Ile Ser Pro Val Ile  Thr Phe Arg Glu Pro  Phe Leu Leu
    1055                 1060                 1065

act gaa  aag ggg atg aga tgc  tct acc cga gat tat  ttt gca gag        3249
Thr Glu  Lys Gly Met Arg Cys  Ser Thr Arg Asp Tyr  Phe Ala Glu
    1070                 1075                 1080

cag gtt  tac tgg tct cct ctc  ctc aat aaa gaa ttc  aaa gaa atg        3294
Gln Val  Tyr Trp Ser Pro Leu  Leu Asn Lys Glu Phe  Lys Glu Met
    1085                 1090                 1095

gag aac  agg agg aag aaa cag  ctg ctc agg gat ctc  tct gga ctt        3339
Glu Asn  Arg Arg Lys Lys Gln  Leu Leu Arg Asp Leu  Ser Gly Leu
    1100                 1105                 1110

cag ggc  atg aat gga agt att  cag gcc aag tct att  caa gtc tta        3384
Gln Gly  Met Asn Gly Ser Ile  Gln Ala Lys Ser Ile  Gln Val Leu
    1115                 1120                 1125

ccc tca  cat gag cta gtg agc  act aga att gct gag  cat ctg ggc        3429
Pro Ser  His Glu Leu Val Ser  Thr Arg Ile Ala Glu  His Leu Gly
    1130                 1135                 1140

gat agc  cag agc ttg ggt aga  atg ctg gcc gat tat  cga gcc aga        3474
Asp Ser  Gln Ser Leu Gly Arg  Met Leu Ala Asp Tyr  Arg Ala Arg
    1145                 1150                 1155

gga gga  aga att cag ccc aaa  aat tca gac cct ttt  gct cat tca        3519
Gly Gly  Arg Ile Gln Pro Lys  Asn Ser Asp Pro Phe  Ala His Ser
    1160                 1165                 1170

aag gat  gca tca agt act tca  agt ggc aaa tca gga  agc aaa aat        3564
Lys Asp  Ala Ser Ser Thr Ser  Ser Gly Lys Ser Gly  Ser Lys Asn
    1175                 1180                 1185

gag ggt gat gaa gag aga ggg ctt att ctg agt gat gct gtg tgg 3609
Glu Gly Asp Glu Glu Arg Gly Leu Ile Leu Ser Asp Ala Val Trp
    1190                1195                1200

tca aca aag gtg gac tgt ctg aat ccc att aat cac cag aga ctt 3654
Ser Thr Lys Val Asp Cys Leu Asn Pro Ile Asn His Gln Arg Leu
    1205                1210                1215

tgt gtg ctc ttc agc agc tct tct gcc cag tcc agc aat gct cct 3699
Cys Val Leu Phe Ser Ser Ser Ser Ala Gln Ser Ser Asn Ala Pro
    1220                1225                1230

agt gcc tgt gtc agt cct tgg att gta aca atg gaa ttt tat gga 3744
Ser Ala Cys Val Ser Pro Trp Ile Val Thr Met Glu Phe Tyr Gly
    1235                1240                1245

aag aat gat ctt aca tta gga ata ttt tta gag aga tac tgt ttc 3789
Lys Asn Asp Leu Thr Leu Gly Ile Phe Leu Glu Arg Tyr Cys Phe
    1250                1255                1260

agg cct tct tat cag tgt cca agc atg ttc tgt gat acc ccc atg 3834
Arg Pro Ser Tyr Gln Cys Pro Ser Met Phe Cys Asp Thr Pro Met
    1265                1270                1275

gta cat cat att cgg cgc ttt gtt cat ggc caa ggc tgt gtg cag 3879
Val His His Ile Arg Arg Phe Val His Gly Gln Gly Cys Val Gln
    1280                1285                1290

ata atc ctg aag gag ttg gat tct cca gta cct gga tat cag cat 3924
Ile Ile Leu Lys Glu Leu Asp Ser Pro Val Pro Gly Tyr Gln His
    1295                1300                1305

aca att ctt aca tat tcc tgg tgt aga atc tgc aaa cag gta aca 3969
Thr Ile Leu Thr Tyr Ser Trp Cys Arg Ile Cys Lys Gln Val Thr
    1310                1315                1320

cca gtt gtt gct ctt tcc aat gag tcc tgg tct atg tca ttt gca 4014
Pro Val Val Ala Leu Ser Asn Glu Ser Trp Ser Met Ser Phe Ala
    1325                1330                1335

aaa tac ctt gaa ctt agg ttt tat ggg cac cag tat act cgc aga 4059
Lys Tyr Leu Glu Leu Arg Phe Tyr Gly His Gln Tyr Thr Arg Arg
    1340                1345                1350

gcc aac gct gag ccc tgt ggt cac tcc atc cat cat gat tat cac 4104
Ala Asn Ala Glu Pro Cys Gly His Ser Ile His His Asp Tyr His
    1355                1360                1365

cag tat ttc tcc tat aac cag atg gtg gcg tct ttc agt tat tct 4149
Gln Tyr Phe Ser Tyr Asn Gln Met Val Ala Ser Phe Ser Tyr Ser
    1370                1375                1380

ccc att cgg ctt ctt gaa gta tgt gtt cca ctc ccc aaa ata ttc 4194
Pro Ile Arg Leu Leu Glu Val Cys Val Pro Leu Pro Lys Ile Phe
    1385                1390                1395

att aag cgt cag gcc cca tta aaa gtg tcc ctt ctt cag gat ctg 4239
Ile Lys Arg Gln Ala Pro Leu Lys Val Ser Leu Leu Gln Asp Leu
    1400                1405                1410

aag gac ttc ttt caa aaa gtt tca cag gta tat gtt gcc att gat 4284
Lys Asp Phe Phe Gln Lys Val Ser Gln Val Tyr Val Ala Ile Asp
    1415                1420                1425

gaa aga ctt gca tct ttg aaa act gat aca ttt agt aaa aca aga 4329
Glu Arg Leu Ala Ser Leu Lys Thr Asp Thr Phe Ser Lys Thr Arg
    1430                1435                1440

gag gaa aaa atg gaa gat att ttt gca cag aaa gag atg gaa gaa 4374
Glu Glu Lys Met Glu Asp Ile Phe Ala Gln Lys Glu Met Glu Glu
    1445                1450                1455

ggt gag ttc aag aac tgg att gag aag atg caa gca agg ctc atg 4419
Gly Glu Phe Lys Asn Trp Ile Glu Lys Met Gln Ala Arg Leu Met
    1460                1465                1470

tct tcc tct gta gat acc cct cag caa ctg cag tcg gtc ttt gag 4464
Ser Ser Ser Val Asp Thr Pro Gln Gln Leu Gln Ser Val Phe Glu
    1475                1480                1485

tca ctc att gcc aag aaa caa agt ctc tgt gaa gtg ctg caa gct 4509
Ser Leu Ile Ala Lys Lys Gln Ser Leu Cys Glu Val Leu Gln Ala
    1490                1495                1500

tgg aat aac agg ttg cag gac ctt ttc caa cag gaa aag ggt aga 4554
Trp Asn Asn Arg Leu Gln Asp Leu Phe Gln Gln Glu Lys Gly Arg
    1505                1510                1515

aag aga cct tca gtt cct cca agt cct gga aga ctg aga caa ggg 4599
Lys Arg Pro Ser Val Pro Pro Ser Pro Gly Arg Leu Arg Gln Gly
    1520                1525                1530

gaa gaa agc aag ata agt gcg atg gat gca tct cca cgg aat att 4644
Glu Glu Ser Lys Ile Ser Ala Met Asp Ala Ser Pro Arg Asn Ile
    1535                1540                1545

tct cca gga ctt cag aat gga gaa aaa gag gat cgc ttc tta aca 4689
Ser Pro Gly Leu Gln Asn Gly Glu Lys Glu Asp Arg Phe Leu Thr
    1550                1555                1560

act ttg tcc agc cag agc tcc acc agt tct act cat ctc caa ttg 4734
Thr Leu Ser Ser Gln Ser Ser Thr Ser Ser Thr His Leu Gln Leu
    1565                1570                1575

cct acg cca cct gaa gtc atg tct gaa cag tca gtg gga ggg ccc 4779
Pro Thr Pro Pro Glu Val Met Ser Glu Gln Ser Val Gly Gly Pro
    1580                1585                1590

cct gag cta gat aca gcc agc agt tcc gaa gat gtg ttt gat ggg 4824
Pro Glu Leu Asp Thr Ala Ser Ser Ser Glu Asp Val Phe Asp Gly
    1595                1600                1605

cat ttg ctg gga tcc aca gac agc caa gtg aag gaa aag tca acc 4869
His Leu Leu Gly Ser Thr Asp Ser Gln Val Lys Glu Lys Ser Thr
    1610                1615                1620

atg aaa gcc atc ttt gca aat ttg ctt cca gga aat agc tat aat 4914
Met Lys Ala Ile Phe Ala Asn Leu Leu Pro Gly Asn Ser Tyr Asn
    1625                1630                1635

cct att cca ttt cct ttt gat cca gat aaa cac tac tta atg tat 4959
Pro Ile Pro Phe Pro Phe Asp Pro Asp Lys His Tyr Leu Met Tyr
    1640                1645                1650

gaa cat gaa cga gtg ccc att gca gtc tgc gag aag gaa ccc agc 5004
Glu His Glu Arg Val Pro Ile Ala Val Cys Glu Lys Glu Pro Ser
    1655                1660                1665

tcc atc att gct ttt gct ctc agt tgt aaa gaa tac cga aat gcc 5049

Ser Ile Ile Ala Phe Ala Leu Ser Cys Lys Glu Tyr Arg Asn Ala
    1670                1675                1680

tta gag gaa ttg tct aaa gcg act cag tgg aac agt gcc gaa gaa 5094
Leu Glu Glu Leu Ser Lys Ala Thr Gln Trp Asn Ser Ala Glu Glu
    1685                1690                1695

ggg ctt cca aca aat agt act tca gat agc aga cca aag agt agc 5139
Gly Leu Pro Thr Asn Ser Thr Ser Asp Ser Arg Pro Lys Ser Ser
    1700                1705                1710

agc cct atc aga tta cct gaa atg agt gga gga cag aca aat cgt 5184
Ser Pro Ile Arg Leu Pro Glu Met Ser Gly Gly Gln Thr Asn Arg
    1715                1720                1725

aca aca gaa aca gaa cca caa cca acc aaa aag gct tct gga atg 5229
Thr Thr Glu Thr Glu Pro Gln Pro Thr Lys Lys Ala Ser Gly Met
    1730                1735                1740

ttg tcc ttc ttc aga ggg aca gca ggg aaa agc ccc gat ctc tct 5274
Leu Ser Phe Phe Arg Gly Thr Ala Gly Lys Ser Pro Asp Leu Ser
    1745                1750                1755

tcc cag aag aga gag acc tta cgt gga gca gat agt gct tac tac 5319
Ser Gln Lys Arg Glu Thr Leu Arg Gly Ala Asp Ser Ala Tyr Tyr
    1760                1765                1770

cag gtt ggg cag acg ggc aag gag ggg acc gag aat caa ggc gtt 5364
Gln Val Gly Gln Thr Gly Lys Glu Gly Thr Glu Asn Gln Gly Val
    1775                1780                1785

gag cct caa gat gaa gta gat gga gga gat aca caa aag aag caa 5409
Glu Pro Gln Asp Glu Val Asp Gly Gly Asp Thr Gln Lys Lys Gln
    1790                1795                1800

ctc ata aat cct cat gtg gaa ctt caa ttt tca gat gct aat gcc 5454
Leu Ile Asn Pro His Val Glu Leu Gln Phe Ser Asp Ala Asn Ala
    1805                1810                1815

aag ttt tac tgt cgg ctc tac tat gcg gga gag ttt cat aag atg 5499
Lys Phe Tyr Cys Arg Leu Tyr Tyr Ala Gly Glu Phe His Lys Met
    1820                1825                1830

cgt gaa gtg att ctg gac agc agt gaa gaa gat ttc att cgt tcc 5544
Arg Glu Val Ile Leu Asp Ser Ser Glu Glu Asp Phe Ile Arg Ser
    1835                1840                1845

ctc tcc cac tca tca ccc tgg cag gcc cgg gga ggc aaa tca gga 5589
Leu Ser His Ser Ser Pro Trp Gln Ala Arg Gly Gly Lys Ser Gly
    1850                1855                1860

gct gcc ttc tat gca act gag gat gat aga ttt att ttg aag caa 5634
Ala Ala Phe Tyr Ala Thr Glu Asp Asp Arg Phe Ile Leu Lys Gln
    1865                1870                1875

atg cct cgt ctg gaa gtc cag tcc ttc ctc gac ttt gca cca cat 5679
Met Pro Arg Leu Glu Val Gln Ser Phe Leu Asp Phe Ala Pro His
    1880                1885                1890

tac ttc aat tat att aca aat gct gtt caa caa aag agg ccc acg 5724
Tyr Phe Asn Tyr Ile Thr Asn Ala Val Gln Gln Lys Arg Pro Thr
    1895                1900                1905

gcg ttg gcc aaa att ctt gga gtt tac aga att ggt tat aag aac 5769
Ala Leu Ala Lys Ile Leu Gly Val Tyr Arg Ile Gly Tyr Lys Asn

1910                1915                1920

tct cag  aac aac act gag aag  aag tta gat ctc ctt  gtc atg gaa        5814
Ser Gln  Asn Asn Thr Glu Lys  Lys Leu Asp Leu Leu  Val Met Glu
    1925                1930                1935

aat ctt  ttc tac ggg aga aag  atg gca cag gtt ttt  gat ttg aag        5859
Asn Leu  Phe Tyr Gly Arg Lys  Met Ala Gln Val Phe  Asp Leu Lys
    1940                1945                1950

ggc tct  ctt agg aat cgg aat  gta aaa act gac act  gga aaa gag        5904
Gly Ser  Leu Arg Asn Arg Asn  Val Lys Thr Asp Thr  Gly Lys Glu
    1955                1960                1965

agt tgt  gat gtg gtc ctg cta  gat gaa aat ctc cta  aag atg gtt        5949
Ser Cys  Asp Val Val Leu Leu  Asp Glu Asn Leu Leu  Lys Met Val
    1970                1975                1980

cga gac  aac cct cta tat att  cgt tct cat tcc aaa  gct gtg ctg        5994
Arg Asp  Asn Pro Leu Tyr Ile  Arg Ser His Ser Lys  Ala Val Leu
    1985                1990                1995

aga acc  tcg atc cat agt gac  tcc cat ttc ctt tct  agc cac ctc        6039
Arg Thr  Ser Ile His Ser Asp  Ser His Phe Leu Ser  Ser His Leu
    2000                2005                2010

att ata  gat tat tct ttg ctg  gtt ggg cga gat gat  act agc aat        6084
Ile Ile  Asp Tyr Ser Leu Leu  Val Gly Arg Asp Asp  Thr Ser Asn
    2015                2020                2025

gag cta  gta gtt gga att ata  gat tat att cga aca  ttt aca tgg        6129
Glu Leu  Val Val Gly Ile Ile  Asp Tyr Ile Arg Thr  Phe Thr Trp
    2030                2035                2040

gac aaa  aag ctt gag atg gtt  gtg aaa tca aca gga  att tta ggt        6174
Asp Lys  Lys Leu Glu Met Val  Val Lys Ser Thr Gly  Ile Leu Gly
    2045                2050                2055

gga caa  ggt aaa atg cca aca  gtg gtg tct ccg gag  ttg tac agg        6219
Gly Gln  Gly Lys Met Pro Thr  Val Val Ser Pro Glu  Leu Tyr Arg
    2060                2065                2070

act agg  ttt tgt gag gca atg  gac aag tat ttc cta  atg gta cca        6264
Thr Arg  Phe Cys Glu Ala Met  Asp Lys Tyr Phe Leu  Met Val Pro
    2075                2080                2085

gac cac  tgg aca ggc ttg ggt  ctg aat tgc tga                         6297
Asp His  Trp Thr Gly Leu Gly  Leu Asn Cys
    2090                2095

<210>  2
<211>  2098
<212>  PRT
<213>  human PIKfyve

<400>  2

Met Ala Thr Asp Asp Lys Thr Ser Pro Thr Leu Asp Ser Ala Asn Asp
1               5                   10                  15

Leu Pro Arg Ser Pro Thr Ser Pro Ser His Leu Thr His Phe Lys Pro
            20                  25                  30

Leu Thr Pro Asp Gln Asp Glu Pro Pro Phe Lys Ser Ala Tyr Ser Ser
        35                  40                  45

Phe Val Asn Leu Phe Arg Phe Asn Lys Glu Arg Ala Glu Gly Gly Gln
    50                  55                  60

Gly Glu Gln Gln Pro Leu Ser Gly Ser Trp Thr Ser Pro Gln Leu Pro
65                  70                  75                  80

Ser Arg Thr Gln Ser Val Arg Ser Pro Thr Pro Tyr Lys Lys Gln Leu
                85                  90                  95

Asn Glu Glu Leu Gln Arg Arg Ser Ser Ala Leu Asp Thr Arg Arg Lys
            100                 105                 110

Ala Glu Pro Thr Phe Gly Gly His Asp Pro Arg Thr Ala Val Gln Leu
        115                 120                 125

Arg Ser Leu Ser Thr Val Leu Lys Arg Leu Lys Glu Ile Met Glu Gly
    130                 135                 140

Lys Ser Gln Asp Ser Asp Leu Lys Gln Tyr Trp Met Pro Asp Ser Gln
145                 150                 155                 160

Cys Lys Glu Cys Tyr Asp Cys Ser Glu Lys Phe Thr Thr Phe Arg Arg
                165                 170                 175

Arg His His Cys Arg Leu Cys Gly Gln Ile Phe Cys Ser Arg Cys Cys
            180                 185                 190

Asn Gln Glu Ile Pro Gly Lys Phe Met Gly Tyr Thr Gly Asp Leu Arg
        195                 200                 205

Ala Cys Thr Tyr Cys Arg Lys Ile Ala Leu Ser Tyr Ala His Ser Thr
    210                 215                 220

Asp Ser Asn Ser Ile Gly Glu Asp Leu Asn Ala Leu Ser Asp Ser Ala
225                 230                 235                 240

Cys Ser Val Ser Val Leu Asp Pro Ser Glu Pro Arg Thr Pro Val Gly
                245                 250                 255

Ser Arg Lys Ala Ser Arg Asn Ile Phe Leu Glu Asp Asp Leu Ala Trp
            260                 265                 270

Gln Ser Leu Ile His Pro Asp Ser Ser Asn Thr Pro Leu Ser Thr Arg
        275                 280                 285

Leu Val Ser Val Gln Glu Asp Ala Gly Lys Ser Pro Ala Arg Asn Arg
    290                 295                 300

Ser Ala Ser Ile Thr Asn Leu Ser Leu Asp Arg Ser Gly Ser Pro Met
305                 310                 315                 320

Val Pro Ser Tyr Glu Thr Ser Val Ser Pro Gln Ala Asn Arg Thr Tyr
                325                 330                 335

Val Arg Thr Glu Thr Thr Glu Asp Glu Arg Lys Ile Leu Leu Asp Ser
            340                 345                 350

Val Gln Leu Lys Asp Leu Trp Lys Lys Ile Cys His His Ser Ser Gly
        355                 360                 365

Met Glu Phe Gln Asp His Arg Tyr Trp Leu Arg Thr His Pro Asn Cys
    370                 375                 380

Ile Val Gly Lys Glu Leu Val Asn Trp Leu Ile Arg Asn Gly His Ile
385                 390                 395                 400

Ala Thr Arg Ala Gln Ala Ile Ala Ile Gly Gln Ala Met Val Asp Gly
                405                 410                 415

Arg Trp Leu Asp Cys Val Ser His His Asp Gln Leu Phe Arg Asp Glu
            420                 425                 430

Tyr Ala Leu Tyr Arg Pro Leu Gln Ser Thr Glu Phe Ser Glu Thr Pro
        435                 440                 445

Ser Pro Asp Ser Asp Ser Val Asn Ser Val Glu Gly His Ser Glu Pro
    450                 455                 460

Ser Trp Phe Lys Asp Ile Lys Phe Asp Asp Ser Asp Thr Glu Gln Ile
465                 470                 475                 480

Ala Glu Glu Gly Asp Asp Asn Leu Ala Asn Ser Ala Ser Pro Ser Lys
                485                 490                 495

Arg Thr Ser Val Ser Ser Phe Gln Ser Thr Val Asp Ser Asp Ser Ala
            500                 505                 510

Ala Ser Ile Ser Leu Asn Val Glu Leu Asp Asn Val Asn Phe His Ile
        515                 520                 525

Lys Lys Pro Ser Lys Tyr Pro His Val Pro Pro His Pro Ala Asp Gln
    530                 535                 540

Lys Glu Tyr Leu Ile Ser Asp Thr Gly Gly Gln Gln Leu Ser Ile Ser

545                 550                 555                 560

Asp Ala Phe Ile Lys Glu Ser Leu Phe Asn Arg Arg Val Glu Glu Lys
                565                 570                 575

Ser Lys Glu Leu Pro Phe Thr Pro Leu Gly Trp His His Asn Asn Leu
            580                 585                 590

Glu Leu Leu Arg Glu Glu Asn Gly Glu Lys Gln Ala Met Glu Arg Leu
        595                 600                 605

Leu Ser Ala Asn His Asn His Met Met Ala Leu Leu Gln Gln Leu Leu
    610                 615                 620

His Ser Asp Ser Leu Ser Ser Ser Trp Arg Asp Ile Ile Val Ser Leu
625                 630                 635                 640

Val Cys Gln Val Val Gln Thr Val Arg Pro Asp Val Lys Asn Gln Asp
                645                 650                 655

Asp Asp Met Asp Ile Arg Gln Phe Val His Ile Lys Lys Ile Pro Gly
            660                 665                 670

Gly Lys Lys Phe Asp Ser Val Val Val Asn Gly Phe Val Cys Thr Lys
        675                 680                 685

Asn Ile Ala His Lys Lys Met Asn Ser Cys Ile Lys Asn Pro Lys Ile
    690                 695                 700

Leu Leu Leu Lys Cys Ser Ile Glu Tyr Leu Tyr Arg Glu Glu Thr Lys
705                 710                 715                 720

Phe Thr Cys Ile Asp Pro Ile Val Leu Gln Glu Arg Glu Phe Leu Lys
                725                 730                 735

Asn Tyr Val Gln Arg Ile Val Asp Val Arg Pro Thr Leu Val Leu Val
            740                 745                 750

Glu Lys Thr Val Ser Arg Ile Ala Gln Asp Met Leu Leu Glu His Gly
        755                 760                 765

Ile Thr Leu Val Ile Asn Val Lys Ser Gln Val Leu Glu Arg Ile Ser
    770                 775                 780

Arg Met Thr Gln Gly Asp Leu Val Met Ser Met Asp Gln Leu Leu Thr
785                 790                 795                 800

Lys Pro His Leu Gly Thr Cys His Lys Phe Tyr Met Gln Ile Phe Gln
                805                 810                 815

Leu Pro Asn Glu Gln Thr Lys Thr Leu Met Phe Phe Glu Gly Cys Pro
            820                 825                 830

Gln His Leu Gly Cys Thr Ile Lys Leu Arg Gly Gly Ser Asp Tyr Glu
        835                 840                 845

Leu Ala Arg Val Lys Glu Ile Leu Ile Phe Met Ile Cys Val Ala Tyr
    850                 855                 860

His Ser Gln Leu Glu Ile Ser Phe Leu Met Asp Glu Phe Ala Met Pro
865                 870                 875                 880

Pro Thr Leu Met Gln Asn Pro Ser Phe His Ser Leu Ile Glu Gly Arg
                885                 890                 895

Gly His Glu Gly Ala Val Gln Glu Gln Tyr Gly Gly Gly Ser Ile Pro
            900                 905                 910

Trp Asp Pro Asp Ile Pro Pro Glu Ser Leu Pro Cys Asp Asp Ser Ser
        915                 920                 925

Leu Leu Glu Ser Arg Ile Val Phe Glu Lys Gly Glu Gln Glu Asn Lys
    930                 935                 940

Asn Leu Pro Gln Ala Val Ala Ser Val Lys His Gln Glu His Ser Thr
945                 950                 955                 960

Thr Ala Cys Pro Ala Gly Leu Pro Cys Ala Phe Phe Ala Pro Val Pro
                965                 970                 975

Glu Ser Leu Leu Pro Leu Pro Val Asp Asp Gln Gln Asp Ala Leu Gly
            980                 985                 990

Ser Glu Leu Pro Glu Ser Leu Gln  Gln Thr Val Val Leu  Gln Asp Pro
        995                 1000                 1005

Lys Ser  Gln Ile Arg Ala Phe  Arg Asp Pro Leu Gln  Asp Asp Thr
    1010                 1015                 1020

Gly Leu  Tyr Val Thr Glu Glu  Val Thr Ser Ser Glu  Asp Lys Arg
    1025                 1030                 1035

Lys Thr  Tyr Ser Leu Ala Phe  Lys Gln Glu Leu Lys  Asp Val Ile
    1040                 1045                 1050

Leu Cys  Ile Ser Pro Val Ile  Thr Phe Arg Glu Pro  Phe Leu Leu
    1055                 1060                 1065

Thr Glu Lys Gly Met Arg Cys Ser Thr Arg Asp Tyr Phe Ala Glu
    1070                1075                1080

Gln Val Tyr Trp Ser Pro Leu Leu Asn Lys Glu Phe Lys Glu Met
    1085                1090                1095

Glu Asn Arg Arg Lys Lys Gln Leu Leu Arg Asp Leu Ser Gly Leu
    1100                1105                1110

Gln Gly Met Asn Gly Ser Ile Gln Ala Lys Ser Ile Gln Val Leu
    1115                1120                1125

Pro Ser His Glu Leu Val Ser Thr Arg Ile Ala Glu His Leu Gly
    1130                1135                1140

Asp Ser Gln Ser Leu Gly Arg Met Leu Ala Asp Tyr Arg Ala Arg
    1145                1150                1155

Gly Gly Arg Ile Gln Pro Lys Asn Ser Asp Pro Phe Ala His Ser
    1160                1165                1170

Lys Asp Ala Ser Ser Thr Ser Ser Gly Lys Ser Gly Ser Lys Asn
    1175                1180                1185

Glu Gly Asp Glu Glu Arg Gly Leu Ile Leu Ser Asp Ala Val Trp
    1190                1195                1200

Ser Thr Lys Val Asp Cys Leu Asn Pro Ile Asn His Gln Arg Leu
    1205                1210                1215

Cys Val Leu Phe Ser Ser Ser Ser Ala Gln Ser Ser Asn Ala Pro
    1220                1225                1230

Ser Ala Cys Val Ser Pro Trp Ile Val Thr Met Glu Phe Tyr Gly
    1235                1240                1245

Lys Asn Asp Leu Thr Leu Gly Ile Phe Leu Glu Arg Tyr Cys Phe
    1250                1255                1260

Arg Pro Ser Tyr Gln Cys Pro Ser Met Phe Cys Asp Thr Pro Met
    1265                1270                1275

Val His His Ile Arg Arg Phe Val His Gly Gln Gly Cys Val Gln
    1280                1285                1290

Ile Ile Leu Lys Glu Leu Asp Ser Pro Val Pro Gly Tyr Gln His
    1295                1300                1305

Thr Ile Leu Thr Tyr Ser Trp Cys Arg Ile Cys Lys Gln Val Thr
    1310                1315                1320

Pro Val Val Ala Leu Ser Asn Glu Ser Trp Ser Met Ser Phe Ala
    1325                1330                1335

Lys Tyr Leu Glu Leu Arg Phe Tyr Gly His Gln Tyr Thr Arg Arg
    1340                1345                1350

Ala Asn Ala Glu Pro Cys Gly His Ser Ile His His Asp Tyr His
    1355                1360                1365

Gln Tyr Phe Ser Tyr Asn Gln Met Val Ala Ser Phe Ser Tyr Ser
    1370                1375                1380

Pro Ile Arg Leu Leu Glu Val Cys Val Pro Leu Pro Lys Ile Phe
    1385                1390                1395

Ile Lys Arg Gln Ala Pro Leu Lys Val Ser Leu Leu Gln Asp Leu
    1400                1405                1410

Lys Asp Phe Phe Gln Lys Val Ser Gln Val Tyr Val Ala Ile Asp
    1415                1420                1425

Glu Arg Leu Ala Ser Leu Lys Thr Asp Thr Phe Ser Lys Thr Arg
    1430                1435                1440

Glu Glu Lys Met Glu Asp Ile Phe Ala Gln Lys Glu Met Glu Glu
    1445                1450                1455

Gly Glu Phe Lys Asn Trp Ile Glu Lys Met Gln Ala Arg Leu Met
    1460                1465                1470

Ser Ser Ser Val Asp Thr Pro Gln Gln Leu Gln Ser Val Phe Glu
    1475                1480                1485

Ser Leu Ile Ala Lys Lys Gln Ser Leu Cys Glu Val Leu Gln Ala
    1490                1495                1500

Trp Asn Asn Arg Leu Gln Asp Leu Phe Gln Gln Glu Lys Gly Arg
    1505                1510                1515

Lys Arg Pro Ser Val Pro Pro Ser Pro Gly Arg Leu Arg Gln Gly
    1520                1525                1530

Glu Glu Ser Lys Ile Ser Ala Met Asp Ala Ser Pro Arg Asn Ile
    1535                1540                1545

Ser Pro Gly Leu Gln Asn Gly Glu Lys Glu Asp Arg Phe Leu Thr

1550                1555                1560

Thr Leu Ser Ser Gln Ser Ser Thr Ser Ser Thr His Leu Gln Leu
    1565                1570                1575

Pro Thr Pro Pro Glu Val Met Ser Glu Gln Ser Val Gly Gly Pro
    1580                1585                1590

Pro Glu Leu Asp Thr Ala Ser Ser Ser Glu Asp Val Phe Asp Gly
    1595                1600                1605

His Leu Leu Gly Ser Thr Asp Ser Gln Val Lys Glu Lys Ser Thr
    1610                1615                1620

Met Lys Ala Ile Phe Ala Asn Leu Leu Pro Gly Asn Ser Tyr Asn
    1625                1630                1635

Pro Ile Pro Phe Pro Phe Asp Pro Asp Lys His Tyr Leu Met Tyr
    1640                1645                1650

Glu His Glu Arg Val Pro Ile Ala Val Cys Glu Lys Glu Pro Ser
    1655                1660                1665

Ser Ile Ile Ala Phe Ala Leu Ser Cys Lys Glu Tyr Arg Asn Ala
    1670                1675                1680

Leu Glu Glu Leu Ser Lys Ala Thr Gln Trp Asn Ser Ala Glu Glu
    1685                1690                1695

Gly Leu Pro Thr Asn Ser Thr Ser Asp Ser Arg Pro Lys Ser Ser
    1700                1705                1710

Ser Pro Ile Arg Leu Pro Glu Met Ser Gly Gly Gln Thr Asn Arg
    1715                1720                1725

Thr Thr Glu Thr Glu Pro Gln Pro Thr Lys Lys Ala Ser Gly Met
    1730                1735                1740

Leu Ser Phe Phe Arg Gly Thr Ala Gly Lys Ser Pro Asp Leu Ser
    1745                1750                1755

Ser Gln Lys Arg Glu Thr Leu Arg Gly Ala Asp Ser Ala Tyr Tyr
    1760                1765                1770

Gln Val Gly Gln Thr Gly Lys Glu Gly Thr Glu Asn Gln Gly Val
    1775                1780                1785

Glu Pro Gln Asp Glu Val Asp Gly Gly Asp Thr Gln Lys Lys Gln
    1790                1795                1800

Leu Ile Asn Pro His Val Glu Leu Gln Phe Ser Asp Ala Asn Ala
    1805                1810                1815

Lys Phe Tyr Cys Arg Leu Tyr Tyr Ala Gly Glu Phe His Lys Met
    1820                1825                1830

Arg Glu Val Ile Leu Asp Ser Ser Glu Glu Asp Phe Ile Arg Ser
    1835                1840                1845

Leu Ser His Ser Ser Pro Trp Gln Ala Arg Gly Gly Lys Ser Gly
    1850                1855                1860

Ala Ala Phe Tyr Ala Thr Glu Asp Asp Arg Phe Ile Leu Lys Gln
    1865                1870                1875

Met Pro Arg Leu Glu Val Gln Ser Phe Leu Asp Phe Ala Pro His
    1880                1885                1890

Tyr Phe Asn Tyr Ile Thr Asn Ala Val Gln Gln Lys Arg Pro Thr
    1895                1900                1905

Ala Leu Ala Lys Ile Leu Gly Val Tyr Arg Ile Gly Tyr Lys Asn
    1910                1915                1920

Ser Gln Asn Asn Thr Glu Lys Lys Leu Asp Leu Leu Val Met Glu
    1925                1930                1935

Asn Leu Phe Tyr Gly Arg Lys Met Ala Gln Val Phe Asp Leu Lys
    1940                1945                1950

Gly Ser Leu Arg Asn Arg Asn Val Lys Thr Asp Thr Gly Lys Glu
    1955                1960                1965

Ser Cys Asp Val Val Leu Leu Asp Glu Asn Leu Leu Lys Met Val
    1970                1975                1980

Arg Asp Asn Pro Leu Tyr Ile Arg Ser His Ser Lys Ala Val Leu
    1985                1990                1995

Arg Thr Ser Ile His Ser Asp Ser His Phe Leu Ser Ser His Leu
    2000                2005                2010

Ile Ile Asp Tyr Ser Leu Leu Val Gly Arg Asp Asp Thr Ser Asn
    2015                2020                2025

Glu Leu Val Val Gly Ile Ile Asp Tyr Ile Arg Thr Phe Thr Trp
    2030                2035                2040

Asp Lys Lys Leu Glu Met Val Val Lys Ser Thr Gly Ile Leu Gly
    2045                2050                2055

Gly Gln Gly Lys Met Pro Thr Val Val Ser Pro Glu Leu Tyr Arg
    2060                2065                2070

Thr Arg Phe Cys Glu Ala Met Asp Lys Tyr Phe Leu Met Val Pro
    2075                2080                2085

Asp His Trp Thr Gly Leu Gly Leu Asn Cys
    2090                2095

<210> 3
<211> 6159
<212> DNA
<213> mouse PIKfyve

<220>
<221> CDS
<222> (1)..(6159)

<400> 3
atg gcc aca gat gac aag agt tcc ccg aca ctg gac tct gct aat gat 48
Met Ala Thr Asp Asp Lys Ser Ser Pro Thr Leu Asp Ser Ala Asn Asp
1               5                   10                  15

ttg cct cgc tct cct gcc agt cct tct cac ctc act cac ttt aaa ccc 96
Leu Pro Arg Ser Pro Ala Ser Pro Ser His Leu Thr His Phe Lys Pro
            20                  25                  30

ttg act cct gac cag gat gag ccc ccc ttc aag tca gca tat agt tct 144
Leu Thr Pro Asp Gln Asp Glu Pro Pro Phe Lys Ser Ala Tyr Ser Ser
        35                  40                  45

ttt gta aac ctt ttt cgt ttt aac aaa gag cga gga gaa ggg ggc caa 192
Phe Val Asn Leu Phe Arg Phe Asn Lys Glu Arg Gly Glu Gly Gly Gln
    50                  55                  60

gga gag cag cag tct ccg agt tca agt tgg gcc agc cct cag atc cct 240
Gly Glu Gln Gln Ser Pro Ser Ser Ser Trp Ala Ser Pro Gln Ile Pro
65                  70                  75                  80

tca aga aca cag tct gtg agg tcg cct gta cct tat aaa aaa cag ctt 288
Ser Arg Thr Gln Ser Val Arg Ser Pro Val Pro Tyr Lys Lys Gln Leu
                85                  90                  95

aat gag gag ctc cac cgg cgc tct tca gtg tta gag aac act ttg cca 336
Asn Glu Glu Leu His Arg Arg Ser Ser Val Leu Glu Asn Thr Leu Pro
            100                 105                 110

cat cct cag gag agc aca gac tcc aga agg aaa gca gaa cca gcc tgt 384
His Pro Gln Glu Ser Thr Asp Ser Arg Arg Lys Ala Glu Pro Ala Cys
        115                 120                 125

gga ggt cat gac cca cgt aca gct gtt cag ctt cga agc ctc agc aca 432
Gly Gly His Asp Pro Arg Thr Ala Val Gln Leu Arg Ser Leu Ser Thr
    130                 135                 140

gta ttg aaa cgc ctc aaa gaa atc atg gaa gga aaa agc cag gac agt 480

Val Leu Lys Arg Leu Lys Glu Ile Met Glu Gly Lys Ser Gln Asp Ser
145                 150                 155                 160

gac ctg aag caa tat tgg atg cca gat agc cag tgt aaa gag tgc tat      528
Asp Leu Lys Gln Tyr Trp Met Pro Asp Ser Gln Cys Lys Glu Cys Tyr
                165                 170                 175

gac tgc agt gaa aag ttt aca aca ttt agg cgc aga cac cat tgc aga      576
Asp Cys Ser Glu Lys Phe Thr Thr Phe Arg Arg Arg His His Cys Arg
            180                 185                 190

ctg tgt ggg cag att ttc tgc agt cgt tgt tgt aat caa gaa atc cct      624
Leu Cys Gly Gln Ile Phe Cys Ser Arg Cys Cys Asn Gln Glu Ile Pro
        195                 200                 205

gga aaa ttt atg ggc tat aca gga gac ctc cga gca tgc acc tac tgt      672
Gly Lys Phe Met Gly Tyr Thr Gly Asp Leu Arg Ala Cys Thr Tyr Cys
    210                 215                 220

aga aaa ata gcc tta agt tat gct cat tct aca gac agt aat tcc att      720
Arg Lys Ile Ala Leu Ser Tyr Ala His Ser Thr Asp Ser Asn Ser Ile
225                 230                 235                 240

ggg gaa gac ttg aat gct ctt tca gat tca act tgc tct gtg tct ata      768
Gly Glu Asp Leu Asn Ala Leu Ser Asp Ser Thr Cys Ser Val Ser Ile
                245                 250                 255

ctt gat cca agc gaa cct cgg aca cca gtt ggg agt aga aaa gcc agt      816
Leu Asp Pro Ser Glu Pro Arg Thr Pro Val Gly Ser Arg Lys Ala Ser
            260                 265                 270

cgt aac ata ttc tta gag gat gat tta gcc tgg caa agc ttg att cat      864
Arg Asn Ile Phe Leu Glu Asp Asp Leu Ala Trp Gln Ser Leu Ile His
        275                 280                 285

ccg gat tcc tca aat agt gct ctt tca aca aga ctc gta tct gtt caa      912
Pro Asp Ser Ser Asn Ser Ala Leu Ser Thr Arg Leu Val Ser Val Gln
    290                 295                 300

gag gat gct ggg aag tct cct gct cga aac aga tca gcc agc att act      960
Glu Asp Ala Gly Lys Ser Pro Ala Arg Asn Arg Ser Ala Ser Ile Thr
305                 310                 315                 320

aat ctg tca ctg gat cgg tct ggt tct cct atg gtt cct tca tat gag     1008
Asn Leu Ser Leu Asp Arg Ser Gly Ser Pro Met Val Pro Ser Tyr Glu
                325                 330                 335

aca tct gtc agt ccc cag gct aac cga aac tac att agg aca gag acg     1056
Thr Ser Val Ser Pro Gln Ala Asn Arg Asn Tyr Ile Arg Thr Glu Thr
            340                 345                 350

act gag gat gaa cgc aaa att ctt ctg gac agt gct cag tta aag gat     1104
Thr Glu Asp Glu Arg Lys Ile Leu Leu Asp Ser Ala Gln Leu Lys Asp
        355                 360                 365

ctg tgg aag aaa atc tgc cat cac acc agt ggg atg gaa ttt caa gat     1152
Leu Trp Lys Lys Ile Cys His His Thr Ser Gly Met Glu Phe Gln Asp
    370                 375                 380

cac cgt tac tgg ttg aga aca cat ccc aac tgc att gta ggg aag gaa     1200
His Arg Tyr Trp Leu Arg Thr His Pro Asn Cys Ile Val Gly Lys Glu
385                 390                 395                 400

tta gtc aac tgg cta atc aga aat gga cac atc gct aca agg gca caa     1248
Leu Val Asn Trp Leu Ile Arg Asn Gly His Ile Ala Thr Arg Ala Gln

405                 410                 415

gct ata gca att gga caa gca atg gtt gat gga cgt tgg ttg gat tgt        1296
Ala Ile Ala Ile Gly Gln Ala Met Val Asp Gly Arg Trp Leu Asp Cys
            420                 425                 430

gtt agt cat cat gat cag ctt ttc agg gac gaa tat gcg ttg tat aga        1344
Val Ser His His Asp Gln Leu Phe Arg Asp Glu Tyr Ala Leu Tyr Arg
        435                 440                 445

cca ctt cag agt aca gaa ttt tct gag aca cct tct cca gac agt gac        1392
Pro Leu Gln Ser Thr Glu Phe Ser Glu Thr Pro Ser Pro Asp Ser Asp
    450                 455                 460

tct gtg aac tct gtg gaa gga cac tcc gag cca tcc tgg ttt aaa gac        1440
Ser Val Asn Ser Val Glu Gly His Ser Glu Pro Ser Trp Phe Lys Asp
465                 470                 475                 480

ata aaa ttt gat gac agt gac aca gaa cag att gct gaa gaa ggt gac        1488
Ile Lys Phe Asp Asp Ser Asp Thr Glu Gln Ile Ala Glu Glu Gly Asp
                485                 490                 495

gat aat ttg gct aag tat ttg gtt tct gac act gga gga cag cag ctc        1536
Asp Asn Leu Ala Lys Tyr Leu Val Ser Asp Thr Gly Gly Gln Gln Leu
            500                 505                 510

tca ata agt gat gcc ttc atc aaa gag tcc tta ttt aat cga cga gta        1584
Ser Ile Ser Asp Ala Phe Ile Lys Glu Ser Leu Phe Asn Arg Arg Val
        515                 520                 525

gag gaa aaa tcc aaa gag ctg cct ttt acc cct ttg ggc tgg cat cat        1632
Glu Glu Lys Ser Lys Glu Leu Pro Phe Thr Pro Leu Gly Trp His His
    530                 535                 540

aac aac ctg gaa ctc ttg cga gag gag aat gag gag aag caa gcc atg        1680
Asn Asn Leu Glu Leu Leu Arg Glu Glu Asn Glu Glu Lys Gln Ala Met
545                 550                 555                 560

gaa agg ctg ctt tca gct aat cat aac cac atg atg gcc cta ctc cag        1728
Glu Arg Leu Leu Ser Ala Asn His Asn His Met Met Ala Leu Leu Gln
                565                 570                 575

cag ttg ctt caa aac gag tca ttg tca tcg tct tgg agg gac atc att        1776
Gln Leu Leu Gln Asn Glu Ser Leu Ser Ser Ser Trp Arg Asp Ile Ile
            580                 585                 590

gtg tca cta gtc tgc cag gtt gtt cag aca gtc cga cct gat gtc aag        1824
Val Ser Leu Val Cys Gln Val Val Gln Thr Val Arg Pro Asp Val Lys
        595                 600                 605

cac cag gat gat gac atg gat atc cgt cag ttt gtc cat atc aag aag        1872
His Gln Asp Asp Asp Met Asp Ile Arg Gln Phe Val His Ile Lys Lys
    610                 615                 620

atc cca ggt gga aag aaa ttt gac tct gtg gtt gtc aat ggc ttt gtt        1920
Ile Pro Gly Gly Lys Lys Phe Asp Ser Val Val Val Asn Gly Phe Val
625                 630                 635                 640

tgt acc aag aac att gca cat aaa aag atg aat tcc tgt att aaa aac        1968
Cys Thr Lys Asn Ile Ala His Lys Lys Met Asn Ser Cys Ile Lys Asn
                645                 650                 655

ccc aaa atc ctt ctg ttg aag tgt tct att gag tat ctc tat aga gaa        2016
Pro Lys Ile Leu Leu Leu Lys Cys Ser Ile Glu Tyr Leu Tyr Arg Glu
            660                 665                 670

gaa act aag ttt acc tgc att gat cct att gtg ctt cag gaa agg gaa 2064
Glu Thr Lys Phe Thr Cys Ile Asp Pro Ile Val Leu Gln Glu Arg Glu
        675                 680                 685

ttc ttg aag aat tat gtt caa cga ata gtt gat gtt cga ccc aca ttg 2112
Phe Leu Lys Asn Tyr Val Gln Arg Ile Val Asp Val Arg Pro Thr Leu
    690                 695                 700

gtt ctt gtt gag aaa aca gtg tct cgg att gct cag gac atg tta ctg 2160
Val Leu Val Glu Lys Thr Val Ser Arg Ile Ala Gln Asp Met Leu Leu
705                 710                 715                 720

gaa cat ggc att act ctg gtc att aat gta aag tca caa gtt tta gaa 2208
Glu His Gly Ile Thr Leu Val Ile Asn Val Lys Ser Gln Val Leu Glu
                725                 730                 735

aga atc agt cga atg acc caa ggt gat tta gtg gtg tcc atg gac cag 2256
Arg Ile Ser Arg Met Thr Gln Gly Asp Leu Val Val Ser Met Asp Gln
            740                 745                 750

ctg ctc acc aaa ccc cac ttg ggc act tgc cac aaa ttt tat atg cag 2304
Leu Leu Thr Lys Pro His Leu Gly Thr Cys His Lys Phe Tyr Met Gln
        755                 760                 765

ata ttt cag ctg cct aat gaa caa acc aaa aca ctg atg ttt ttt gaa 2352
Ile Phe Gln Leu Pro Asn Glu Gln Thr Lys Thr Leu Met Phe Phe Glu
    770                 775                 780

ggt tgt cca cag cat cta ggc tgc aca atc aag ctc aga gga ggc tct 2400
Gly Cys Pro Gln His Leu Gly Cys Thr Ile Lys Leu Arg Gly Gly Ser
785                 790                 795                 800

gat tat gag ctg gct cga gtt aag gag atc cta ata ttt atg atc tgt 2448
Asp Tyr Glu Leu Ala Arg Val Lys Glu Ile Leu Ile Phe Met Ile Cys
                805                 810                 815

gta gct tat cat tct cag cta gaa atc tct ttt ctc atg gat gag ttc 2496
Val Ala Tyr His Ser Gln Leu Glu Ile Ser Phe Leu Met Asp Glu Phe
            820                 825                 830

gct atg cct cca aca tta atg caa agc cct tca ttc cat ctt ctg acg 2544
Ala Met Pro Pro Thr Leu Met Gln Ser Pro Ser Phe His Leu Leu Thr
        835                 840                 845

gag gga cga ggt gaa gag gga gcc tct cag gag cag gtc agt ggc agc 2592
Glu Gly Arg Gly Glu Glu Gly Ala Ser Gln Glu Gln Val Ser Gly Ser
    850                 855                 860

tcc ctt cct cag gat cct gag tgc cct cgt gag gcc ctg tct tct gag 2640
Ser Leu Pro Gln Asp Pro Glu Cys Pro Arg Glu Ala Leu Ser Ser Glu
865                 870                 875                 880

gat agc act ttg ttg gaa tca agg act gtg cta gag aag ggt gaa cta 2688
Asp Ser Thr Leu Leu Glu Ser Arg Thr Val Leu Glu Lys Gly Glu Leu
                885                 890                 895

gac aat aaa agt att cca caa gct gtt gcc tct ttg aag cat caa gat 2736
Asp Asn Lys Ser Ile Pro Gln Ala Val Ala Ser Leu Lys His Gln Asp
            900                 905                 910

tat acc acc ccc act tgc cca gca ggt att ccc tgt gct ctt ttt gca 2784
Tyr Thr Thr Pro Thr Cys Pro Ala Gly Ile Pro Cys Ala Leu Phe Ala
        915                 920                 925

ttg gta cca gag tca ttg ttg cct ctc cat atg gat caa cag gat gcc 2832
Leu Val Pro Glu Ser Leu Leu Pro Leu His Met Asp Gln Gln Asp Ala
    930                 935                 940

gta gga aat gaa cac cga gag act tca cag caa acg gat gag caa cag 2880
Val Gly Asn Glu His Arg Glu Thr Ser Gln Gln Thr Asp Glu Gln Gln
945                 950                 955                 960

gat ccc aaa agc cag atg aaa gct ttt aga gac cct tta cag gat gac 2928
Asp Pro Lys Ser Gln Met Lys Ala Phe Arg Asp Pro Leu Gln Asp Asp
                965                 970                 975

act gga atg tac gtt act gag gaa gtc acc tcc tct gaa gat caa cga 2976
Thr Gly Met Tyr Val Thr Glu Glu Val Thr Ser Ser Glu Asp Gln Arg
            980                 985                 990

aag act tat gcc ttg aca ttt aaa cag gag tta aaa gat gta atc ctc 3024
Lys Thr Tyr Ala Leu Thr Phe Lys Gln Glu Leu Lys Asp Val Ile Leu
        995                 1000                1005

tgt atc tct cca gtt att aca ttc cgt gaa cct ttc ctt tta act 3069
Cys Ile Ser Pro Val Ile Thr Phe Arg Glu Pro Phe Leu Leu Thr
    1010                1015                1020

gaa aag ggg atg aga tgc tca act cga gat tat ttt cca gag cag 3114
Glu Lys Gly Met Arg Cys Ser Thr Arg Asp Tyr Phe Pro Glu Gln
    1025                1030                1035

att tac tgg tct cct ctt ctc aac aaa gag gtg aag gaa atg gag 3159
Ile Tyr Trp Ser Pro Leu Leu Asn Lys Glu Val Lys Glu Met Glu
    1040                1045                1050

agc agg agg aag aaa cag ctg ctc agg gat ctc tct gga ctt cag 3204
Ser Arg Arg Lys Lys Gln Leu Leu Arg Asp Leu Ser Gly Leu Gln
    1055                1060                1065

ggc atg aat ggc agt gtt cag gcc aag tct att caa gtc tta ccc 3249
Gly Met Asn Gly Ser Val Gln Ala Lys Ser Ile Gln Val Leu Pro
    1070                1075                1080

tca cat gag cta gtg agc acc agg att gct gaa cat gtg ggt gac 3294
Ser His Glu Leu Val Ser Thr Arg Ile Ala Glu His Val Gly Asp
    1085                1090                1095

agc cag acc ttg ggt aga atg cta gct gat tat cga gct aga gga 3339
Ser Gln Thr Leu Gly Arg Met Leu Ala Asp Tyr Arg Ala Arg Gly
    1100                1105                1110

gga gaa ttc agt caa aac att tgg aac ccc ttt gtc cat tca aaa 3384
Gly Glu Phe Ser Gln Asn Ile Trp Asn Pro Phe Val His Ser Lys
    1115                1120                1125

gat gac atc atg tac ttc agg tgg caa atc agg gaa aca aaa ctg 3429
Asp Asp Ile Met Tyr Phe Arg Trp Gln Ile Arg Glu Thr Lys Leu
    1130                1135                1140

aga gtg atg aaa gag agg gga ttg att cca agt gat gta ata tgg 3474
Arg Val Met Lys Glu Arg Gly Leu Ile Pro Ser Asp Val Ile Trp
    1145                1150                1155

cca aca aag gtg gac tgt ctg aac cct gct aac cac cag agg ctc 3519
Pro Thr Lys Val Asp Cys Leu Asn Pro Ala Asn His Gln Arg Leu
    1160                1165                1170

tgt gtg ctc ttc agc agc tct tct gcc cag tcc agc aat gct ccc 3564

Cys Val Leu Phe Ser Ser Ser Ser Ala Gln Ser Ser Asn Ala Pro
    1175                1180                1185

agt gct tgt gtc agt cct tgg att gta aca atg gag ttt tat gga 3609
Ser Ala Cys Val Ser Pro Trp Ile Val Thr Met Glu Phe Tyr Gly
    1190                1195                1200

aag aat gac ctt aca ctg ggg ata ttt tta gaa aga tac tgt ttc 3654
Lys Asn Asp Leu Thr Leu Gly Ile Phe Leu Glu Arg Tyr Cys Phe
    1205                1210                1215

agg tac tct tac cag tgt ccg agc atg ttc tgt gac acc ccc atg 3699
Arg Tyr Ser Tyr Gln Cys Pro Ser Met Phe Cys Asp Thr Pro Met
    1220                1225                1230

gtt cat cac att cga cgc ttt gtt cat ggc caa ggc tgt gta cag 3744
Val His His Ile Arg Arg Phe Val His Gly Gln Gly Cys Val Gln
    1235                1240                1245

ata att ctg aag gag ttg gat tct cca gtg cct gga tat caa cat 3789
Ile Ile Leu Lys Glu Leu Asp Ser Pro Val Pro Gly Tyr Gln His
    1250                1255                1260

aca att ctc aca tat tcc tgg tgc aga atc tgc aaa caa gta aca 3834
Thr Ile Leu Thr Tyr Ser Trp Cys Arg Ile Cys Lys Gln Val Thr
    1265                1270                1275

cca gtt gtt gct ctt tca aat gaa tcc tgg tct atg tca ttt gca 3879
Pro Val Val Ala Leu Ser Asn Glu Ser Trp Ser Met Ser Phe Ala
    1280                1285                1290

aag tac ctt gaa ctt cga ttt tat ggc cac cag tac aca cgc aga 3924
Lys Tyr Leu Glu Leu Arg Phe Tyr Gly His Gln Tyr Thr Arg Arg
    1295                1300                1305

gcc aac gct gag ccc tgc ggt cac tct atc cac cat gat tat cac 3969
Ala Asn Ala Glu Pro Cys Gly His Ser Ile His His Asp Tyr His
    1310                1315                1320

cag tat ttc tct tat aac cag atg gtg gca tct ttc agt tac tct 4014
Gln Tyr Phe Ser Tyr Asn Gln Met Val Ala Ser Phe Ser Tyr Ser
    1325                1330                1335

cct att cgg ctt ctt gaa gta tgt gtt cca cta cca aaa ata ttc 4059
Pro Ile Arg Leu Leu Glu Val Cys Val Pro Leu Pro Lys Ile Phe
    1340                1345                1350

att aag cgt caa gcc cca ctg aag gta tct ctt ctt cag gac ctc 4104
Ile Lys Arg Gln Ala Pro Leu Lys Val Ser Leu Leu Gln Asp Leu
    1355                1360                1365

aaa gac ttt ttt cag aag gtt tca cag gtg tac cta gct gtt gat 4149
Lys Asp Phe Phe Gln Lys Val Ser Gln Val Tyr Leu Ala Val Asp
    1370                1375                1380

gag aga ctt gca tcc ttg aaa acg gat aca ttt agc aaa act aga 4194
Glu Arg Leu Ala Ser Leu Lys Thr Asp Thr Phe Ser Lys Thr Arg
    1385                1390                1395

gag gaa aag atg gaa gat atc ttt gca caa aag gag atg gaa gag 4239
Glu Glu Lys Met Glu Asp Ile Phe Ala Gln Lys Glu Met Glu Glu
    1400                1405                1410

ggt gag ttt aag aac tgg aca gag aag atg caa gca agg ctc atg 4284
Gly Glu Phe Lys Asn Trp Thr Glu Lys Met Gln Ala Arg Leu Met

1415                    1420                    1425

tct tcc tct gtg gat acc cct cag caa ctg cag tcc att ttt gag          4329
Ser Ser Ser Val Asp Thr Pro Gln Gln Leu Gln Ser Ile Phe Glu
    1430                    1435                    1440

tca ctg att gcc aag aag caa agc ctc tgt gag gtg ctc cag gcg          4374
Ser Leu Ile Ala Lys Lys Gln Ser Leu Cys Glu Val Leu Gln Ala
    1445                    1450                    1455

tgg aac agc agg ttg cag gac ctc ttc cag cag gaa aaa ggt aga          4419
Trp Asn Ser Arg Leu Gln Asp Leu Phe Gln Gln Glu Lys Gly Arg
    1460                    1465                    1470

aag agg cct tca gtt cct ccc agt cct ggg aga ctg aga caa ggt          4464
Lys Arg Pro Ser Val Pro Pro Ser Pro Gly Arg Leu Arg Gln Gly
    1475                    1480                    1485

gaa gaa agc aag ata aat gca atg gac aca tct cca agg aat att          4509
Glu Glu Ser Lys Ile Asn Ala Met Asp Thr Ser Pro Arg Asn Ile
    1490                    1495                    1500

tct cca gga ctt tca caa tgg aga aaa aga aga tcg ctt ctt gac          4554
Ser Pro Gly Leu Ser Gln Trp Arg Lys Arg Arg Ser Leu Leu Asp
    1505                    1510                    1515

aac cct gtc cag cca gct acg agc tcc acc cac ctc cag ctg ccc          4599
Asn Pro Val Gln Pro Ala Thr Ser Ser Thr His Leu Gln Leu Pro
    1520                    1525                    1530

act cct ccc gag gcc ctg gcc gag cag gta gtg gga ggg ccg act          4644
Thr Pro Pro Glu Ala Leu Ala Glu Gln Val Val Gly Gly Pro Thr
    1535                    1540                    1545

gat ctg gat tca gcc agt ggc tct gaa gat gta ttt gat ggt cat          4689
Asp Leu Asp Ser Ala Ser Gly Ser Glu Asp Val Phe Asp Gly His
    1550                    1555                    1560

ttg ctg gga tcc aca gac agc cag gtg aag gaa aag tca acc atg          4734
Leu Leu Gly Ser Thr Asp Ser Gln Val Lys Glu Lys Ser Thr Met
    1565                    1570                    1575

aaa gcc atc ttt gct aat ttg ctt cca gga aac agc tac aat ccc          4779
Lys Ala Ile Phe Ala Asn Leu Leu Pro Gly Asn Ser Tyr Asn Pro
    1580                    1585                    1590

att cca ttt cct ttt gat cca gat aaa cac tac tta atg tat gaa          4824
Ile Pro Phe Pro Phe Asp Pro Asp Lys His Tyr Leu Met Tyr Glu
    1595                    1600                    1605

cat gaa cgg gtg ccc att gct gtc tgt gag aaa gag ccc agc tcc          4869
His Glu Arg Val Pro Ile Ala Val Cys Glu Lys Glu Pro Ser Ser
    1610                    1615                    1620

atc att gct ttt gca ctc agt tgt aaa gaa tac cgc aat gcc tta          4914
Ile Ile Ala Phe Ala Leu Ser Cys Lys Glu Tyr Arg Asn Ala Leu
    1625                    1630                    1635

gag gaa ttg tcc aaa gca act ctg cgg aac agt gct gaa gaa ggg          4959
Glu Glu Leu Ser Lys Ala Thr Leu Arg Asn Ser Ala Glu Glu Gly
    1640                    1645                    1650

ctc cca gcc aat agt gct tta gat aac aga cct aag agt agc agc          5004
Leu Pro Ala Asn Ser Ala Leu Asp Asn Arg Pro Lys Ser Ser Ser
    1655                    1660                    1665

cct att aga cta cct gaa atc agt gga gga cag aca aac cgc aca 5049
Pro Ile Arg Leu Pro Glu Ile Ser Gly Gly Gln Thr Asn Arg Thr
    1670                1675                1680

gta gaa gca gaa cct cag cca acc aaa aaa gct tca gga atg ttg 5094
Val Glu Ala Glu Pro Gln Pro Thr Lys Lys Ala Ser Gly Met Leu
    1685                1690                1695

tcc ttc ttc aga gga aca gca ggg aag agc cct gat ctg tct tcc 5139
Ser Phe Phe Arg Gly Thr Ala Gly Lys Ser Pro Asp Leu Ser Ser
    1700                1705                1710

cag aag agg gag acc ttg cga ggg gca gac agt gct tac tac cag 5184
Gln Lys Arg Glu Thr Leu Arg Gly Ala Asp Ser Ala Tyr Tyr Gln
    1715                1720                1725

gtt ggg cag gcc ggc aag gag ggg ttg gag agt caa ggc ctg gag 5229
Val Gly Gln Ala Gly Lys Glu Gly Leu Glu Ser Gln Gly Leu Glu
    1730                1735                1740

cct caa gat gaa gta gat gga gga gat aca cag aag aaa caa ctc 5274
Pro Gln Asp Glu Val Asp Gly Gly Asp Thr Gln Lys Lys Gln Leu
    1745                1750                1755

aca aat cct cat gtg gaa ctt caa ttt tct gat gct aat gcc aag 5319
Thr Asn Pro His Val Glu Leu Gln Phe Ser Asp Ala Asn Ala Lys
    1760                1765                1770

ttt tac tgt cgg ctg tac tac gcg gga gag ttc cac aag atg cgt 5364
Phe Tyr Cys Arg Leu Tyr Tyr Ala Gly Glu Phe His Lys Met Arg
    1775                1780                1785

gaa gtg att ctg ggc agc agt gag gag gaa ttc atc cgt tcc ctt 5409
Glu Val Ile Leu Gly Ser Ser Glu Glu Glu Phe Ile Arg Ser Leu
    1790                1795                1800

tct cac tca tct ccc tgg cag gcc cgg gga ggc aag tca gga gct 5454
Ser His Ser Ser Pro Trp Gln Ala Arg Gly Gly Lys Ser Gly Ala
    1805                1810                1815

gct ttc tat gcc acc gaa gat gat aga ttc att ctg aag caa atg 5499
Ala Phe Tyr Ala Thr Glu Asp Asp Arg Phe Ile Leu Lys Gln Met
    1820                1825                1830

cct cgt ttg gaa gtc cag tct ttc ctt gac ttt gca cca cac tac 5544
Pro Arg Leu Glu Val Gln Ser Phe Leu Asp Phe Ala Pro His Tyr
    1835                1840                1845

ttc aat tat atc aca aat gct gtt caa caa aag agg ccc acc gcc 5589
Phe Asn Tyr Ile Thr Asn Ala Val Gln Gln Lys Arg Pro Thr Ala
    1850                1855                1860

ttg gct aaa att ctt gga gtt tac aga att ggt tat aag aac tct 5634
Leu Ala Lys Ile Leu Gly Val Tyr Arg Ile Gly Tyr Lys Asn Ser
    1865                1870                1875

cag aac aac act gag aag aag tta gat ctc ctt gtc atg gaa aat 5679
Gln Asn Asn Thr Glu Lys Lys Leu Asp Leu Leu Val Met Glu Asn
    1880                1885                1890

ctt ttc tat ggg aga aag atg gca cag gtt ttt gat ttg aag ggt 5724
Leu Phe Tyr Gly Arg Lys Met Ala Gln Val Phe Asp Leu Lys Gly
    1895                1900                1905

tca ctt agg aat cga aat gta aaa act gac act ggg aaa gag agc 5769
Ser Leu Arg Asn Arg Asn Val Lys Thr Asp Thr Gly Lys Glu Ser
    1910                1915                1920

tgt gat gtg gtt ctg ttg gat gaa aac ctc cta aag atg gtt cga 5814
Cys Asp Val Val Leu Leu Asp Glu Asn Leu Leu Lys Met Val Arg
    1925                1930                1935

gac aac cct ctc tat att cgt tcc cat tcc aaa tct gag ctg aga 5859
Asp Asn Pro Leu Tyr Ile Arg Ser His Ser Lys Ser Glu Leu Arg
    1940                1945                1950

acc tcc atc cac agc gac gcc cat ttc ctt tcc agc cac ctc att 5904
Thr Ser Ile His Ser Asp Ala His Phe Leu Ser Ser His Leu Ile
    1955                1960                1965

ata gac tat tct ctg ctg gtt ggg cga gat gac act agc aat gag 5949
Ile Asp Tyr Ser Leu Leu Val Gly Arg Asp Asp Thr Ser Asn Glu
    1970                1975                1980

ctt gtg gtt ggc atc ata gat tac att cga aca ttt aca tgg gac 5994
Leu Val Val Gly Ile Ile Asp Tyr Ile Arg Thr Phe Thr Trp Asp
    1985                1990                1995

aaa aaa ctt gag atg gtt gtg aag tca aca gga att tta gga gga 6039
Lys Lys Leu Glu Met Val Val Lys Ser Thr Gly Ile Leu Gly Gly
    2000                2005                2010

caa ggt aaa atg cca act gtg gtc tct cca gag ttg tat agg act 6084
Gln Gly Lys Met Pro Thr Val Val Ser Pro Glu Leu Tyr Arg Thr
    2015                2020                2025

aga ttt tgt gaa gca atg gac aag tat ttc ttg atg gtg cca gac 6129
Arg Phe Cys Glu Ala Met Asp Lys Tyr Phe Leu Met Val Pro Asp
    2030                2035                2040

cac tgg aca ggg ttg gat ctg aat tgc tga 6159
His Trp Thr Gly Leu Asp Leu Asn Cys
    2045                2050

<210> 4
<211> 2052
<212> PRT
<213> mouse PIKfyve

<400> 4

Met Ala Thr Asp Asp Lys Ser Ser Pro Thr Leu Asp Ser Ala Asn Asp
1               5                   10                  15

Leu Pro Arg Ser Pro Ala Ser Pro Ser His Leu Thr His Phe Lys Pro
            20                  25                  30

Leu Thr Pro Asp Gln Asp Glu Pro Pro Phe Lys Ser Ala Tyr Ser Ser
        35                  40                  45

Phe Val Asn Leu Phe Arg Phe Asn Lys Glu Arg Gly Glu Gly Gly Gln
    50                  55                  60

Gly Glu Gln Gln Ser Pro Ser Ser Ser Trp Ala Ser Pro Gln Ile Pro

65                  70                  75                  80

Ser Arg Thr Gln Ser Val Arg Ser Pro Val Pro Tyr Lys Lys Gln Leu
                85                  90                  95

Asn Glu Glu Leu His Arg Arg Ser Ser Val Leu Glu Asn Thr Leu Pro
            100                 105                 110

His Pro Gln Glu Ser Thr Asp Ser Arg Arg Lys Ala Glu Pro Ala Cys
        115                 120                 125

Gly Gly His Asp Pro Arg Thr Ala Val Gln Leu Arg Ser Leu Ser Thr
    130                 135                 140

Val Leu Lys Arg Leu Lys Glu Ile Met Glu Gly Lys Ser Gln Asp Ser
145                 150                 155                 160

Asp Leu Lys Gln Tyr Trp Met Pro Asp Ser Gln Cys Lys Glu Cys Tyr
                165                 170                 175

Asp Cys Ser Glu Lys Phe Thr Thr Phe Arg Arg Arg His His Cys Arg
            180                 185                 190

Leu Cys Gly Gln Ile Phe Cys Ser Arg Cys Cys Asn Gln Glu Ile Pro
        195                 200                 205

Gly Lys Phe Met Gly Tyr Thr Gly Asp Leu Arg Ala Cys Thr Tyr Cys
    210                 215                 220

Arg Lys Ile Ala Leu Ser Tyr Ala His Ser Thr Asp Ser Asn Ser Ile
225                 230                 235                 240

Gly Glu Asp Leu Asn Ala Leu Ser Asp Ser Thr Cys Ser Val Ser Ile
                245                 250                 255

Leu Asp Pro Ser Glu Pro Arg Thr Pro Val Gly Ser Arg Lys Ala Ser
            260                 265                 270

Arg Asn Ile Phe Leu Glu Asp Asp Leu Ala Trp Gln Ser Leu Ile His
        275                 280                 285

Pro Asp Ser Ser Asn Ser Ala Leu Ser Thr Arg Leu Val Ser Val Gln
    290                 295                 300

Glu Asp Ala Gly Lys Ser Pro Ala Arg Asn Arg Ser Ala Ser Ile Thr
305                 310                 315                 320

Asn Leu Ser Leu Asp Arg Ser Gly Ser Pro Met Val Pro Ser Tyr Glu
                325                 330                 335

Thr Ser Val Ser Pro Gln Ala Asn Arg Asn Tyr Ile Arg Thr Glu Thr
            340                 345                 350

Thr Glu Asp Glu Arg Lys Ile Leu Leu Asp Ser Ala Gln Leu Lys Asp
        355                 360                 365

Leu Trp Lys Lys Ile Cys His His Thr Ser Gly Met Glu Phe Gln Asp
    370                 375                 380

His Arg Tyr Trp Leu Arg Thr His Pro Asn Cys Ile Val Gly Lys Glu
385                 390                 395                 400

Leu Val Asn Trp Leu Ile Arg Asn Gly His Ile Ala Thr Arg Ala Gln
                405                 410                 415

Ala Ile Ala Ile Gly Gln Ala Met Val Asp Gly Arg Trp Leu Asp Cys
            420                 425                 430

Val Ser His His Asp Gln Leu Phe Arg Asp Glu Tyr Ala Leu Tyr Arg
        435                 440                 445

Pro Leu Gln Ser Thr Glu Phe Ser Glu Thr Pro Ser Pro Asp Ser Asp
    450                 455                 460

Ser Val Asn Ser Val Glu Gly His Ser Glu Pro Ser Trp Phe Lys Asp
465                 470                 475                 480

Ile Lys Phe Asp Asp Ser Asp Thr Glu Gln Ile Ala Glu Glu Gly Asp
                485                 490                 495

Asp Asn Leu Ala Lys Tyr Leu Val Ser Asp Thr Gly Gly Gln Gln Leu
            500                 505                 510

Ser Ile Ser Asp Ala Phe Ile Lys Glu Ser Leu Phe Asn Arg Arg Val
        515                 520                 525

Glu Glu Lys Ser Lys Glu Leu Pro Phe Thr Pro Leu Gly Trp His His
    530                 535                 540

Asn Asn Leu Glu Leu Leu Arg Glu Glu Asn Glu Glu Lys Gln Ala Met
545                 550                 555                 560

Glu Arg Leu Leu Ser Ala Asn His Asn His Met Met Ala Leu Leu Gln
                565                 570                 575

Gln Leu Leu Gln Asn Glu Ser Leu Ser Ser Ser Trp Arg Asp Ile Ile
            580                 585                 590

Val Ser Leu Val Cys Gln Val Val Gln Thr Val Arg Pro Asp Val Lys
        595                 600                 605

His Gln Asp Asp Asp Met Asp Ile Arg Gln Phe Val His Ile Lys Lys
    610                 615                 620

Ile Pro Gly Gly Lys Lys Phe Asp Ser Val Val Val Asn Gly Phe Val
625                 630                 635                 640

Cys Thr Lys Asn Ile Ala His Lys Lys Met Asn Ser Cys Ile Lys Asn
                645                 650                 655

Pro Lys Ile Leu Leu Leu Lys Cys Ser Ile Glu Tyr Leu Tyr Arg Glu
            660                 665                 670

Glu Thr Lys Phe Thr Cys Ile Asp Pro Ile Val Leu Gln Glu Arg Glu
        675                 680                 685

Phe Leu Lys Asn Tyr Val Gln Arg Ile Val Asp Val Arg Pro Thr Leu
    690                 695                 700

Val Leu Val Glu Lys Thr Val Ser Arg Ile Ala Gln Asp Met Leu Leu
705                 710                 715                 720

Glu His Gly Ile Thr Leu Val Ile Asn Val Lys Ser Gln Val Leu Glu
                725                 730                 735

Arg Ile Ser Arg Met Thr Gln Gly Asp Leu Val Val Ser Met Asp Gln
            740                 745                 750

Leu Leu Thr Lys Pro His Leu Gly Thr Cys His Lys Phe Tyr Met Gln
        755                 760                 765

Ile Phe Gln Leu Pro Asn Glu Gln Thr Lys Thr Leu Met Phe Phe Glu
    770                 775                 780

Gly Cys Pro Gln His Leu Gly Cys Thr Ile Lys Leu Arg Gly Gly Ser
785                 790                 795                 800

Asp Tyr Glu Leu Ala Arg Val Lys Glu Ile Leu Ile Phe Met Ile Cys
                805                 810                 815

Val Ala Tyr His Ser Gln Leu Glu Ile Ser Phe Leu Met Asp Glu Phe
            820                 825                 830

Ala Met Pro Pro Thr Leu Met Gln Ser Pro Ser Phe His Leu Leu Thr
        835                 840                 845

Glu Gly Arg Gly Glu Glu Gly Ala Ser Gln Glu Gln Val Ser Gly Ser
    850                 855                 860

Ser Leu Pro Gln Asp Pro Glu Cys Pro Arg Glu Ala Leu Ser Ser Glu
865                 870                 875                 880

Asp Ser Thr Leu Leu Glu Ser Arg Thr Val Leu Glu Lys Gly Glu Leu
                885                 890                 895

Asp Asn Lys Ser Ile Pro Gln Ala Val Ala Ser Leu Lys His Gln Asp
            900                 905                 910

Tyr Thr Thr Pro Thr Cys Pro Ala Gly Ile Pro Cys Ala Leu Phe Ala
        915                 920                 925

Leu Val Pro Glu Ser Leu Leu Pro Leu His Met Asp Gln Gln Asp Ala
    930                 935                 940

Val Gly Asn Glu His Arg Glu Thr Ser Gln Gln Thr Asp Glu Gln Gln
945                 950                 955                 960

Asp Pro Lys Ser Gln Met Lys Ala Phe Arg Asp Pro Leu Gln Asp Asp
                965                 970                 975

Thr Gly Met Tyr Val Thr Glu Glu Val Thr Ser Ser Glu Asp Gln Arg
            980                 985                 990

Lys Thr Tyr Ala Leu Thr Phe Lys  Gln Glu Leu Lys Asp  Val Ile Leu
        995                 1000                 1005

Cys Ile  Ser Pro Val Ile Thr  Phe Arg Glu Pro Phe  Leu Leu Thr
    1010                 1015                 1020

Glu Lys  Gly Met Arg Cys Ser  Thr Arg Asp Tyr Phe  Pro Glu Gln
    1025                 1030                 1035

Ile Tyr  Trp Ser Pro Leu Leu  Asn Lys Glu Val Lys  Glu Met Glu
    1040                 1045                 1050

Ser Arg  Arg Lys Lys Gln Leu  Leu Arg Asp Leu Ser  Gly Leu Gln
    1055                 1060                 1065

Gly Met  Asn Gly Ser Val Gln  Ala Lys Ser Ile Gln  Val Leu Pro
    1070                 1075                 1080

Ser His  Glu Leu Val Ser Thr  Arg Ile Ala Glu His  Val Gly Asp
    1085                 1090                 1095

Ser Gln  Thr Leu Gly Arg Met  Leu Ala Asp Tyr Arg  Ala Arg Gly

1100                1105                1110

Gly Glu Phe Ser Gln Asn Ile Trp Asn Pro Phe Val His Ser Lys
    1115                1120                1125

Asp Asp Ile Met Tyr Phe Arg Trp Gln Ile Arg Glu Thr Lys Leu
    1130                1135                1140

Arg Val Met Lys Glu Arg Gly Leu Ile Pro Ser Asp Val Ile Trp
    1145                1150                1155

Pro Thr Lys Val Asp Cys Leu Asn Pro Ala Asn His Gln Arg Leu
    1160                1165                1170

Cys Val Leu Phe Ser Ser Ser Ser Ala Gln Ser Ser Asn Ala Pro
    1175                1180                1185

Ser Ala Cys Val Ser Pro Trp Ile Val Thr Met Glu Phe Tyr Gly
    1190                1195                1200

Lys Asn Asp Leu Thr Leu Gly Ile Phe Leu Glu Arg Tyr Cys Phe
    1205                1210                1215

Arg Tyr Ser Tyr Gln Cys Pro Ser Met Phe Cys Asp Thr Pro Met
    1220                1225                1230

Val His His Ile Arg Arg Phe Val His Gly Gln Gly Cys Val Gln
    1235                1240                1245

Ile Ile Leu Lys Glu Leu Asp Ser Pro Val Pro Gly Tyr Gln His
    1250                1255                1260

Thr Ile Leu Thr Tyr Ser Trp Cys Arg Ile Cys Lys Gln Val Thr
    1265                1270                1275

Pro Val Val Ala Leu Ser Asn Glu Ser Trp Ser Met Ser Phe Ala
    1280                1285                1290

Lys Tyr Leu Glu Leu Arg Phe Tyr Gly His Gln Tyr Thr Arg Arg
    1295                1300                1305

Ala Asn Ala Glu Pro Cys Gly His Ser Ile His His Asp Tyr His
    1310                1315                1320

Gln Tyr Phe Ser Tyr Asn Gln Met Val Ala Ser Phe Ser Tyr Ser
    1325                1330                1335

Pro Ile Arg Leu Leu Glu Val Cys Val Pro Leu Pro Lys Ile Phe
    1340                1345                1350

Ile Lys Arg Gln Ala Pro Leu Lys Val Ser Leu Leu Gln Asp Leu
    1355                1360                1365

Lys Asp Phe Phe Gln Lys Val Ser Gln Val Tyr Leu Ala Val Asp
    1370                1375                1380

Glu Arg Leu Ala Ser Leu Lys Thr Asp Thr Phe Ser Lys Thr Arg
    1385                1390                1395

Glu Glu Lys Met Glu Asp Ile Phe Ala Gln Lys Glu Met Glu Glu
    1400                1405                1410

Gly Glu Phe Lys Asn Trp Thr Glu Lys Met Gln Ala Arg Leu Met
    1415                1420                1425

Ser Ser Ser Val Asp Thr Pro Gln Gln Leu Gln Ser Ile Phe Glu
    1430                1435                1440

Ser Leu Ile Ala Lys Lys Gln Ser Leu Cys Glu Val Leu Gln Ala
    1445                1450                1455

Trp Asn Ser Arg Leu Gln Asp Leu Phe Gln Gln Glu Lys Gly Arg
    1460                1465                1470

Lys Arg Pro Ser Val Pro Pro Ser Pro Gly Arg Leu Arg Gln Gly
    1475                1480                1485

Glu Glu Ser Lys Ile Asn Ala Met Asp Thr Ser Pro Arg Asn Ile
    1490                1495                1500

Ser Pro Gly Leu Ser Gln Trp Arg Lys Arg Arg Ser Leu Leu Asp
    1505                1510                1515

Asn Pro Val Gln Pro Ala Thr Ser Ser Thr His Leu Gln Leu Pro
    1520                1525                1530

Thr Pro Pro Glu Ala Leu Ala Glu Gln Val Val Gly Gly Pro Thr
    1535                1540                1545

Asp Leu Asp Ser Ala Ser Gly Ser Glu Asp Val Phe Asp Gly His
    1550                1555                1560

Leu Leu Gly Ser Thr Asp Ser Gln Val Lys Glu Lys Ser Thr Met
    1565                1570                1575

Lys Ala Ile Phe Ala Asn Leu Leu Pro Gly Asn Ser Tyr Asn Pro
    1580                1585                1590

Ile Pro Phe Pro Phe Asp Pro Asp Lys His Tyr Leu Met Tyr Glu
    1595                1600                1605

His Glu Arg Val Pro Ile Ala Val Cys Glu Lys Glu Pro Ser Ser
    1610                1615                1620

Ile Ile Ala Phe Ala Leu Ser Cys Lys Glu Tyr Arg Asn Ala Leu
    1625                1630                1635

Glu Glu Leu Ser Lys Ala Thr Leu Arg Asn Ser Ala Glu Glu Gly
    1640                1645                1650

Leu Pro Ala Asn Ser Ala Leu Asp Asn Arg Pro Lys Ser Ser Ser
    1655                1660                1665

Pro Ile Arg Leu Pro Glu Ile Ser Gly Gly Gln Thr Asn Arg Thr
    1670                1675                1680

Val Glu Ala Glu Pro Gln Pro Thr Lys Lys Ala Ser Gly Met Leu
    1685                1690                1695

Ser Phe Phe Arg Gly Thr Ala Gly Lys Ser Pro Asp Leu Ser Ser
    1700                1705                1710

Gln Lys Arg Glu Thr Leu Arg Gly Ala Asp Ser Ala Tyr Tyr Gln
    1715                1720                1725

Val Gly Gln Ala Gly Lys Glu Gly Leu Glu Ser Gln Gly Leu Glu
    1730                1735                1740

Pro Gln Asp Glu Val Asp Gly Gly Asp Thr Gln Lys Lys Gln Leu
    1745                1750                1755

Thr Asn Pro His Val Glu Leu Gln Phe Ser Asp Ala Asn Ala Lys
    1760                1765                1770

Phe Tyr Cys Arg Leu Tyr Tyr Ala Gly Glu Phe His Lys Met Arg
    1775                1780                1785

Glu Val Ile Leu Gly Ser Ser Glu Glu Glu Phe Ile Arg Ser Leu
    1790                1795                1800

Ser His Ser Ser Pro Trp Gln Ala Arg Gly Gly Lys Ser Gly Ala
    1805                1810                1815

Ala Phe Tyr Ala Thr Glu Asp Asp Arg Phe Ile Leu Lys Gln Met
    1820                1825                1830

Pro Arg Leu Glu Val Gln Ser Phe Leu Asp Phe Ala Pro His Tyr
    1835                1840                1845

Phe Asn Tyr Ile Thr Asn Ala Val Gln Gln Lys Arg Pro Thr Ala
    1850                1855                1860

Leu Ala Lys Ile Leu Gly Val Tyr Arg Ile Gly Tyr Lys Asn Ser
    1865                1870                1875

Gln Asn Asn Thr Glu Lys Lys Leu Asp Leu Leu Val Met Glu Asn
    1880                1885                1890

Leu Phe Tyr Gly Arg Lys Met Ala Gln Val Phe Asp Leu Lys Gly
    1895                1900                1905

Ser Leu Arg Asn Arg Asn Val Lys Thr Asp Thr Gly Lys Glu Ser
    1910                1915                1920

Cys Asp Val Val Leu Leu Asp Glu Asn Leu Leu Lys Met Val Arg
    1925                1930                1935

Asp Asn Pro Leu Tyr Ile Arg Ser His Ser Lys Ser Glu Leu Arg
    1940                1945                1950

Thr Ser Ile His Ser Asp Ala His Phe Leu Ser Ser His Leu Ile
    1955                1960                1965

Ile Asp Tyr Ser Leu Leu Val Gly Arg Asp Asp Thr Ser Asn Glu
    1970                1975                1980

Leu Val Val Gly Ile Ile Asp Tyr Ile Arg Thr Phe Thr Trp Asp
    1985                1990                1995

Lys Lys Leu Glu Met Val Val Lys Ser Thr Gly Ile Leu Gly Gly
    2000                2005                2010

Gln Gly Lys Met Pro Thr Val Val Ser Pro Glu Leu Tyr Arg Thr
    2015                2020                2025

Arg Phe Cys Glu Ala Met Asp Lys Tyr Phe Leu Met Val Pro Asp
    2030                2035                2040

His Trp Thr Gly Leu Asp Leu Asn Cys
    2045                2050

<210> 5
<211> 2349
<212> DNA
<213> human Vac14

<220>
<221>  CDS
<222>  (1)..(2349)

<400>  5
atg aac ccc gag aag gat ttc gcg ccg ctc acg cct aac atc gtg cgc      48
Met Asn Pro Glu Lys Asp Phe Ala Pro Leu Thr Pro Asn Ile Val Arg
1               5                   10                  15

gcc ctc aat gac aag ctg tac gaa aag cgg aag gtg gca gcg ctg gag      96
Ala Leu Asn Asp Lys Leu Tyr Glu Lys Arg Lys Val Ala Ala Leu Glu
            20                  25                  30

atc gag aag ctg gtc cgg gag ttc gtg gcc cag aac aat acc gtg caa      144
Ile Glu Lys Leu Val Arg Glu Phe Val Ala Gln Asn Asn Thr Val Gln
        35                  40                  45

atc aag cat gtg atc cag acc ctg tcc cag gag ttt gcc ctg tct cag      192
Ile Lys His Val Ile Gln Thr Leu Ser Gln Glu Phe Ala Leu Ser Gln
    50                  55                  60

cac ccc cac agc cgg aaa ggg ggc ctc atc ggc ctg gcc gcc tgc tcc      240
His Pro His Ser Arg Lys Gly Gly Leu Ile Gly Leu Ala Ala Cys Ser
65                  70                  75                  80

atc gca ctg ggc aag gac tca ggg ctc tac ctg aag gag ctg atc gag      288
Ile Ala Leu Gly Lys Asp Ser Gly Leu Tyr Leu Lys Glu Leu Ile Glu
                85                  90                  95

cca gtg ctg acc tgc ttc aat gat gca gac agc agg ctg cgc tac tat      336
Pro Val Leu Thr Cys Phe Asn Asp Ala Asp Ser Arg Leu Arg Tyr Tyr
            100                 105                 110

gcc tgc gag gcc ctc tac aac atc gtc aag gtg gcc cgg ggc gct gtg      384
Ala Cys Glu Ala Leu Tyr Asn Ile Val Lys Val Ala Arg Gly Ala Val
        115                 120                 125

ctg ccc cac ttc aac gtg ctc ttt gac ggg ctg agc aag ctg gca gcc      432
Leu Pro His Phe Asn Val Leu Phe Asp Gly Leu Ser Lys Leu Ala Ala
    130                 135                 140

gac cca gac ccc aat gtg aaa agc gga tct gag ctc cta gac cgc ctt      480
Asp Pro Asp Pro Asn Val Lys Ser Gly Ser Glu Leu Leu Asp Arg Leu
145                 150                 155                 160

tta aag gac att gtg act gag agc aac aag ttt gac ctg gtg agc ttc      528
Leu Lys Asp Ile Val Thr Glu Ser Asn Lys Phe Asp Leu Val Ser Phe
                165                 170                 175

atc ccc ttg ttg cga gag agg att tac tcc aac aac cag tat gcc cgg      576
Ile Pro Leu Leu Arg Glu Arg Ile Tyr Ser Asn Asn Gln Tyr Ala Arg
            180                 185                 190

cag ttc atc atc tcc tgg atc ctg gtt ctg gag tcg gtg cca gac att      624
Gln Phe Ile Ile Ser Trp Ile Leu Val Leu Glu Ser Val Pro Asp Ile
        195                 200                 205

aac ctg ctg gat tac ctg ccg gag atc ctg gat gga ctc ttc cag atc      672
Asn Leu Leu Asp Tyr Leu Pro Glu Ile Leu Asp Gly Leu Phe Gln Ile
    210                 215                 220

ctg ggt gac aat ggc aaa gag att cgc aaa atg tgt gag gtt gtt ctt      720
Leu Gly Asp Asn Gly Lys Glu Ile Arg Lys Met Cys Glu Val Val Leu
225                 230                 235                 240

gga gaa ttc tta aaa gaa att aag aag aac ccc tcc agt gtg aag ttt     768
Gly Glu Phe Leu Lys Glu Ile Lys Lys Asn Pro Ser Ser Val Lys Phe
            245                 250                 255

gct gag atg gcc aac atc ctg gtg atc cac tgc cag aca aca gat gac     816
Ala Glu Met Ala Asn Ile Leu Val Ile His Cys Gln Thr Thr Asp Asp
        260                 265                 270

ctc atc cag ctg aca gcc atg tgc tgg atg cgg gag ttc atc cag ctg     864
Leu Ile Gln Leu Thr Ala Met Cys Trp Met Arg Glu Phe Ile Gln Leu
    275                 280                 285

gcg ggc cgc gtc atg ctg cct tac tcc tcc ggg atc ctg act gct gtc     912
Ala Gly Arg Val Met Leu Pro Tyr Ser Ser Gly Ile Leu Thr Ala Val
    290                 295                 300

ttg ccc tgc ttg gcc tac gat gac cgc aag aaa agc atc aaa gaa gtg     960
Leu Pro Cys Leu Ala Tyr Asp Asp Arg Lys Lys Ser Ile Lys Glu Val
305                 310                 315                 320

gcc aac gtg tgc aac cag agc ctg atg aag ctg gtc acc ccc gag gac     1008
Ala Asn Val Cys Asn Gln Ser Leu Met Lys Leu Val Thr Pro Glu Asp
            325                 330                 335

gac gag ctg gat gag ctg aga cct ggg cag agg cag gca gag ccc acc     1056
Asp Glu Leu Asp Glu Leu Arg Pro Gly Gln Arg Gln Ala Glu Pro Thr
        340                 345                 350

cct gac gat gcc ctg cca aag cag gag ggc aca gcc agt gga ggt cca     1104
Pro Asp Asp Ala Leu Pro Lys Gln Glu Gly Thr Ala Ser Gly Gly Pro
    355                 360                 365

gat ggt tcc tgt gac tcc agc ttc agt agc ggc atc agt gtc ttc act     1152
Asp Gly Ser Cys Asp Ser Ser Phe Ser Ser Gly Ile Ser Val Phe Thr
    370                 375                 380

gca gcc agc act gaa aga gcc cca gtg acc ctt cac ctc gac ggg atc     1200
Ala Ala Ser Thr Glu Arg Ala Pro Val Thr Leu His Leu Asp Gly Ile
385                 390                 395                 400

gtg cag gtc cta aac tgc cac ctc agt gac acg gcc att ggg atg atg     1248
Val Gln Val Leu Asn Cys His Leu Ser Asp Thr Ala Ile Gly Met Met
            405                 410                 415

acc agg att gca gtt ctc aag tgg ctc tac cac ctc tac atc aaa act     1296
Thr Arg Ile Ala Val Leu Lys Trp Leu Tyr His Leu Tyr Ile Lys Thr
        420                 425                 430

cct cgg aag atg ttc cgg cac acg gac agc ctc ttt ccc atc cta ctg     1344
Pro Arg Lys Met Phe Arg His Thr Asp Ser Leu Phe Pro Ile Leu Leu
    435                 440                 445

cag acg tta tcg gat gaa tcg gat gag gtg atc ctg aag gac ctg gag     1392
Gln Thr Leu Ser Asp Glu Ser Asp Glu Val Ile Leu Lys Asp Leu Glu
    450                 455                 460

gtg ctg gca gaa atc gct tcc tcc ccc gca ggc cag acg gat gac cca     1440
Val Leu Ala Glu Ile Ala Ser Ser Pro Ala Gly Gln Thr Asp Asp Pro
465                 470                 475                 480

ggc ccc ctc gat ggc cct gac ctc cag gcc agc cac tca gag ctc cag     1488
Gly Pro Leu Asp Gly Pro Asp Leu Gln Ala Ser His Ser Glu Leu Gln
            485                 490                 495

gtg ccc acc cct ggc aga gcc ggc cta ctg aac acc tct ggt acc aaa     1536
Val Pro Thr Pro Gly Arg Ala Gly Leu Leu Asn Thr Ser Gly Thr Lys
        500                 505                 510

ggc tta gaa tgt tct cct tca act ccc acc atg aat tct tac ttt tat     1584
Gly Leu Glu Cys Ser Pro Ser Thr Pro Thr Met Asn Ser Tyr Phe Tyr
        515                 520                 525

aag ttc atg atc aac ctt ctc aag aga ttc agc agc gaa cgg aag ctc     1632
Lys Phe Met Ile Asn Leu Leu Lys Arg Phe Ser Ser Glu Arg Lys Leu
    530                 535                 540

ctg gag gtc aga ggc cct ttc atc atc agg cag ctg tgc ctc ctg ctg     1680
Leu Glu Val Arg Gly Pro Phe Ile Ile Arg Gln Leu Cys Leu Leu Leu
545                 550                 555                 560

aat gcg gag aac atc ttc cac tca atg gca gac atc ctg ctg cgg gag     1728
Asn Ala Glu Asn Ile Phe His Ser Met Ala Asp Ile Leu Leu Arg Glu
                565                 570                 575

gag gac ctc aag ttc gcc tcg acc atg gtc cac gcc ctc aac acc atc     1776
Glu Asp Leu Lys Phe Ala Ser Thr Met Val His Ala Leu Asn Thr Ile
            580                 585                 590

ctg ctg acc tcc aca gag ctc ttc cag cta agg aac cag ctg aag gac     1824
Leu Leu Thr Ser Thr Glu Leu Phe Gln Leu Arg Asn Gln Leu Lys Asp
        595                 600                 605

ctg aag acc ctg gag agc cag aac ctg ttc tgc tgc ctg tac cgc tcc     1872
Leu Lys Thr Leu Glu Ser Gln Asn Leu Phe Cys Cys Leu Tyr Arg Ser
    610                 615                 620

tgg tgc cac aac cca gtc acc acg gtg tcc ctc tgc ttc ctc acc cag     1920
Trp Cys His Asn Pro Val Thr Thr Val Ser Leu Cys Phe Leu Thr Gln
625                 630                 635                 640

aac tac cgg cac gcc tat gac ctc atc cag aag ttt ggg gac ctg gag     1968
Asn Tyr Arg His Ala Tyr Asp Leu Ile Gln Lys Phe Gly Asp Leu Glu
                645                 650                 655

gtc acc gtg gac ttc ctc gca gag gtg gac aag ctg gtg cag ctg att     2016
Val Thr Val Asp Phe Leu Ala Glu Val Asp Lys Leu Val Gln Leu Ile
            660                 665                 670

gag tgc ccc atc ttc aca tat ctg cgc ctg cag ctg ctg gac gtg aag     2064
Glu Cys Pro Ile Phe Thr Tyr Leu Arg Leu Gln Leu Leu Asp Val Lys
        675                 680                 685

aac aac ccc tac ctg atc aag gcc ctc tac ggc ctg ctc atg ctc ctg     2112
Asn Asn Pro Tyr Leu Ile Lys Ala Leu Tyr Gly Leu Leu Met Leu Leu
    690                 695                 700

ccg cag agc agc gcc ttc cag ctg ctc tcg cac cgg ctc cag tgc gtg     2160
Pro Gln Ser Ser Ala Phe Gln Leu Leu Ser His Arg Leu Gln Cys Val
705                 710                 715                 720

ccc aac cct gag ctg ctg cag acc gaa gac agt cta aag gca gcc ccc     2208
Pro Asn Pro Glu Leu Leu Gln Thr Glu Asp Ser Leu Lys Ala Ala Pro
                725                 730                 735

aag tcc cag aaa gct gac tcc cct agc atc gac tac gca gag ctg ctg     2256
Lys Ser Gln Lys Ala Asp Ser Pro Ser Ile Asp Tyr Ala Glu Leu Leu
            740                 745                 750

cag cac ttt gag aag gtc cag aac aag cac ctg gaa gtg cgg cac cag     2304

Gln His Phe Glu Lys Val Gln Asn Lys His Leu Glu Val Arg His Gln
        755                 760                 765

cgg agc ggg cgt ggg gac cac ctg gac cgg agg gtt gtc ctc tga          2349
Arg Ser Gly Arg Gly Asp His Leu Asp Arg Arg Val Val Leu
    770                 775                 780

<210>  6
<211>  782
<212>  PRT
<213>  human Vac14

<400>  6

Met Asn Pro Glu Lys Asp Phe Ala Pro Leu Thr Pro Asn Ile Val Arg
1               5                   10                  15

Ala Leu Asn Asp Lys Leu Tyr Glu Lys Arg Lys Val Ala Ala Leu Glu
            20                  25                  30

Ile Glu Lys Leu Val Arg Glu Phe Val Ala Gln Asn Asn Thr Val Gln
        35                  40                  45

Ile Lys His Val Ile Gln Thr Leu Ser Gln Glu Phe Ala Leu Ser Gln
    50                  55                  60

His Pro His Ser Arg Lys Gly Gly Leu Ile Gly Leu Ala Ala Cys Ser
65                  70                  75                  80

Ile Ala Leu Gly Lys Asp Ser Gly Leu Tyr Leu Lys Glu Leu Ile Glu
                85                  90                  95

Pro Val Leu Thr Cys Phe Asn Asp Ala Asp Ser Arg Leu Arg Tyr Tyr
            100                 105                 110

Ala Cys Glu Ala Leu Tyr Asn Ile Val Lys Val Ala Arg Gly Ala Val
        115                 120                 125

Leu Pro His Phe Asn Val Leu Phe Asp Gly Leu Ser Lys Leu Ala Ala
    130                 135                 140

Asp Pro Asp Pro Asn Val Lys Ser Gly Ser Glu Leu Leu Asp Arg Leu
145                 150                 155                 160

Leu Lys Asp Ile Val Thr Glu Ser Asn Lys Phe Asp Leu Val Ser Phe
                165                 170                 175

Ile Pro Leu Leu Arg Glu Arg Ile Tyr Ser Asn Asn Gln Tyr Ala Arg
            180                 185                 190

Gln Phe Ile Ile Ser Trp Ile Leu Val Leu Glu Ser Val Pro Asp Ile
        195                 200                 205

Asn Leu Leu Asp Tyr Leu Pro Glu Ile Leu Asp Gly Leu Phe Gln Ile
    210                 215                 220

Leu Gly Asp Asn Gly Lys Glu Ile Arg Lys Met Cys Glu Val Val Leu
225                 230                 235                 240

Gly Glu Phe Leu Lys Glu Ile Lys Lys Asn Pro Ser Ser Val Lys Phe
                245                 250                 255

Ala Glu Met Ala Asn Ile Leu Val Ile His Cys Gln Thr Thr Asp Asp
            260                 265                 270

Leu Ile Gln Leu Thr Ala Met Cys Trp Met Arg Glu Phe Ile Gln Leu
        275                 280                 285

Ala Gly Arg Val Met Leu Pro Tyr Ser Ser Gly Ile Leu Thr Ala Val
    290                 295                 300

Leu Pro Cys Leu Ala Tyr Asp Asp Arg Lys Lys Ser Ile Lys Glu Val
305                 310                 315                 320

Ala Asn Val Cys Asn Gln Ser Leu Met Lys Leu Val Thr Pro Glu Asp
                325                 330                 335

Asp Glu Leu Asp Glu Leu Arg Pro Gly Gln Arg Gln Ala Glu Pro Thr
            340                 345                 350

Pro Asp Asp Ala Leu Pro Lys Gln Glu Gly Thr Ala Ser Gly Gly Pro
        355                 360                 365

Asp Gly Ser Cys Asp Ser Ser Phe Ser Ser Gly Ile Ser Val Phe Thr
    370                 375                 380

Ala Ala Ser Thr Glu Arg Ala Pro Val Thr Leu His Leu Asp Gly Ile
385                 390                 395                 400

Val Gln Val Leu Asn Cys His Leu Ser Asp Thr Ala Ile Gly Met Met
                405                 410                 415

Thr Arg Ile Ala Val Leu Lys Trp Leu Tyr His Leu Tyr Ile Lys Thr
            420                 425                 430

Pro Arg Lys Met Phe Arg His Thr Asp Ser Leu Phe Pro Ile Leu Leu
        435                 440                 445

Gln Thr Leu Ser Asp Glu Ser Asp Glu Val Ile Leu Lys Asp Leu Glu
    450                 455                 460

Val Leu Ala Glu Ile Ala Ser Ser Pro Ala Gly Gln Thr Asp Asp Pro
465                 470                 475                 480

Gly Pro Leu Asp Gly Pro Asp Leu Gln Ala Ser His Ser Glu Leu Gln
                485                 490                 495

Val Pro Thr Pro Gly Arg Ala Gly Leu Leu Asn Thr Ser Gly Thr Lys
            500                 505                 510

Gly Leu Glu Cys Ser Pro Ser Thr Pro Thr Met Asn Ser Tyr Phe Tyr
        515                 520                 525

Lys Phe Met Ile Asn Leu Leu Lys Arg Phe Ser Ser Glu Arg Lys Leu
    530                 535                 540

Leu Glu Val Arg Gly Pro Phe Ile Ile Arg Gln Leu Cys Leu Leu Leu
545                 550                 555                 560

Asn Ala Glu Asn Ile Phe His Ser Met Ala Asp Ile Leu Leu Arg Glu
                565                 570                 575

Glu Asp Leu Lys Phe Ala Ser Thr Met Val His Ala Leu Asn Thr Ile
            580                 585                 590

Leu Leu Thr Ser Thr Glu Leu Phe Gln Leu Arg Asn Gln Leu Lys Asp
        595                 600                 605

Leu Lys Thr Leu Glu Ser Gln Asn Leu Phe Cys Cys Leu Tyr Arg Ser
    610                 615                 620

Trp Cys His Asn Pro Val Thr Thr Val Ser Leu Cys Phe Leu Thr Gln
625                 630                 635                 640

Asn Tyr Arg His Ala Tyr Asp Leu Ile Gln Lys Phe Gly Asp Leu Glu
                645                 650                 655

Val Thr Val Asp Phe Leu Ala Glu Val Asp Lys Leu Val Gln Leu Ile
            660                 665                 670

Glu Cys Pro Ile Phe Thr Tyr Leu Arg Leu Gln Leu Leu Asp Val Lys
        675                 680                 685

Asn Asn Pro Tyr Leu Ile Lys Ala Leu Tyr Gly Leu Leu Met Leu Leu
    690                 695                 700

Pro Gln Ser Ser Ala Phe Gln Leu Leu Ser His Arg Leu Gln Cys Val
705                 710                 715                 720

Pro Asn Pro Glu Leu Leu Gln Thr Glu Asp Ser Leu Lys Ala Ala Pro
                725                 730                 735

Lys Ser Gln Lys Ala Asp Ser Pro Ser Ile Asp Tyr Ala Glu Leu Leu
            740                 745                 750

Gln His Phe Glu Lys Val Gln Asn Lys His Leu Glu Val Arg His Gln
        755                 760                 765

Arg Ser Gly Arg Gly Asp His Leu Asp Arg Arg Val Val Leu
    770                 775                 780

<210>  7
<211>  2349
<212>  DNA
<213>  mouse Vac14

<220>
<221>  CDS
<222>  (1)..(2349)

<400>  7
atg aac cca gag aag gat ttt gcg ccg ctc act ccc aac atc gta agg      48
Met Asn Pro Glu Lys Asp Phe Ala Pro Leu Thr Pro Asn Ile Val Arg
1               5                   10                  15

gcc ctg aat gat aaa ctc tac gaa aag cgg aag gtt gca gcg ctg gag      96
Ala Leu Asn Asp Lys Leu Tyr Glu Lys Arg Lys Val Ala Ala Leu Glu
            20                  25                  30

atc gag aag ctg gtg cgg gac ttt gtg gcc cag aac aac acc atg caa      144
Ile Glu Lys Leu Val Arg Asp Phe Val Ala Gln Asn Asn Thr Met Gln
        35                  40                  45

atc aag cac gtg atc cag acc ctg tct cag gag ttt gcc ctg tcc cag      192
Ile Lys His Val Ile Gln Thr Leu Ser Gln Glu Phe Ala Leu Ser Gln
    50                  55                  60

cac ccc cac agc cgg aaa ggg ggc ctc atc ggt ctg gca gcc tgc tct      240
His Pro His Ser Arg Lys Gly Gly Leu Ile Gly Leu Ala Ala Cys Ser
65                  70                  75                  80

att gcc cta ggc aag gac tca ggg ctc tac ctg aag gag ctg ata gag      288
Ile Ala Leu Gly Lys Asp Ser Gly Leu Tyr Leu Lys Glu Leu Ile Glu
                85                  90                  95

cca gtg ctg acc tgc ttt aat gac gca gac agc agg ctg cgc tac tac      336
Pro Val Leu Thr Cys Phe Asn Asp Ala Asp Ser Arg Leu Arg Tyr Tyr
            100                 105                 110

gct tgt gag gcc ctc tac aat atc gtc aaa gtg gcc cga ggt gcc gtg      384
Ala Cys Glu Ala Leu Tyr Asn Ile Val Lys Val Ala Arg Gly Ala Val
        115                 120                 125

ctg ccg cac ttc aat gtt ctc ttc gat gga ctg agt aag ttg gct gct      432
Leu Pro His Phe Asn Val Leu Phe Asp Gly Leu Ser Lys Leu Ala Ala
    130                 135                 140

gac cca gac ccc aat gtg aaa agt gga tct gag ctc ctg gac cgc ctc      480
Asp Pro Asp Pro Asn Val Lys Ser Gly Ser Glu Leu Leu Asp Arg Leu

145                 150                 155                 160

tta aag gac att gtg aca gaa agc agc aag ttt gac ctg gtg agc ttc      528
Leu Lys Asp Ile Val Thr Glu Ser Ser Lys Phe Asp Leu Val Ser Phe
                165                 170                 175

atc ccc ctt ttg cgg gag agg att tac tcc aac aac caa tac gcc cga      576
Ile Pro Leu Leu Arg Glu Arg Ile Tyr Ser Asn Asn Gln Tyr Ala Arg
            180                 185                 190

cag ttc atc atc tcc tgg atc ctg gtt ctg gtg tcg gtg cca gac att      624
Gln Phe Ile Ile Ser Trp Ile Leu Val Leu Val Ser Val Pro Asp Ile
        195                 200                 205

aac ctg ctg gat tac ctg cca gag atc cta gat ggg ctc ttc cag atc      672
Asn Leu Leu Asp Tyr Leu Pro Glu Ile Leu Asp Gly Leu Phe Gln Ile
    210                 215                 220

ctg ggt gac aat ggc aag gag att cgg aaa atg tgt gag gtg gtt ctt      720
Leu Gly Asp Asn Gly Lys Glu Ile Arg Lys Met Cys Glu Val Val Leu
225                 230                 235                 240

gga gag ttc ttg aaa gaa att aag aag aac cca tcc agt gtg aag ttt      768
Gly Glu Phe Leu Lys Glu Ile Lys Lys Asn Pro Ser Ser Val Lys Phe
                245                 250                 255

gct gag atg gcc aac atc ctg gtg atc cac tgc cag acc aca gat gac      816
Ala Glu Met Ala Asn Ile Leu Val Ile His Cys Gln Thr Thr Asp Asp
            260                 265                 270

ctg att cag ctg acg gct atg tgc tgg atg cgg gaa ttc atc cag ctg      864
Leu Ile Gln Leu Thr Ala Met Cys Trp Met Arg Glu Phe Ile Gln Leu
        275                 280                 285

gcg ggc cgg gta atg ctg ccc tat tcc tct ggg atc ctg act gct gtc      912
Ala Gly Arg Val Met Leu Pro Tyr Ser Ser Gly Ile Leu Thr Ala Val
    290                 295                 300

ctg cca tgc ttg gcc tat gat gac cgc aag aaa agt atc aag gag gtg      960
Leu Pro Cys Leu Ala Tyr Asp Asp Arg Lys Lys Ser Ile Lys Glu Val
305                 310                 315                 320

gcc aat gtg tgt aac cag agc ttg atg aag ctg gtc acc cct gag gat     1008
Ala Asn Val Cys Asn Gln Ser Leu Met Lys Leu Val Thr Pro Glu Asp
                325                 330                 335

gat gaa ccc gat gag cca aag tca gta gca cag aag cag acc gaa ccc     1056
Asp Glu Pro Asp Glu Pro Lys Ser Val Ala Gln Lys Gln Thr Glu Pro
            340                 345                 350

aac cct gag gat tcc ctg cca aag cag gag ggg aca gcc agt gga ggt     1104
Asn Pro Glu Asp Ser Leu Pro Lys Gln Glu Gly Thr Ala Ser Gly Gly
        355                 360                 365

cca ggt tcc tgt gac tcc agc ttt gga agt ggc atc aat gtc ttc acc     1152
Pro Gly Ser Cys Asp Ser Ser Phe Gly Ser Gly Ile Asn Val Phe Thr
    370                 375                 380

tca gcc aac act gat agg gct cca gtg acc ctg cac ctg gac ggg att     1200
Ser Ala Asn Thr Asp Arg Ala Pro Val Thr Leu His Leu Asp Gly Ile
385                 390                 395                 400

gtt caa gtc ctg aac tgt cac ctt agt gac aca acc att ggg atg atg     1248
Val Gln Val Leu Asn Cys His Leu Ser Asp Thr Thr Ile Gly Met Met
                405                 410                 415

acc agg att gct gtt ctg aag tgg ctc tac cat ctc tac atc aaa act     1296
Thr Arg Ile Ala Val Leu Lys Trp Leu Tyr His Leu Tyr Ile Lys Thr
            420                 425                 430

cct cga aag atg ttc cgg cac aca gac agc ctc ttc ccc atc tta ctt     1344
Pro Arg Lys Met Phe Arg His Thr Asp Ser Leu Phe Pro Ile Leu Leu
        435                 440                 445

cag aca tta tct gac gaa tct gat gag gtt gtc ttg aag gat ctg gaa     1392
Gln Thr Leu Ser Asp Glu Ser Asp Glu Val Val Leu Lys Asp Leu Glu
    450                 455                 460

gtg ttg gca gaa att gct tcc tcc cct gca ggc cag acg gat gac cca     1440
Val Leu Ala Glu Ile Ala Ser Ser Pro Ala Gly Gln Thr Asp Asp Pro
465                 470                 475                 480

ggt gca cct gat ggc cct gac ctc cga gtc aac cac tcg gag ctt cag     1488
Gly Ala Pro Asp Gly Pro Asp Leu Arg Val Asn His Ser Glu Leu Gln
                485                 490                 495

gtg ccc acc tcc ggc aga gcc aac cta cta aac cct ccc agt acc aaa     1536
Val Pro Thr Ser Gly Arg Ala Asn Leu Leu Asn Pro Pro Ser Thr Lys
            500                 505                 510

ggc tta gaa ggc tcc ccc tcc act ccc acc atg aac tcc tac ttt tac     1584
Gly Leu Glu Gly Ser Pro Ser Thr Pro Thr Met Asn Ser Tyr Phe Tyr
        515                 520                 525

aag ttc atg atc aac ctt cta cag acg ttc agc agt gaa cgg aag ctc     1632
Lys Phe Met Ile Asn Leu Leu Gln Thr Phe Ser Ser Glu Arg Lys Leu
    530                 535                 540

ctg gag gcc aga ggc ccg ttc atc atc agg cag ctc tgc ctc ctg ctg     1680
Leu Glu Ala Arg Gly Pro Phe Ile Ile Arg Gln Leu Cys Leu Leu Leu
545                 550                 555                 560

aat gca gag aac atc ttc cat tca atg gca gac atc ctg ctt cgg gag     1728
Asn Ala Glu Asn Ile Phe His Ser Met Ala Asp Ile Leu Leu Arg Glu
                565                 570                 575

gag gac ctc aag ttc gcg tcc acc atg gtg cac acc ctc aac acc atc     1776
Glu Asp Leu Lys Phe Ala Ser Thr Met Val His Thr Leu Asn Thr Ile
            580                 585                 590

ctg ctc acc tcc acc gag ctc ttc cag ctg agg aac cag ctc aag gac     1824
Leu Leu Thr Ser Thr Glu Leu Phe Gln Leu Arg Asn Gln Leu Lys Asp
        595                 600                 605

ctg cag acc ccg gaa agc cag aac ctc ttc tgc tgc ctg tac cgc tcc     1872
Leu Gln Thr Pro Glu Ser Gln Asn Leu Phe Cys Cys Leu Tyr Arg Ser
    610                 615                 620

tgg tgc cac aac cca gtc acc acc gtg tcc ctc tgc ttc ctc acc cag     1920
Trp Cys His Asn Pro Val Thr Thr Val Ser Leu Cys Phe Leu Thr Gln
625                 630                 635                 640

aac tac cgc cac gcc tat gac ctc atc cag aag ttt ggg gac ctg gaa     1968
Asn Tyr Arg His Ala Tyr Asp Leu Ile Gln Lys Phe Gly Asp Leu Glu
                645                 650                 655

gtc aca gtg gat ttc ctc aca gag gtg gac aaa ctc gtg cag cta att     2016
Val Thr Val Asp Phe Leu Thr Glu Val Asp Lys Leu Val Gln Leu Ile
            660                 665                 670

gag tgc ccc atc ttt aca tat cta cgc ctg caa ctg ctg gac gtg aag 2064
Glu Cys Pro Ile Phe Thr Tyr Leu Arg Leu Gln Leu Leu Asp Val Lys
        675                 680                 685

aac aac cca tac ctg atc aag gcc ctg tac ggc ctg ctc atg ctg ttg 2112
Asn Asn Pro Tyr Leu Ile Lys Ala Leu Tyr Gly Leu Leu Met Leu Leu
    690                 695                 700

ccc cag agc agt gcc ttc cag ctg ctc tct cac cgg ctc cag tgt gtg 2160
Pro Gln Ser Ser Ala Phe Gln Leu Leu Ser His Arg Leu Gln Cys Val
705                 710                 715                 720

ccc aac cct gag ctg ctg cag act gaa gac tgt ctg aaa gcg gcc ccc 2208
Pro Asn Pro Glu Leu Leu Gln Thr Glu Asp Cys Leu Lys Ala Ala Pro
                725                 730                 735

aag tcc cag aaa ggt gac tcc ccc agc atc gac tac aca gag ctg ctg 2256
Lys Ser Gln Lys Gly Asp Ser Pro Ser Ile Asp Tyr Thr Glu Leu Leu
            740                 745                 750

cag cac ttt gaa aaa gtc cag aaa cag cac ctg gaa gta aga cac cag 2304
Gln His Phe Glu Lys Val Gln Lys Gln His Leu Glu Val Arg His Gln
        755                 760                 765

cgc agt ggg cga ggg gat cac ctg gac cgc aga gtt atc ctc tga 2349
Arg Ser Gly Arg Gly Asp His Leu Asp Arg Arg Val Ile Leu
    770                 775                 780

<210> 8
<211> 782
<212> PRT
<213> mouse Vac14

<400> 8

Met Asn Pro Glu Lys Asp Phe Ala Pro Leu Thr Pro Asn Ile Val Arg
1               5                   10                  15

Ala Leu Asn Asp Lys Leu Tyr Glu Lys Arg Lys Val Ala Ala Leu Glu
            20                  25                  30

Ile Glu Lys Leu Val Arg Asp Phe Val Ala Gln Asn Asn Thr Met Gln
        35                  40                  45

Ile Lys His Val Ile Gln Thr Leu Ser Gln Glu Phe Ala Leu Ser Gln
    50                  55                  60

His Pro His Ser Arg Lys Gly Gly Leu Ile Gly Leu Ala Ala Cys Ser
65                  70                  75                  80

Ile Ala Leu Gly Lys Asp Ser Gly Leu Tyr Leu Lys Glu Leu Ile Glu
                85                  90                  95

Pro Val Leu Thr Cys Phe Asn Asp Ala Asp Ser Arg Leu Arg Tyr Tyr
            100                 105                 110

Ala Cys Glu Ala Leu Tyr Asn Ile Val Lys Val Ala Arg Gly Ala Val

        115                 120                 125

Leu Pro His Phe Asn Val Leu Phe Asp Gly Leu Ser Lys Leu Ala Ala
    130                 135                 140

Asp Pro Asp Pro Asn Val Lys Ser Gly Ser Glu Leu Leu Asp Arg Leu
145                 150                 155                 160

Leu Lys Asp Ile Val Thr Glu Ser Ser Lys Phe Asp Leu Val Ser Phe
                165                 170                 175

Ile Pro Leu Leu Arg Glu Arg Ile Tyr Ser Asn Asn Gln Tyr Ala Arg
            180                 185                 190

Gln Phe Ile Ile Ser Trp Ile Leu Val Leu Val Ser Val Pro Asp Ile
        195                 200                 205

Asn Leu Leu Asp Tyr Leu Pro Glu Ile Leu Asp Gly Leu Phe Gln Ile
    210                 215                 220

Leu Gly Asp Asn Gly Lys Glu Ile Arg Lys Met Cys Glu Val Val Leu
225                 230                 235                 240

Gly Glu Phe Leu Lys Glu Ile Lys Lys Asn Pro Ser Ser Val Lys Phe
                245                 250                 255

Ala Glu Met Ala Asn Ile Leu Val Ile His Cys Gln Thr Thr Asp Asp
            260                 265                 270

Leu Ile Gln Leu Thr Ala Met Cys Trp Met Arg Glu Phe Ile Gln Leu
        275                 280                 285

Ala Gly Arg Val Met Leu Pro Tyr Ser Ser Gly Ile Leu Thr Ala Val
    290                 295                 300

Leu Pro Cys Leu Ala Tyr Asp Asp Arg Lys Lys Ser Ile Lys Glu Val
305                 310                 315                 320

Ala Asn Val Cys Asn Gln Ser Leu Met Lys Leu Val Thr Pro Glu Asp
                325                 330                 335

Asp Glu Pro Asp Glu Pro Lys Ser Val Ala Gln Lys Gln Thr Glu Pro
            340                 345                 350

Asn Pro Glu Asp Ser Leu Pro Lys Gln Glu Gly Thr Ala Ser Gly Gly
        355                 360                 365

Pro Gly Ser Cys Asp Ser Ser Phe Gly Ser Gly Ile Asn Val Phe Thr
    370                 375                 380

Ser Ala Asn Thr Asp Arg Ala Pro Val Thr Leu His Leu Asp Gly Ile
385                 390                 395                 400

Val Gln Val Leu Asn Cys His Leu Ser Asp Thr Thr Ile Gly Met Met
                405                 410                 415

Thr Arg Ile Ala Val Leu Lys Trp Leu Tyr His Leu Tyr Ile Lys Thr
            420                 425                 430

Pro Arg Lys Met Phe Arg His Thr Asp Ser Leu Phe Pro Ile Leu Leu
        435                 440                 445

Gln Thr Leu Ser Asp Glu Ser Asp Glu Val Val Leu Lys Asp Leu Glu
    450                 455                 460

Val Leu Ala Glu Ile Ala Ser Ser Pro Ala Gly Gln Thr Asp Asp Pro
465                 470                 475                 480

Gly Ala Pro Asp Gly Pro Asp Leu Arg Val Asn His Ser Glu Leu Gln
                485                 490                 495

Val Pro Thr Ser Gly Arg Ala Asn Leu Leu Asn Pro Pro Ser Thr Lys
            500                 505                 510

Gly Leu Glu Gly Ser Pro Ser Thr Pro Thr Met Asn Ser Tyr Phe Tyr
        515                 520                 525

Lys Phe Met Ile Asn Leu Leu Gln Thr Phe Ser Ser Glu Arg Lys Leu
    530                 535                 540

Leu Glu Ala Arg Gly Pro Phe Ile Ile Arg Gln Leu Cys Leu Leu Leu
545                 550                 555                 560

Asn Ala Glu Asn Ile Phe His Ser Met Ala Asp Ile Leu Leu Arg Glu
                565                 570                 575

Glu Asp Leu Lys Phe Ala Ser Thr Met Val His Thr Leu Asn Thr Ile
            580                 585                 590

Leu Leu Thr Ser Thr Glu Leu Phe Gln Leu Arg Asn Gln Leu Lys Asp
        595                 600                 605

Leu Gln Thr Pro Glu Ser Gln Asn Leu Phe Cys Cys Leu Tyr Arg Ser
    610                 615                 620

Trp Cys His Asn Pro Val Thr Thr Val Ser Leu Cys Phe Leu Thr Gln
625                 630                 635                 640

Asn Tyr Arg His Ala Tyr Asp Leu Ile Gln Lys Phe Gly Asp Leu Glu
                645                 650                 655

Val Thr Val Asp Phe Leu Thr Glu Val Asp Lys Leu Val Gln Leu Ile
            660                 665                 670

Glu Cys Pro Ile Phe Thr Tyr Leu Arg Leu Gln Leu Leu Asp Val Lys
        675                 680                 685

Asn Asn Pro Tyr Leu Ile Lys Ala Leu Tyr Gly Leu Leu Met Leu Leu
    690                 695                 700

Pro Gln Ser Ser Ala Phe Gln Leu Leu Ser His Arg Leu Gln Cys Val
705                 710                 715                 720

Pro Asn Pro Glu Leu Leu Gln Thr Glu Asp Cys Leu Lys Ala Ala Pro
                725                 730                 735

Lys Ser Gln Lys Gly Asp Ser Pro Ser Ile Asp Tyr Thr Glu Leu Leu
            740                 745                 750

Gln His Phe Glu Lys Val Gln Lys Gln His Leu Glu Val Arg His Gln
        755                 760                 765

Arg Ser Gly Arg Gly Asp His Leu Asp Arg Arg Val Ile Leu
    770                 775                 780

<210>  9
<211>  966
<212>  DNA
<213>  human p40

<220>
<221>  CDS
<222>  (1)..(966)

<400>  9
atg aag caa ctg cca gtc ttg gaa cct gga gac aag ccc agg aaa gca      48
Met Lys Gln Leu Pro Val Leu Glu Pro Gly Asp Lys Pro Arg Lys Ala
1               5                   10                  15

aca tgg tac acc ttg act gtc cct gga gac agc ccc tgt gct cga gtt      96
Thr Trp Tyr Thr Leu Thr Val Pro Gly Asp Ser Pro Cys Ala Arg Val
            20                  25                  30

ggc cac agc tgt tca tat tta ccc cca gtt ggt aat gcc aag aga ggg      144
Gly His Ser Cys Ser Tyr Leu Pro Pro Val Gly Asn Ala Lys Arg Gly
        35                  40                  45

aag gtc ttc att gtt ggg gga gca aat cca aac aga agc ttc tca gac      192
Lys Val Phe Ile Val Gly Gly Ala Asn Pro Asn Arg Ser Phe Ser Asp
    50                  55                  60

gtg cac acc atg gat ctg gaa acc agg acg tgg acc acg cca gaa gtg      240

Val His Thr Met Asp Leu Glu Thr Arg Thr Trp Thr Thr Pro Glu Val
65                  70                  75                  80

acc agc ccc cca cca tcc cca aga aca ttc cac aca tca tcg gca gcc     288
Thr Ser Pro Pro Pro Ser Pro Arg Thr Phe His Thr Ser Ser Ala Ala
                85                  90                  95

att gga aac cag cta tat gtc ttt ggg ggc gga gag aga ggt gcc cag     336
Ile Gly Asn Gln Leu Tyr Val Phe Gly Gly Gly Glu Arg Gly Ala Gln
            100                 105                 110

ccc gtg cag gac acg aag ctg cat gtg ttt gac gca aac act ctg acc     384
Pro Val Gln Asp Thr Lys Leu His Val Phe Asp Ala Asn Thr Leu Thr
        115                 120                 125

tgg tca cag cca gag aca ctt gga aat cct cca tct ccc cgg cat ggt     432
Trp Ser Gln Pro Glu Thr Leu Gly Asn Pro Pro Ser Pro Arg His Gly
    130                 135                 140

cat gtg atg gtg gca gca ggg aca aag ctc ttc atc cac gga ggc ttg     480
His Val Met Val Ala Ala Gly Thr Lys Leu Phe Ile His Gly Gly Leu
145                 150                 155                 160

gcg ggg gac aga ttc tat gat gac ctc cac tgc att gat ata agt gac     528
Ala Gly Asp Arg Phe Tyr Asp Asp Leu His Cys Ile Asp Ile Ser Asp
                165                 170                 175

atg aaa tgg cag aag cta aat ccc act ggg gct gct cca gca ggc tgt     576
Met Lys Trp Gln Lys Leu Asn Pro Thr Gly Ala Ala Pro Ala Gly Cys
            180                 185                 190

gct gcc cac tca gct gtg gcc atg gga aaa cat gtg tac atc ttt ggt     624
Ala Ala His Ser Ala Val Ala Met Gly Lys His Val Tyr Ile Phe Gly
        195                 200                 205

gga atg act cct gca gga gca ctg gac aca atg tac cag tat cac aca     672
Gly Met Thr Pro Ala Gly Ala Leu Asp Thr Met Tyr Gln Tyr His Thr
    210                 215                 220

gaa gag cag cat tgg acc ttg ctt aaa ttt gat act ctt cta ccc cct     720
Glu Glu Gln His Trp Thr Leu Leu Lys Phe Asp Thr Leu Leu Pro Pro
225                 230                 235                 240

gga cga ttg gac cat tcc atg tgt atc att cca tgg cca gtg acg tgt     768
Gly Arg Leu Asp His Ser Met Cys Ile Ile Pro Trp Pro Val Thr Cys
                245                 250                 255

gct tct gag aaa gaa gat tcc aac tct ctc act ctg aac cat gaa gct     816
Ala Ser Glu Lys Glu Asp Ser Asn Ser Leu Thr Leu Asn His Glu Ala
            260                 265                 270

gag aaa gag gat tca gct gac aaa gta atg agc cac agt ggt gac tca     864
Glu Lys Glu Asp Ser Ala Asp Lys Val Met Ser His Ser Gly Asp Ser
        275                 280                 285

cat gag gaa agc cag act gct aca ctg ctc tgt ttg gtg ttt ggt ggg     912
His Glu Glu Ser Gln Thr Ala Thr Leu Leu Cys Leu Val Phe Gly Gly
    290                 295                 300

atg aat aca gaa ggg gaa atc tat gac gat tgt att gtg act gta gtg     960
Met Asn Thr Glu Gly Glu Ile Tyr Asp Asp Cys Ile Val Thr Val Val
305                 310                 315                 320

gac taa                                                             966
Asp

<210>  10
<211>  321
<212>  PRT
<213>  human p40

<400>  10

Met Lys Gln Leu Pro Val Leu Glu Pro Gly Asp Lys Pro Arg Lys Ala
1               5                   10                  15

Thr Trp Tyr Thr Leu Thr Val Pro Gly Asp Ser Pro Cys Ala Arg Val
            20                  25                  30

Gly His Ser Cys Ser Tyr Leu Pro Pro Val Gly Asn Ala Lys Arg Gly
        35                  40                  45

Lys Val Phe Ile Val Gly Gly Ala Asn Pro Asn Arg Ser Phe Ser Asp
    50                  55                  60

Val His Thr Met Asp Leu Glu Thr Arg Thr Trp Thr Thr Pro Glu Val
65                  70                  75                  80

Thr Ser Pro Pro Pro Ser Pro Arg Thr Phe His Thr Ser Ser Ala Ala
                85                  90                  95

Ile Gly Asn Gln Leu Tyr Val Phe Gly Gly Gly Glu Arg Gly Ala Gln
            100                 105                 110

Pro Val Gln Asp Thr Lys Leu His Val Phe Asp Ala Asn Thr Leu Thr
        115                 120                 125

Trp Ser Gln Pro Glu Thr Leu Gly Asn Pro Pro Ser Pro Arg His Gly
    130                 135                 140

His Val Met Val Ala Ala Gly Thr Lys Leu Phe Ile His Gly Gly Leu
145                 150                 155                 160

Ala Gly Asp Arg Phe Tyr Asp Asp Leu His Cys Ile Asp Ile Ser Asp
                165                 170                 175

Met Lys Trp Gln Lys Leu Asn Pro Thr Gly Ala Ala Pro Ala Gly Cys
            180                 185                 190

Ala Ala His Ser Ala Val Ala Met Gly Lys His Val Tyr Ile Phe Gly
        195                 200                 205

Gly Met Thr Pro Ala Gly Ala Leu Asp Thr Met Tyr Gln Tyr His Thr
    210                 215                 220

Glu Glu Gln His Trp Thr Leu Leu Lys Phe Asp Thr Leu Leu Pro Pro
225                 230                 235                 240

Gly Arg Leu Asp His Ser Met Cys Ile Ile Pro Trp Pro Val Thr Cys
                245                 250                 255

Ala Ser Glu Lys Glu Asp Ser Asn Ser Leu Thr Leu Asn His Glu Ala
            260                 265                 270

Glu Lys Glu Asp Ser Ala Asp Lys Val Met Ser His Ser Gly Asp Ser
        275                 280                 285

His Glu Glu Ser Gln Thr Ala Thr Leu Leu Cys Leu Val Phe Gly Gly
    290                 295                 300

Met Asn Thr Glu Gly Glu Ile Tyr Asp Asp Cys Ile Val Thr Val Val
305                 310                 315                 320

Asp

<210>  11
<211>  21
<212>  DNA
<213>  Vac14 siRNA (ID#: s107929) sense

<220>
<221>  misc_feature
<222>  (1)..(19)
<223>  RNA

<220>
<221>  misc_feature
<222>  (20)..(21)
<223>  DNA

<400>  11
gauuuacucc aacaaccaat t                                              21

<210>  12
<211>  21
<212>  DNA
<213>  Vac14 siRNA (ID#: s107929) antisense

<220>
<221>  misc_feature
<222>  (1)..(19)
<223>  RNA

<220>
<221>  misc_feature
<222>  (20)..(21)
<223>  DNA

<400>  12
uugguuguug gaguaaaucc t                                                21


<210>  13
<211>  21
<212>  DNA
<213>  PIKfyve siRNA sense


<220>
<221>  misc_feature
<222>  (1)..(19)
<223>  RNA

<220>
<221>  misc_feature
<222>  (20)..(21)
<223>  DNA

<400>  13
gacgucccca acacuggact t                                                21


<210>  14
<211>  21
<212>  DNA
<213>  PIKfyve siRNA antisense


<220>
<221>  misc_feature
<222>  (1)..(19)
<223>  RNA

<220>
<221>  misc_feature
<222>  (20)..(21)
<223>  DNA

<400>  14
guccaguguu ggggacguct t                                                21


<210>  15
<211>  21
<212>  DNA
<213>  PIKfyve siRNA sense


<220>
<221>  misc_feature
<222>  (1)..(19)
<223>  RNA

<220>
<221>  misc_feature
<222>  (20)..(21)
<223>  DNA

<400>  15
cacuggacuc ugcuaaugat t                                                21


<210>  16

<211>  21
<212>  DNA
<213>  PIKfyve siRNA antisense


<220>
<221>  misc_feature
<222>  (1)..(19)
<223>  RNA

<220>
<221>  misc_feature
<222>  (20)..(21)
<223>  DNA

<400>  16
ucauuagcag aguccagugt t                                                21


<210>  17
<211>  21
<212>  DNA
<213>  PIKfyve siRNA sense


<220>
<221>  misc_feature
<222>  (1)..(19)
<223>  RNA

<220>
<221>  misc_feature
<222>  (20)..(21)
<223>  DNA

<400>  17
ugauuugccu cgaucuccut t                                                21


<210>  18
<211>  21
<212>  DNA
<213>  PIKfyve siRNA antisense


<220>
<221>  misc_feature
<222>  (1)..(19)
<223>  RNA

<220>
<221>  misc_feature
<222>  (20)..(21)
<223>  DNA

<400>  18
aggagaucga ggcaaaucat t                                                21


<210>  19
<211>  21
<212>  DNA
<213>  PIKfyve siRNA sense

<220>
<221>  misc_feature
<222>  (1)..(19)
<223>  RNA

<220>
<221>  misc_feature
<222>  (20)..(21)
<223>  DNA

<400>  19
cagcagccuu ugaguggaat t                                                21


<210>  20
<211>  21
<212>  DNA
<213>  PIKfyve siRNA antisense


<220>
<221>  misc_feature
<222>  (1)..(19)
<223>  RNA

<220>
<221>  misc_feature
<222>  (20)..(21)
<223>  DNA

<400>  20
uuccacucaa aggcugcugt t                                                21


<210>  21
<211>  21
<212>  DNA
<213>  PIKfyve siRNA sense


<220>
<221>  misc_feature
<222>  (1)..(19)
<223>  RNA

<220>
<221>  misc_feature
<222>  (20)..(21)
<223>  DNA

<400>  21
aaagcagcuu aaugaggaat t                                                21


<210>  22
<211>  21
<212>  DNA
<213>  PIKfyve siRNA antisense


<220>
<221>  misc_feature
<222>  (1)..(19)
<223>  RNA

<220>
<221>  misc_feature
<222>  (20)..(21)
<223>  DNA

<400>  22
uuccucauua agcugcuuut t                                                21


<210>  23
<211>  21
<212>  DNA
<213>  PIKfyve siRNA sense


<220>
<221>  misc_feature
<222>  (1)..(19)
<223>  RNA

<220>
<221>  misc_feature
<222>  (20)..(21)
<223>  DNA

<400>  23
ggaaagcaga accuaccuut t                                                21


<210>  24
<211>  21
<212>  DNA
<213>  PIKfyve siRNA antisense


<220>
<221>  misc_feature
<222>  (1)..(19)
<223>  RNA

<220>
<221>  misc_feature
<222>  (20)..(21)
<223>  DNA

<400>  24
aagguagguu cugcuuucct t                                                21


<210>  25
<211>  21
<212>  DNA
<213>  PIKfyve siRNA sense


<220>
<221>  misc_feature
<222>  (1)..(19)
<223>  RNA

<220>
<221>  misc_feature
<222>  (20)..(21)
<223>  DNA

<400>  25
ccuaccuuug gaggucaugt t                                                21


<210>  26
<211>  21
<212>  DNA
<213>  PIKfyve siRNA antisense


<220>
<221>  misc_feature
<222>  (1)..(19)
<223>  RNA

<220>
<221>  misc_feature
<222>  (20)..(21)
<223>  DNA

<400>  26
caugaccucc aaagguaggt t                                                21


<210>  27
<211>  21
<212>  DNA
<213>  PIKfyve siRNA sense


<220>
<221>  misc_feature
<222>  (1)..(19)
<223>  RNA

<220>
<221>  misc_feature
<222>  (20)..(21)
<223>  DNA

<400>  27
cgccucaagg aaaucauggt t                                                21


<210>  28
<211>  21
<212>  DNA
<213>  PIKfyve siRNA antisense


<220>
<221>  misc_feature
<222>  (1)..(19)
<223>  RNA

<220>
<221>  misc_feature
<222>  (20)..(21)
<223>  DNA

<400>  28
ccaugauuuc cuugaggcgt t                                                21


<210>  29

<211>  21
<212>  DNA
<213>  PIKfyve siRNA sense


<220>
<221>  misc_feature
<222>  (1)..(19)
<223>  RNA

<220>
<221>  misc_feature
<222>  (20)..(21)
<223>  DNA

<400>  29
guuaugcuca uuccacagat t                                                21


<210>  30
<211>  21
<212>  DNA
<213>  PIKfyve siRNA antisense


<220>
<221>  misc_feature
<222>  (1)..(19)
<223>  RNA

<220>
<221>  misc_feature
<222>  (20)..(21)
<223>  DNA

<400>  30
ucuguggaau gagcauaact t                                                21


<210>  31
<211>  21
<212>  DNA
<213>  PIKfyve siRNA sense


<220>
<221>  misc_feature
<222>  (1)..(19)
<223>  RNA

<220>
<221>  misc_feature
<222>  (20)..(21)
<223>  DNA

<400>  31
cauauguuag gacagagact t                                                21


<210>  32
<211>  21
<212>  DNA
<213>  PIKfyve siRNA antisense

<220>
<221>  misc_feature
<222>  (1)..(19)
<223>  RNA

<220>
<221>  misc_feature
<222>  (20)..(21)
<223>  DNA

<400>  32
gucucugucc uaacauaugt t                                                21


<210>  33
<211>  21
<212>  DNA
<213>  vac14 siRNA sense


<220>
<221>  misc_feature
<222>  (1)..(19)
<223>  RNA

<220>
<221>  misc_feature
<222>  (20)..(21)
<223>  DNA

<400>  33
ccccgagaag gauuucgcgt t                                                21


<210>  34
<211>  21
<212>  DNA
<213>  vac14 siRNA antisense


<220>
<221>  misc_feature
<222>  (1)..(19)
<223>  RNA

<220>
<221>  misc_feature
<222>  (20)..(21)
<223>  DNA

<400>  34
cgcgaaaucc uucucggggt t                                                21


<210>  35
<211>  21
<212>  DNA
<213>  vac14 siRNA sense


<220>
<221>  misc_feature
<222>  (1)..(19)
<223>  RNA

<220>
<221>  misc_feature
<222>  (20)..(21)
<223>  DNA

<400>  35
aagcggaagg uggcagcgct t                                                21


<210>  36
<211>  21
<212>  DNA
<213>  vac14 siRNA antisense


<220>
<221>  misc_feature
<222>  (1)..(19)
<223>  RNA

<220>
<221>  misc_feature
<222>  (20)..(21)
<223>  DNA

<400>  36
gcgcugccac cuuccgcuut t                                                21


<210>  37
<211>  21
<212>  DNA
<213>  vac14 siRNA sense


<220>
<221>  misc_feature
<222>  (1)..(19)
<223>  RNA

<220>
<221>  misc_feature
<222>  (20)..(21)
<223>  DNA

<400>  37
aucaagcaug ugauccagat t                                                21


<210>  38
<211>  21
<212>  DNA
<213>  vac14 siRNA antisense


<220>
<221>  misc_feature
<222>  (1)..(19)
<223>  RNA

<220>
<221>  misc_feature
<222>  (20)..(21)
<223>  DNA

<400>  38
ucuggaucac augcuugaut t                                                21


<210>  39
<211>  21
<212>  DNA
<213>  vac14 siRNA sense


<220>
<221>  misc_feature
<222>  (1)..(19)
<223>  RNA

<220>
<221>  misc_feature
<222>  (20)..(21)
<223>  DNA

<400>  39
ggagcugauc gagccagugt t                                                21


<210>  40
<211>  21
<212>  DNA
<213>  vac14 siRNA antisense


<220>
<221>  misc_feature
<222>  (1)..(19)
<223>  RNA

<220>
<221>  misc_feature
<222>  (20)..(21)
<223>  DNA

<400>  40
cacuggcucg aucagcucct t                                                21


<210>  41
<211>  21
<212>  DNA
<213>  vac14 siRNA sense


<220>
<221>  misc_feature
<222>  (1)..(19)
<223>  RNA

<220>
<221>  misc_feature
<222>  (20)..(21)
<223>  DNA

<400>  41
gcggaucuga gcuccuagat t                                                21


<210>  42

<211>  21
<212>  DNA
<213>  vac14 siRNA antisense


<220>
<221>  misc_feature
<222>  (1)..(19)
<223>  RNA

<220>
<221>  misc_feature
<222>  (20)..(21)
<223>  DNA

<400>  42
ucuaggagcu cagauccgct t                                                21


<210>  43
<211>  21
<212>  DNA
<213>  vac14 siRNA sense


<220>
<221>  misc_feature
<222>  (1)..(19)
<223>  RNA

<220>
<221>  misc_feature
<222>  (20)..(21)
<223>  DNA

<400>  43
guuugaccug gugagcuuct t                                                21


<210>  44
<211>  21
<212>  DNA
<213>  vac14 siRNA antisense


<220>
<221>  misc_feature
<222>  (1)..(19)
<223>  RNA

<220>
<221>  misc_feature
<222>  (20)..(21)
<223>  DNA

<400>  44
gaagcucacc aggucaaact t                                                21


<210>  45
<211>  21
<212>  DNA
<213>  vac14 siRNA sense

<220>
<221>  misc_feature
<222>  (1)..(19)
<223>  RNA

<220>
<221>  misc_feature
<222>  (20)..(21)
<223>  DNA

<400>  45
auuaagaaga accccuccat t                                                21


<210>  46
<211>  21
<212>  DNA
<213>  vac14 siRNA antisense


<220>
<221>  misc_feature
<222>  (1)..(19)
<223>  RNA

<220>
<221>  misc_feature
<222>  (20)..(21)
<223>  DNA

<400>  46
uggagggguu cuucuuaaut t                                                21


<210>  47
<211>  21
<212>  DNA
<213>  vac14 siRNA sense


<220>
<221>  misc_feature
<222>  (1)..(19)
<223>  RNA

<220>
<221>  misc_feature
<222>  (20)..(21)
<223>  DNA

<400>  47
guuugcugag auggccaact t                                                21


<210>  48
<211>  21
<212>  DNA
<213>  vac14 siRNA antisense


<220>
<221>  misc_feature
<222>  (1)..(19)
<223>  RNA

<220>
<221>  misc_feature
<222>  (20)..(21)
<223>  DNA

<400>  48
guuggccauc ucagcaaact t                                                21


<210>  49
<211>  21
<212>  DNA
<213>  vac14 siRNA sense


<220>
<221>  misc_feature
<222>  (1)..(19)
<223>  RNA

<220>
<221>  misc_feature
<222>  (20)..(21)
<223>  DNA

<400>  49
aagcaucaaa gaaguggcct t                                                21


<210>  50
<211>  21
<212>  DNA
<213>  vac14 siRNA antisense


<220>
<221>  misc_feature
<222>  (1)..(19)
<223>  RNA

<220>
<221>  misc_feature
<222>  (20)..(21)
<223>  DNA

<400>  50
ggccacuucu uugaugcuut t                                                21


<210>  51
<211>  21
<212>  DNA
<213>  vac14 siRNA sense


<220>
<221>  misc_feature
<222>  (1)..(19)
<223>  RNA

<220>
<221>  misc_feature
<222>  (20)..(21)
<223>  DNA

<400>  51
gcuccuggag gucagaggct t                                                21

<210>  52
<211>  21
<212>  DNA
<213>  vac14 siRNA antisense

<220>
<221>  misc_feature
<222>  (1)..(19)
<223>  RNA

<220>
<221>  misc_feature
<222>  (20)..(21)
<223>  DNA

<400>  52
gccucugacc uccaggagct t                                                21

<210>  53
<211>  20
<212>  DNA
<213>  DAPDH primer forward

<400>  53
atcactgcca cccagaagac                                                  20

<210>  54
<211>  20
<212>  DNA
<213>  DAPDH primer reverse

<400>  54
ggatgcaggg atgatgttct                                                  20

<210>  55
<211>  20
<212>  DNA
<213>  IL12b primer forward

<400>  55
cagcaccagc ttcttcatca                                                  20

<210>  56
<211>  20
<212>  DNA
<213>  IL12b primer reverse

<400>  56
tactcccagc tgacctccac                                                  20